# EUROPEAN PATENT APPLICATION

(11) **EP 4 394 382 A1**
(43) Date of publication of application: **03.07.2024**
(21) Application number: 22861471.5
(22) Date of filing: 26.08.2022
(51) Int. Cl.: G01N 33/543, C12M 1/34, C12N 15/11

(54) **TECHNIQUE FOR ALIGNING PARTICLES ON SUBSTRATE WITHOUT AGGLOMERATION**

(30) Priority: 27.08.2021 JP 2021139398
(71) Applicant: National University Corporation Kobe University, Kobe-shi, Hyogo 657-8501 (JP)
(72) Inventor: TAKEUCHI, Toshifumi, Kobe-shi, Hyogo 657-8501 (JP); SUNAYAMA, Hirobumi, Kobe-shi, Hyogo 657-8501 (JP); TAKANO, Eri, Kobe-shi, Hyogo 657-8501 (JP); HORIKAWA, Ryo, Kobe-shi, Hyogo 657-8501 (JP)
(74) Representative: Weickmann & Weickmann PartmbB
(86) International application number: PCT/JP2022/032269
(87) International publication number: WO 2023/027183

(57) **Abstract**

Provided is a novel technique for providing a substrate advantageous when producing a sensor for analysis. The substrate for producing a sensor for analysis provided in the present disclosure has specific characteristics (for example, monodispersion, a single layer, or a specific density), such that it is possible to produce a sensor for analysis that is stable and enables highly accurate analysis. In the present disclosure, it is not required to remove excess fine particles after arranging the fine particles on the substrate, reproducibility of the density of the fine particles immobilized on the substrate is improved, and monodispersibility and single-layer properties are also improved.

## Description

### Technical Field

The present disclosure relates to a technique for aligning particles on a substrate without agglomeration that is used in a measurement technique and an application thereof. More specifically, the present disclosure relates to a substrate for producing a sensor for analysis of an object to be detected, the substrate having a scaffold disposed thereon, and a method for producing the same, a sensor for analysis of an object to be detected and a method for producing the same, and a method for analyzing an object to be detected.

### Background Art

Small extracellular vesicles (sEVs) such as an exosome are one of vesicles released from a cell, and are lipid bilayer membrane vesicles having a diameter of 20 to 200 nm. The small extracellular vesicles contain proteins and nucleic acids such as miRNA and mRNA inside them, and also have proteins on surfaces thereof. Since the small extracellular vesicles are characterized by such substances, it is considered that it is possible to infer what kind of cell the secreted cell is by analyzing the characteristics of the small extracellular vesicles. In addition, small extracellular vesicles have been confirmed to exist in various body fluids, and can be collected relatively easily.

Small extracellular vesicles secreted from cancer cells contain substances derived from tumors. Therefore, it is expected that diagnosis of cancer can be performed by analyzing substances contained in small extracellular vesicles in a body fluid. Furthermore, since small extracellular vesicles are actively secreted by cells, it is expected that the small extracellular vesicles exhibit some characteristics even at an early stage of cancer.

### Summary of Invention

### Solution to Problem

The present disclosure provides a novel technique for providing a substrate that is advantageous in producing a sensor for analysis. More specifically, the substrate for producing a sensor for analysis provided in the present disclosure has specific characteristics (for example, monodispersion, a single layer, or a specific density), such that it is possible to produce a sensor for analysis that is stable and enables highly accurate analysis. In the present disclosure, it is not required to remove excess fine particles after arranging the fine particles on the substrate, reproducibility of the density of the fine particles immobilized on the substrate is improved, and monodispersibility and single-layer properties are also improved. In one preferred embodiment, the present disclosure can reproducibly obtain a monodisperse fine particle-immobilized substrate and/or a single layer fine particle-immobilized substrate by immobilizing particles using an electrostatic interaction between the substrate and the particles and disposing the particles on the substrate in a monodisperse and/or single layer form.

The present disclosure provides, for example, the following items.

(Item A1) A substrate for producing a sensor for analysis, the substrate including:
   A) a substrate body; and
   B) particles disposed on the substrate body in a monodisperse form.
(Item A2-1) A substrate for producing a sensor for analysis, the substrate including:
   A) a substrate body; and
   B) particles disposed on the substrate body at a density of 1 × 10³ particles/mm² to 1 × 10⁸ particles/mm².
(Item A2-2) A substrate for producing a sensor for analysis, the substrate including:
   A) a substrate body; and
   B) particles disposed on the substrate body at a density of 1 × 10³ particles/mm² to 1 × 10¹⁰ particles/mm².
(Item A2-3) The substrate for producing a sensor for analysis according to any one of the preceding items, in which the particles contain a substance used for a sensor for analysis.
(Item A3) The substrate for producing a sensor for analysis according to any one of the preceding items, in which the particles include particles integrated with a modifier.
(Item A4) The substrate for producing a sensor for analysis according to any one of the preceding items, further including C) a polymer matrix disposed on the substrate body, the polymer matrix having a concave portion that at least partially fits into an object and having the particles disposed in the concave portion.
(Item B1A) A convex sensor for analysis, including:
   A) a substrate body;
   B) a polymer matrix disposed on the substrate body, the polymer matrix having a concave portion that at least partially fits into an object and having the particles disposed in the concave portion;
   C) a group for binding a signal substance disposed on the particle; and
   D) a group for binding a specific binding molecule that binds to a molecule to be detected, the group being disposed on the particle,
   in which the particle portions are disposed on the substrate body at a density of 1 × 10³ particles/mm² to 1 × 10⁸ particles/mm²,
(Item B1B) A convex sensor for analysis, including:
   A) a substrate body;
   B) a polymer matrix disposed on the substrate body, the polymer matrix having a concave portion that at least partially fits into an object and having the particles disposed in the concave portion;
   C) a group for binding a signal substance disposed on the particle; and
   D) a group for binding a specific binding molecule that binds to a molecule to be detected, the group being disposed on the particle,
   in which the particle portions are disposed on the substrate body at a density of 1 × 10³ particles/mm² to 1 × 10¹⁰ particles/mm²,
(Item B1C) A sensor for analysis including:
   A) a substrate body;
   B) a polymer matrix disposed on the substrate body, the polymer matrix having a concave portion that at least partially fits into a molecule to be targeted;
   C) a group for binding a signal substance disposed in the concave portion; and
   D) a group for binding a specific binding molecule that binds to a molecule to be detected, the group being disposed in the concave portion,
   in which the concave portions are disposed in the substrate body at a density of 1 × 10³ pieces/mm² to 1 × 10⁸ pieces/mm².
(Item B1D) A sensor for analysis including:
   A) a substrate body;
   B) a polymer matrix disposed on the substrate body, the polymer matrix having a concave portion that at least partially fits into a molecule to be targeted;
   C) a group for binding a signal substance disposed in the concave portion; and
   D) a group for binding a specific binding molecule that binds to a molecule to be detected, the group being disposed in the concave portion,
   in which the concave portions are disposed in the substrate body at a density of 1 × 10³ pieces/mm² to 1 × 10¹⁰ pieces/mm².
(Item B2) The sensor for analysis according to any one of the preceding items, in which the concave portions are present at a density of 1 × 10⁴ pieces/mm² to 1 × 10⁶ pieces/mm².
(Item C1A) A method for producing a substrate for producing a convex sensor for analysis, the method including:
   A) a step of providing particles;
   B) a step of adding the particles to a substrate so that the particles are disposed on a substrate body in a monodisperse form;
   C) a step of providing a raw material of a polymer matrix to the substrate on which the particles are immobilized; and
   D) a step of forming a substrate on which the polymer matrix is disposed by subjecting the substrate to a condition under which the polymer matrix is polymerized.
(Item C1) A method for producing a substrate for producing a sensor for analysis, the method including:
   A) a step of providing particles;
   B) a step of adding the particles to a substrate so that the particles are disposed on a substrate body in a monodisperse form;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized; and
   E) a step of forming a concave portion by subjecting the substrate to a condition under which the particles are dissociated from the substrate.
(Item C1-1) The method according to any one of the preceding items, in which the step of adding the particles so that the particles are disposed in the monodisperse form includes adding particles at a concentration of 1.0 × 10⁰ particles/µL to 2.5 × 10⁸ particles/µL or in an amount of 1.0 × 10⁰ particles to 2.5 × 10⁸ particles per square mm of the substrate.
(Item C1-2) The method according to any one of the preceding items, in which the step of adding the particles so that the particles are disposed in the monodisperse form includes adding particles at a concentration of 1.0 × 10⁰ particles/µL to 1.0 × 10¹⁰ particles/µL or in an amount of 1.0 × 10⁰ particles to 1.0 × 10¹⁰ particles per square mm of the substrate.
(Item C2A) A method for producing a convex sensor for analysis, the method including:
   A) a step of providing particles;
   B) a step of adding the particles to a substrate so that the particles are disposed on a substrate body in a monodisperse form;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized; and
   F) a step of binding a substance required for analysis to the particles.
(Item C2) A method for producing a sensor for analysis, the method including:
   A) a step of providing particles;
   B) a step of adding the particles to a substrate so that the particles are disposed on a substrate body in a monodisperse form;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized;
   E) a step of forming a concave portion by subjecting the substrate to a condition under which the particles are dissociated from the substrate; and
   F) a step of binding a substance required for analysis to the concave portion.
(Item C3A-1) The method for producing a sensor for analysis according to any one of the preceding items, in which the sensors for analysis having a coefficient of variation of 20% or less are produced at a proportion of 80% or more by forming concave portions having a density of 2.0 × 10⁷ pieces/mm² or less.
(Item C3A-2) The method for producing a sensor for analysis according to any one of the preceding items, in which the sensors for analysis having a coefficient of variation of 20% or less are produced at a proportion of 80% or more by forming concave portions having a density of 1.0 × 10¹⁰ pieces/mm² or less.
(Item C3B) The method for producing a sensor for analysis according to any one of the preceding items, in which the sensors for analysis having a coefficient of variation of 20% or less are produced at a proportion of 80% or more by forming concave portions whose area occupancy rate on the substrate is equal to or less than the closest packing.
(Item C4A) The method according to any one of the preceding items, in which the step of disposing the particles in the monodisperse form includes a step of disposing the particles on the substrate at a density of 1 × 10³ particles/mm² to 1 × 10⁸ particles/mm²,
(Item C4B) The method according to any one of the preceding items, in which the step of disposing the particles in the monodisperse form includes a step of disposing the particles on the substrate at a density of 1 × 10³ particles/mm² to 1 × 10¹⁰ particles/mm².
(Item C5A) The method according to any one of the preceding items, in which the step of forming the concave portion, the concave portions disposed in the monodisperse form are present at a density of 1 × 10³ pieces/mm² to 1 × 10⁸ pieces/mm².
(Item C5B) The method according to any one of the preceding items, in which in the step of forming the concave portion, the concave portions disposed in the monodisperse form are present at a density of 1 × 10³ pieces/mm² to 1 × 10¹⁰ pieces/mm².
(Item C6) The method according to any one of the preceding items, in which the step of disposing the particles includes a spin coating method, a method of dropping the particles on a substrate, a method of immersing a substrate in a particle dispersion, a method of pulling up a substrate from a particle dispersion, or a method of spraying a particle dispersion.
(Item 1) A substrate for producing a sensor for analysis, the substrate including:
   A) a substrate body; and
   B) particles disposed on the substrate body in a state of a single layer.
(Item 2) The substrate for producing a sensor for analysis according to any one of the preceding items, in which the particles are disposed without agglomeration.
(Item 1A) A substrate for producing a sensor for analysis, the substrate including:
   A) a substrate body; and
   B) particles disposed on the substrate body without agglomeration.
(Item 2A) The substrate for producing a sensor for analysis according to any one of the preceding items, in which the particles are in a state of a single layer.
(Item 3) The substrate for producing a sensor for analysis according to any one of the preceding items, in which the particles include particles integrated with a modifier.
(Item 4) The substrate for producing a sensor for analysis according to any one of the preceding items, further including C) a polymer matrix disposed on the substrate body, the polymer matrix having a concave portion that at least partially fits into an object, preferably in the sense of functioning as a sensor for analysis, in which the particles are disposed in the concave portion.
(Item 5) A convex sensor for analysis, including:
   A) a substrate body;
   B) a polymer matrix disposed on the substrate body, the polymer matrix having a concave portion that at least partially fits into an object to be detected and having the particles disposed in the concave portion without agglomeration;
   C) a group for binding a signal substance disposed on the particle; and
   D) a group for binding a specific binding molecule that binds to a molecule to be detected, the group being disposed on the particle,
   in which the particles are disposed on the substrate body at a density of 1 × 10³ particles/mm² to 1 × 10¹⁰ particles/mm².
(Item 6) A sensor for analysis including:
   A) a substrate body;
   B) a polymer matrix disposed on the substrate body, the polymer matrix having a concave portion that at least partially fits into an object to be detected;
   C) a group for binding a signal substance disposed in the concave portion; and
   D) a group for binding a specific binding molecule that binds to a molecule to be detected, the group being disposed in the concave portion,
   in which the concave portions are disposed in the substrate body at a density of 1 × 10³ pieces/mm² to 1 × 10¹⁰ pieces/mm² without agglomeration.
(Item 7) The sensor for analysis according to any one of the preceding items, in which the concave portions are present at a density of 1 × 10⁴ pieces/mm² to 1 × 10⁶ pieces/mm² without agglomeration.
(Item 8) A method for producing a substrate for producing a convex sensor for analysis, the method including:
   A) a step of providing particles;
   B) a step of adding the particles to a substrate so that the particles are disposed on a substrate body without agglomeration;
   C) a step of providing a raw material of a polymer matrix to the substrate on which the particles are immobilized; and
   D) a step of forming a substrate on which the polymer matrix is disposed by subjecting the substrate to a condition under which the polymer matrix is polymerized.
(Item 9) A method for producing a substrate for producing a sensor for analysis, the method including:
   A) a step of providing particles;
   B) a step of adding the particles to a substrate so that the particles are disposed on a substrate body without agglomeration;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized; and
   E) a step of forming a concave portion by subjecting the substrate to a condition under which the particles are dissociated from the substrate.
(Item 10) The method according to any one of the preceding items, in which the step of adding the particles so that the particles are disposed without agglomeration includes adding particles to a substrate at a concentration of 1.0 × 10⁰ particles/µL to 1.0 × 10¹⁰ particles/µL or in an amount of 1.0 × 10⁰ particles/mm² to 1.0 × 10¹⁰ particles/mm² per surface area of the substrate.
(Item 11) A method for producing a convex sensor for analysis, the method including:
   A) a step of providing particles;
   B) a step of adding the particles to a substrate so that the particles are disposed on a substrate body without agglomeration;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized; and
   E) a step of binding a substance required for analysis to the particles.
(Item 12) A method for producing a sensor for analysis, the method including:
   A) a step of providing particles;
   B) a step of adding the particles to a substrate so that the particles are disposed on a substrate body without agglomeration;
   C) a step of providing a raw material of a polymer matrix to the substrate on which the particles are immobilized;
   D) a step of forming a substrate on which the polymer matrix is disposed by subjecting the substrate to a condition under which the polymer matrix is polymerized;
   E) a step of forming a concave portion by subjecting the substrate to a condition under which the particles are dissociated from the substrate; and
   F) a step of binding a substance required for analysis to the concave portion.
(Item 13) The method for producing a sensor for analysis according to any one of the preceding items, in which the sensors for analysis having a coefficient of variation of a relative fluorescence intensity change in target substance detection of 20% or less are produced at a proportion of 80% or more by forming concave portions having a density of 1.0 × 10¹⁰ pieces/mm² or less.
(Item 14) The method for producing a sensor for analysis according to any one of the preceding items, in which the sensors for analysis having a coefficient of variation of a relative fluorescence intensity change in target substance detection of 20% or less are produced at a proportion of 80% or more by forming concave portions whose area occupancy rate on the substrate is equal to or less than the closest packing.
(Item 15) The method according to any one of the preceding items, in which the step of disposing the particles without agglomeration includes a step of disposing the particles on the substrate at a density of 1 × 10³ particles/mm² to 1 × 10¹⁰ particles/mm².
(Item 16) The method according to any one of the preceding items, in which the particles are in a state of a single layer.
(Item 17) The method according to any one of the preceding items, in which in the step of forming the concave portion, the concave portions are present at a density of 1 × 10³ pieces/mm² to 1 × 10¹⁰ pieces/mm².
(Item 18) The method according to any one of the preceding items, in which the step of disposing the particles includes a spin coating method, a method of dropping the particles on a substrate, a method of immersing a substrate in a particle dispersion, a method of pulling up a substrate from a particle dispersion, or a method of spraying a particle dispersion.

In the present disclosure, it is intended that the one or a plurality of features may be provided in further combination in addition to the specified combination. Still further embodiments and advantages of the present disclosure will be appreciated by those skilled in the art upon reading and understanding the following detailed description, if necessary.

### Advantageous Effects of Invention

By utilizing the technique related to the present disclosure, monodisperse or single layer pores can be formed on a substrate in a technique for producing pores of a target molecule with particles using a molecular imprinting technique, and reproducibility of pore formation can be improved. The method of the present disclosure is positioned as an important basic technique for producing a uniform sensor substrate.

### Brief Description of Drawings

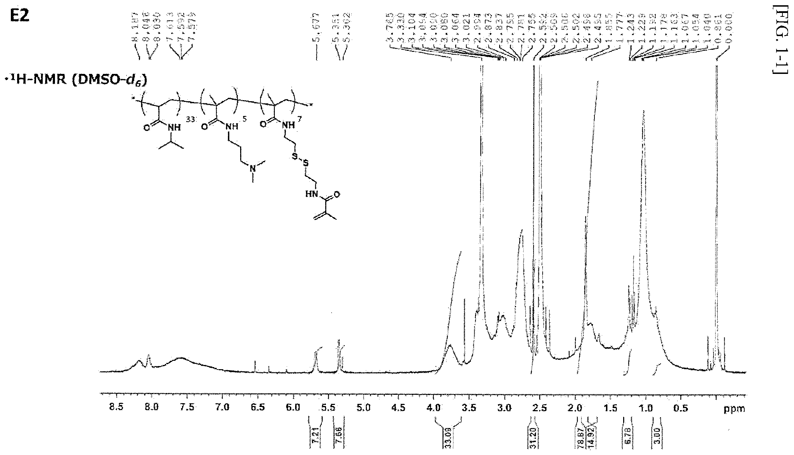

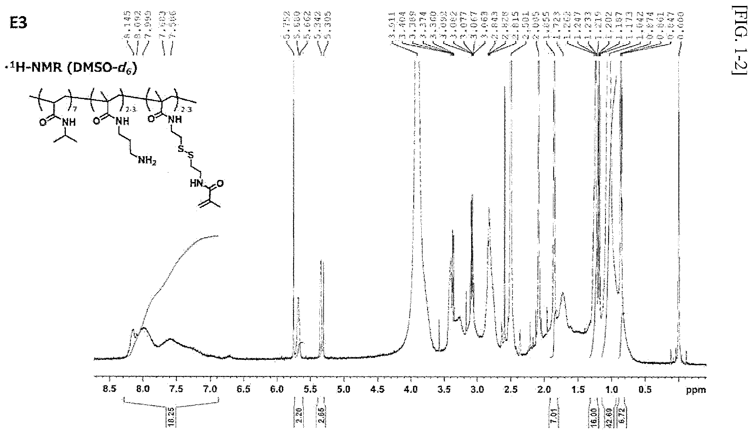

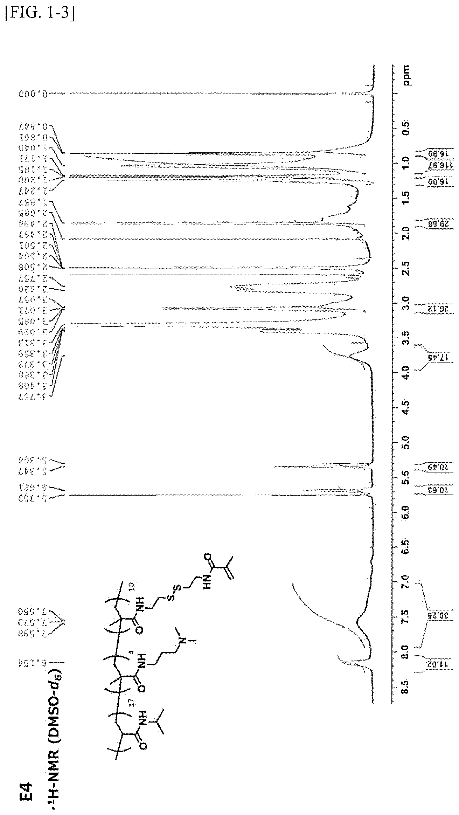

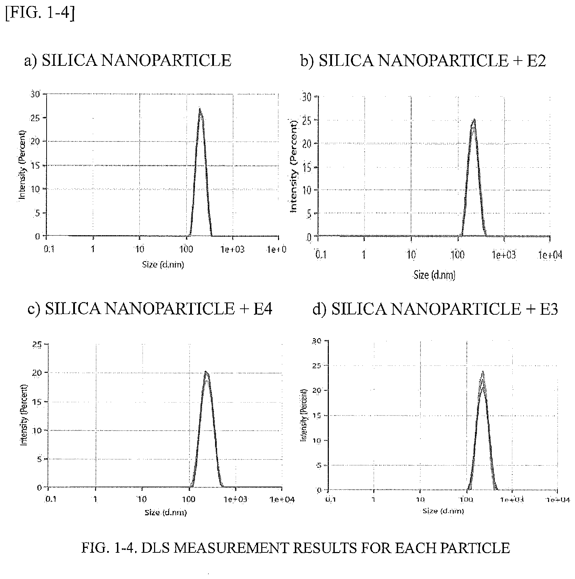

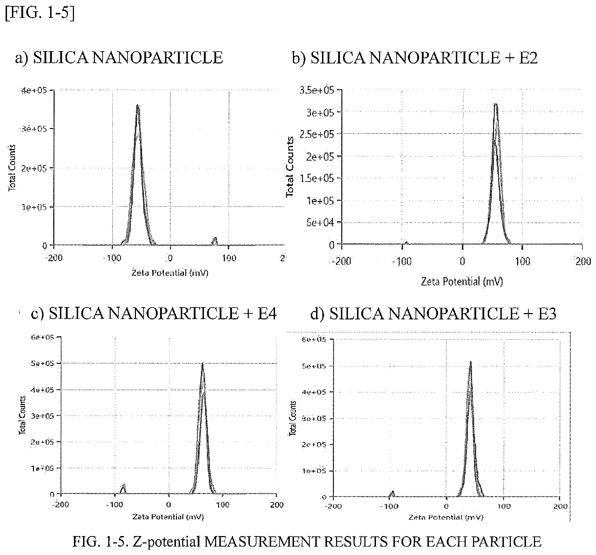

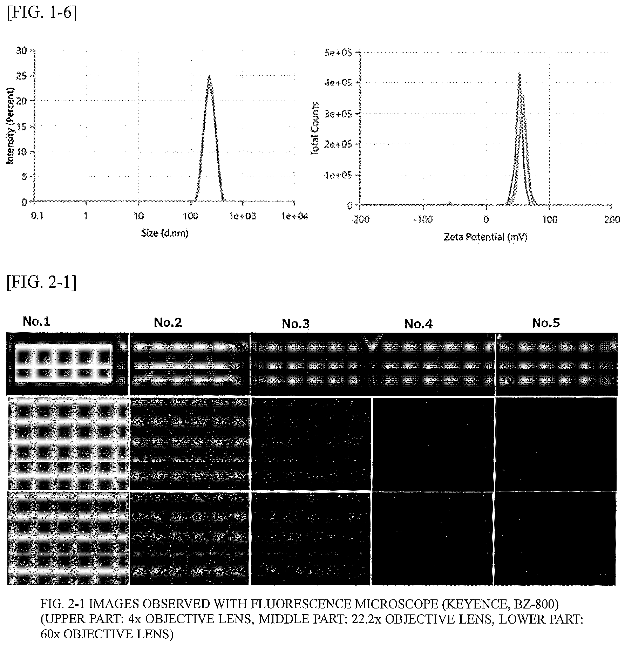

FIG. 1-1 illustrates an NMR chart of a polymer E2.
FIG. 1-2 illustrates an NMR chart of a polymer E3.
FIG. 1-3 illustrates an NMR chart of a polymer E4.
FIG. 1-4 illustrates DLS measurement results of particles in which silica particles and each of polymers E2 to E4 are integrated.
FIG. 1-5 illustrates Z-potential measurement results of particles in which silica particles and each of polymers E2 to E4 are integrated.
FIG. 1-6 illustrates DLS measurement results and Z-potential measurement results of particles in which silica particles and a polymer (E2-0) are integrated.
FIG. 2-1 illustrates images of a particle-immobilized substrate observed with a fluorescence microscope.
FIG. 2-2 illustrates a result of hybrid cell count analysis of a particle-immobilized substrate with a fluorescence microscope.
FIG. 3-1 illustrates images of a particle-immobilized substrate in which silica particles and the polymer E4 are integrated observed with a fluorescence microscope.
FIG. 3-2 illustrates images of a particle-immobilized substrate in which silica particles and the polymer E3 are integrated observed with a fluorescence microscope.
FIG. 4 illustrates images of a particle-immobilized substrate observed with a fluorescence microscope. (Glass substrate).
FIG. 5-1 illustrates images of a particle-immobilized substrate observed with a fluorescence microscope. (Polystyrene particles).
FIG. 5-2 illustrates images of a particle-immobilized substrate observed with a fluorescence microscope. (Polystyrene particles).
FIG. 5-3 illustrates images of a particle-immobilized substrate observed with a fluorescence microscope. (Polystyrene particles).
FIG. 5-4 illustrates a result of hybrid cell count analysis of a particle-immobilized substrate with a fluorescence microscope. (Polystyrene particles).
FIG. 6-1 illustrates images of a particle-immobilized substrate of Example 6 observed with a fluorescence microscope.
FIG. 6-2 illustrates images after polymerization of a polymer layer on a substrate of Example 6 observed with a fluorescence microscope.
FIG. 6-3 illustrates images after removal of silica nanoparticles on the substrate of Example 6 observed with a fluorescence microscope.
FIG. 6-4 illustrates a result of hybrid cell count analysis of a particle-immobilized substrate with a fluorescence microscope.
FIG. 7 illustrates a fluorescence microscopic image of a substrate on which a complex of a His-Tag polymer and silica nanoparticles is immobilized.
FIG. 8 illustrates a fluorescence microscopic image and an SEM image of a substrate on which a complex of cationic silica nanoparticles and an anionic polymer Ex8 is immobilized.
FIG. 9 illustrates a fluorescence microscopic image and an SEM image of a substrate on which particles using biodegradable nanoparticles are immobilized.
FIG. 10-1 illustrates a fluorescence microscopic image and an SEM image of a substrate on which a complex of silica nanoparticles and a cationic polymer E2P20 containing an aromatic component is immobilized.
FIG. 10-2 illustrates a fluorescence microscopic image of a substrate on which a complex of polystyrene nanoparticles and a cationic polymer E2P20 containing an aromatic component is immobilized.
FIG. 11 illustrates a fluorescence microscopic image and an SEM image of a substrate on which silica nanoparticles introduced with a His-tag and a thiol group are immobilized.
FIG. 12 illustrates a change in fluorescence intensity of a substrate surface of a sensor produced without using particles.

### Description of Embodiments

Hereinafter, the present disclosure will be described in more detail.

Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

### (Definitions)

First, terms and general techniques used in the present disclosure will be described.

As used herein, the term "substrate" refers to a substance serving as a base of a sensor for analysis. A material of the substrate may be, for example, a material selected from the group consisting of a metal, a metal oxide, glass, paper (cellulose), a cloth, silicon dioxide, silicon, a resin, and a combination thereof. Examples of the metal include, but are not limited to, gold, silver, copper, aluminum, titanium, tungsten, and molybdenum. Examples of the resin include, but are not limited to, poly(meth)acrylate, polystyrene, an acrylonitrile-butadiene-styrene copolymer (ABS), polycarbonate, polyester, polyethylene, polypropylene, nylon, polyurethane, a silicone resin, a fluororesin, a methylpentene resin, a phenol resin, a melamine resin, an epoxy resin, and a vinyl chloride resin.

As used herein, the term "monodispersion" refers to a dispersion in which a dispersed phase has a uniform size, and in the case of referring to a substrate such as a substrate for producing a sensor for analysis, the term "monodispersion" means that particles are substantially uniformly disposed on the substrate. In the case where the particles are disposed in a monodisperse form, the particles are advantageously disposed without agglomeration. A density to implement monodispersion can vary depending on a target intended in the final sensor for analysis, and is preferably about 10³ particles/mm² to about 10⁸ particles/mm², more preferably about 10⁴ particles/mm² to about 10⁷ particles/mm², and still more preferably about 10⁴ particles/mm² to about 10⁶ particles/mm², Examples of an upper limit of the density include about 1 × 10⁷ particles/mm², about 9 × 10⁶ particles/mm², about 8 × 10⁶ particles/mm², about 7 × 10⁶ particles/mm², about 6 × 10⁶ particles/mm², about 5 × 10⁶ particles/mm², about 4 × 10⁶ particles/mm², about 3 × 10⁶ particles/mm², about 2 × 10⁶ particles/mm², and about 1 × 10⁶ particles/mm², examples of a lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × about 10⁴ particles/mm², and the density is preferably about 10³ particles/mm² to about 10¹⁰ particles/mm², more preferably about 10⁴ particles/mm² to about 10⁹ particles/mm², and still more preferably about 10⁴ particles/mm² to about 10⁸ particles/mm². Examples of the upper limit of the density include about 1 × 10¹⁰ particles/mm², about 9 × 10⁹ particles/mm², about 8 × 10⁹ particles/mm², about 7 × 10⁹ particles/mm², about 6 × 10⁹ particles/mm², about 5 × 10⁹ particles/mm², about 4 × 10⁹ particles/mm², about 3 × 10⁹ particles/mm², about 2 × 10⁹ particles/mm², and about 1 × 10⁹ particles/mm², and examples of the lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × about 10⁴ particles/mm².

As used herein, the term "single layer" refers to a single layer in which particles do not overlap each other. In the case where the particles are disposed in a single layer, the particles are advantageously disposed without agglomeration. A density to implement the single layer can vary depending on a target intended in the final sensor for analysis, and is preferably about 10³ particles/mm² to about 10⁸ particles/mm², more preferably about 10⁴ particles/mm² to about 10⁷ particles/mm², and still more preferably about 10⁴ particles/mm² to about 10⁶ particles/mm², Examples of an upper limit of the density include about 1 × 10⁷ particles/mm², about 9 × 10⁶ particles/mm², about 8 × 10⁶ particles/mm², about 7 × 10⁶ particles/mm², about 6 × 10⁶ particles/mm², about 5 × 10⁶ particles/mm², about 4 × 10⁶ particles/mm², about 3 × 10⁶ particles/mm², about 2 × 10⁶ particles/mm², and about 1 × 10⁶ particles/mm², examples of a lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × about 10⁴ particles/mm², and the density is preferably about 10³ particles/mm² to about 10¹⁰ particles/mm², more preferably about 10⁴ particles/mm² to about 10⁹ particles/mm², and still more preferably about 10⁴ particles/mm² to about 10⁸ particles/mm². Examples of the upper limit of the density include about 1 × 10¹⁰ particles/mm², about 9 × 10⁹ particles/mm², about 8 × 10⁹ particles/mm², about 7 × 10⁹ particles/mm², about 6 × 10⁹ particles/mm², about 5 × 10⁹ particles/mm², about 4 × 10⁹ particles/mm², about 3 × 10⁹ particles/mm², about 2 × 10⁹ particles/mm², and about 1 × 10⁹ particles/mm², and examples of the lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × about 10⁴ particles/mm².

As used herein, the term "agglomeration" refers to agglomeration of a plurality of particles by mutual attraction, and the term "absence of agglomeration" or "no agglomeration" refers to the presence of particles in a state in which such an agglomeration state is substantially absent (that is, equal to or lower than a detection limit). The absence of agglomeration typically means that there is substantially no overlapping and immobilized portion in any direction when measured with an SEM, and the agglomeration is preferably 10% or less of the whole, and more preferably 5% or less, 4% or less, 3% or less, 2% or less, or 1% or less of the whole. The measurement method with an SEM is as follows: after an object to be measured is vacuum-dried, gold sputtering is performed, and conditions are set so that a magnification is 2,000 times or more. In a case where it cannot be determined with an SEM, it can be observed with an atomic force microscope. Using the technique of the present disclosure, particles can be disposed on a substrate without agglomeration.

As used herein, the term "single layer" means that there is substantially no three-dimensionally overlapping and immobilized portion in any direction when measured with an SEM, and the agglomeration is preferably 10% or less of the whole, and more preferably 5% or less, 4% or less, 3% or less, 2% or less, or 1% or less of the whole. Whether or not the object is a single layer is preferably determined by performing measurement on a plurality of points, preferably n = 5 points, and more preferably n = 10 or more points, and calculating an average thereof. In order to determine a single layer, an object to be measured can be vacuum-dried and then subjected to gold sputtering, and conditions can be set so that the magnification is 2,000 times or more, and regarding the determination of the single layer, in a case where it cannot be determined with an SEM, it can be observed with an atomic force microscope. Using the technique of the present disclosure, particles can be disposed on a substrate in a single layer.

As used herein, the term "particle" refers to a basic substance having a structure serving as a template for forming a concave portion provided in a substrate in a sensor for analysis, and may be a substance containing a biomolecule or a substance containing a particle core (for example, a substance containing a particle core and a modifier). Individual particles typically contain substances used in a sensor for analysis. The particle core is not particularly limited as long as it can be used as a template in molecular imprinting, and includes an artificially produced inorganic particle and organic particle. A case where the size is in a range of nm may be referred to as a particle core, but it is understood that the particle core is not strictly distinguished in the present disclosure, and the particle core does not exclude a particle core having a larger or smaller core structure unless the discussion is specifically intended to limit the size. Examples of the inorganic particle include a metal, a metal oxide, a nitride, a fluoride, a sulfide, a boride, and a complex compound thereof, and hydroxyapatite, and preferably include silicon dioxide (silica). In addition, examples of the organic particle include, but are not limited to, a latex cured product, dextran, chitosan, polylactic acid, poly(meth)acrylic acid, polystyrene, and polyethyleneimine. In addition, the particle may be a particle in which a binding functional group is bonded to a surface of a biomolecule or a particle core. A size of the particle can vary depending on a target intended in the final sensor for analysis, and examples thereof include, but are not limited to, about 1 nm to about 100 µm, about 1 nm to about 20 nm, about 20 nm to about 500 nm, about 50 nm to about 200 nm, about 100 nm to about 500 nm, about 1 µm to about 10 µm, and about 10 µm to about 100 µm. The particle used in the present disclosure may be a particle in which a modifier is integrated with a particle core.

As used herein, the term "modifier" is a substance for directly or indirectly imparting an intended function in a sensor for analysis or a substrate for producing a sensor for analysis, which contains various functional groups or has a structure that can contain various functional groups in the structure in order to impart such a function. In the present disclosure, the modifier is advantageously a substance capable of interacting with the particle core, and preferably a substance capable of integrating with the particle core by an interaction that is separable under conditions that do not damage the substrate and/or a substance capable of integrating with the particle core by an interaction that is separable under conditions that do not damage the particle core. Representative examples of the modifier include, but are not limited to, a polyisopropylacrylamide copolymer, a poly(meth)acrylamide copolymer, a polylactic acid derivative, a poly(meth)acrylic acid copolymer, a poly(meth)acrylic acid methyl copolymer, a polyhydroxyethyl (meth)acrylate copolymer, a chitosan derivative, a polylysine derivative, and a combination thereof, which are described in detail elsewhere in the present specification. In addition, one or a plurality of modifiers may also be integrated with the particle core.

As used herein, the term "integration" generally means that substances are put together, and when referring to a modifier and a particle core, the substances may be integrated by any action as long as the substances are in a state of not being dissociated other than a case of being subjected to a condition such as "separable under conditions that do not damage the particle core" and/or "separable under conditions that do not damage the substrate" in an environment in which a sensor for analysis or a substrate for producing a sensor for analysis is produced. Examples of such an action include a covalent bonding or a non-covalent bonding.

As used herein, examples of the "substance used in a sensor for analysis" include, but are not limited to, a substance that is used directly or indirectly in a sensor for analysis or a process of producing the sensor for analysis and contains a group for binding a specific binding molecule to a target substance, a substance containing a reversible linking group, and a group for binding a signal substance.

As used herein, the term "substrate for producing a sensor for analysis" refers to any substrate for producing a sensor for analysis, and any shape and material can be used as long as they are appropriate for the sensor for analysis. Preferably, the group for producing a sensor for analysis is a substrate having a concave portion, and it is advantageous that the group for producing a sensor for analysis is configured so that a user can easily customize an object to be detected to a sensor capable of detecting the objected to be targeted with higher sensitivity by modifying the group for binding a specific binding molecule and the group for binding a signal substance in the concave portion. As used herein, the term "substrate for producing a sensor for analysis" is also referred to as a "measurement substrate".

As used herein, the term "density" refers to the number of substances present per unit area. For example, particles/mm² refers to the number of substances present per mm². Although a method of measuring the density is arbitrary, for example, the density can be measured from a result of observing the surface with a fluorescence microscope or an electron microscope using a particle number measuring function of analysis software attached to a measuring device or free image analysis software such as Image J (https://imagej .nih.gov/ij/).

As used herein, the term "particle integrated with a modifier" refers to a particle in which a modifier is integrated with a particle core, and refers to a particle in which a substance having a group for disposing a group for binding in a molecular imprint concave portion is integrated with the particle.

As used herein, the term "group" refers to a monovalent group unless otherwise specified. Examples of the non-monovalent group include an alkylene group (divalent). In addition, as used as used herein, the term "group" may be omitted.

As used herein, the term "reversible linking group" refers to a direct bond or a divalent or higher valent group in which cleavage and bonding are reversible. Specific examples thereof include, but are not limited to, groups shown in 1-3 of Table 1A below.

**[Table 1A-1]**

| 1 - 1 | 1 - 2 | 1 - 3 | 1 - 4 |
|---|---|---|---|
| Binding functional group | Corresponding binding functional group | Reversible linking group | Mode of bonding of reversible moiety |
| (Group for binding specific binding molecule to target molecule) | (Binding group) | (Reversible linking group) | |
| (Group for binding signal substance) | (Binding group) | | |
| Thiol group, disulfide group, and pyridyl disulfide group | Thiol group, disulfide group, and pyridyl disulfide group | Disulfide group | Covalent bonding |
| Boronyl group | Sugar group | Boronic acid cyclic ester | Covalent bonding |
| Boronyl group | cis-diol group | Boronic acid cyclic ester | Covalent bonding |
| Carbonyl group/aldehyde group | Amino group | Imino binding group | Covalent bonding |
| Amino group | Carbonyl group/aldehyde group | | Covalent bonding |
| Carboxyl group | Hyd roxyl group | Carbonic acid ester group | Covalent bonding |
| Hyd roxyl group | Carboxyl group/carbonic acid activated ester group | | |
| Carboxyl group | Thiol group | Carbonic acid thioester group | Covalent bonding |
| Thiol group | Carboxyl group/carbonic acid activated ester group | | |
| Aminooxy group | Carboxyl group/aldehyde group | Oxime group | Covalent bonding |
| Carbonyl group/aldehyde group | Aminooxy group | | |
| Carboxyl group/aldehyde group | Hydroxyl group | Acetal group | Covalent bonding |
| Hydroxyl group | Carboxyl group/aldehyde group | | |

**[Table 1A-2]**

| Metal complex (Ni-NTA (nickel-nitrilotriacetic acid-derived group) or the like) | Polyhistidine (His) tag (6 ' His, 8 ' His, 10 His, or the like) | Coordination bond | Non-covalent bonding |
|---|---|---|---|
| Basic group (amino group, cyclic secondary amino group (for example, pyrrolidyl group), piperidi group, pyridyl group, imidazole group, guanidine group, or the like) | Acidic group (carboxyl group/carbonic acid activated ester group) | Hydrogen bond | Non-covalent bonding |
| Hydroxyl group | | | |
| Acidic group | Basic group | | Non-covalent bonding |
| (Carboxyl group or the like) | (Amino group, cyclic secondary amino group (for example, pyrrolidyl group or piperidi group), pyridyl group, imidazole group, guanidine group, or the like), hydroxyl group | | |
| Aromatic group (phenyl group of aminophenyl group or the like, naphthyl group of aminonaphthyl group or the like, pyridyl group, or the like) | Aromatic group (phenyl group of aminophenyl group or the like, naphthyl group of aminonaphthyl group or the like, pyridyl group, or the like) | Hydrophobic bond | Non-covalent bonding |
| Hydrazide | Carbonyl group/aldehyde group | Hydrazone binding group | Covalent bonding |
| Carbonyl group/aldehyde group | Hydrazide | | |
| Avidin (neutravidin, streptavidin) | Biotin (biocytin, desbiotin) | Avidin-biotin bond | Non-covalent bonding |
| Biotin (biocytin, desbiotin) | Avidin (neutravidin, streptavidin) | | |
| Glutathione (GHS) | Glutathione-S-transferase (GST) | Glutathione (GHS)-glutathione-s-trans | Non-covalent bonding |

**[Table 1A-3]**

| Glutathione-S-transferase (GST) | Glutathione (GHS) | Ferase (GST) bond | Mode of bonding of reversible moiety |
|---|---|---|---|
| Phos-tag | Phosphate ion | Coordination bond | Non-covalent bonding |
| Phosphate ion | Phos-tag | | |

As used herein, the term "polymer matrix" refers to a polymer that forms a matrix, and usually refers to a substance formed by polymerization of monomers. Advantageously, the matrix may have any shape or structure as long as it has a shape and structure suitable for the sensor when disposed in the sensor for analysis or the substrate for a sensor for analysis of the present disclosure. The shape or structure of the matrix is, for example, a thin film shape or a spherical shape (particle shape). As a preferred configuration of the matrix, a matrix having high biocompatibility is a main component in order to suppress adsorption of substances other than the target as much as possible.

As used herein, the term "molecular imprinted polymer" refers to any polymer used in a molecular imprinting technique (see Takeuchi T. et. al. Chromatography, 2016, 37 (2), 43-64), and preferably refers to a substance recognition material having a binding space for a target substance obtained by forming a complex of a target substance or a derivative thereof and a functional monomer with a covalent bond and/or a non-covalent bond, performing polymerization with a crosslinking agent, and then removing the target substance.

As used herein, the phrase "... at least partially fits into an object" refers to a shape and/or structure that substantially enables detection or analysis of a molecule to be targeted when used in the sensor for analysis, and refers to a shape in which molecules to be targeted can interact within the concave portion when used in the sensor for analysis of the present disclosure. Without wishing to be bound by theory, when a polymer matrix is formed, theoretically, there may be an optimum film thickness that corresponds to less than half the height of the particle core.

As used herein, the term "concave portion" refers to a void or a pore formed so as to capture a target when used in the sensor for analysis of the present disclosure, and preferably refers to a pore portion formed on a polymer matrix formed in the sensor for analysis.

As used herein, the term "group for binding" refers to a group capable of binding to a substance. For example, as used herein, a group for binding a signal substance, a group for binding a specific binding molecule, and the like are used.

As used herein, the term "group for binding a signal substance" refers to a group capable of modifying a signal substance. Examples of the signal substance include a fluorescent molecule, a radioactive element-containing substance, and a magnetic substance. From the viewpoint of ease of detection and the like, the signal substance is preferably a fluorescent substance. Examples of the group for binding a signal substance include binding functional groups listed in 2-1 of Table 2A.

**[Table 2A-1]**

| 2-1 | 2-2 |
|---|---|
| Binding functional group | Corresponding binding functional group |
| (Group for binding specific binding molecule to target molecule) | (Binding group) |
| (Binding group of group for binding signal substance) | (Binding group) |
| Amino group (monovalent amino group, divalent amino group, or the like) | Carbonic acid activated ester group (activated ester group using N-hydroxysuccinimide, nitrophenol, or pentafluorophenol; or carbamic acid activated ester group such as NHS carbamate); carboxyl group; aldehyde group; isocyanate group, isothiocyanate group; epoxy group; maleimide group; and the like |
| Amino group | Carboxyl group |
| Carboxyl group | Amino group |
| Sugar group (cis-diol group) | Boronyl group |
| Boronyl group | Sugar group (cis-diol group) |

**[Table 2A-2]**

| | |
|---|---|
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |
| Carbonyl group/aldehyde group | Amino group |
| Amino group | Carbonyl group/aldehyde group |
| Aminooxy group | Carbonyl group/aldehyde group |
| Carbonyl group/aldehyde group | Aminooxy group |
| Carbonyl group/aldehyde group | Hydroxyl group |
| Hydroxyl group | Carbonyl group/aldehyde group |
| Hydroxyl group and phenolic hydroxyl group | Carbonic acid activated ester group |
| Metal complex (Ni-NTA (nickel-nitrilotriacetic acid-derived group) or the like) | Polyhistidine (His) tag (6 × His, 8 × His, 10 × His, or the like) |
| Hydrazide group | Carbonyl group/aldehyde group |
| Carbonyl group/aldehyde group | Hydrazide |
| Avidin (neutravidin, streptavidin) | Biotin (biocytin, desbiotin) |
| Biotin (biocytin, desbiotin) | Avidin (neutravidin, streptavidin) |
| Glutathione (GSH) | Glutathione-S-transferase |
| Glutathione-S-transferase | Glutathione (GSH) |

As used herein, the term "group for binding a specific binding molecule" refers to a group capable of binding a specific binding molecule capable of specifically binding to a target substance. Examples of the group for binding a specific binding molecule to a target substance include binding functional groups listed in 2-1 of Table 3A.

**[Table 3A-1]**

| 2-1 | 2-2 |
|---|---|
| Binding functional group | Corresponding binding functional group |
| (Group for binding specific binding molecule to target molecule) | (Binding group) |
| (Binding group of group for binding signal substance) | (Binding group) |
| Amino group (monovalent amino group, divalent amino group, or the like) | Carbonic acid activated ester group (activated ester group using N-hydroxysuccinimide, nitrophenol, or pentafluorophenol; or carbamic acid activated ester group such as NHS carbamate); carboxyl group; aldehyde group; isocyanate group, isothiocyanate group; epoxy group; maleimide group; and the like |
| Amino group | Carboxyl group |
| Carboxyl group | Amino group |
| Sugar group (cis-diol group) | Boronyl group |
| Boronyl group | Sugar group (cis-diol group) |
| Thiol group | Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like |
| Electron-deficient unsaturated carbon group such as maleimide group or acrylic acid ester group; iodoacetamide group; thiol group; pyridyl disulfide group; alkene (vinyl sulfone)/alkyne group for radical addition based click chemistry; thioester group for native chemical ligation method; and the like | Thiol group |

**[Table 3A-2]**

| | |
|---|---|
| Carbonyl group/aldehyde group | Amino group |
| Amino group | Carbonyl group/aldehyde group |
| Aminooxy group | Carbonyl group/aldehyde group |
| Carbonyl group/aldehyde group | Aminooxy group |
| Carbonyl group/aldehyde group | Hydroxyl group |
| Hydroxyl group | Carbonyl group/aldehyde group |
| Hydroxyl group and phenolic hydroxyl group | Carbonic acid activated ester group |
| Metal complex (Ni-NTA (nickel-nitrilotriacetic acid-derived group) or the like) | Polyhistidine (His) tag (6 × His, 8 × His, 10 × His, or the like) |
| Hydrazide group | Carbonyl group/aldehyde group |
| Carbonyl group/aldehyde group | Hydrazide |
| Avidin (neutravidin, streptavidin) | Biotin (biocytin, desbiotin) |
| Biotin (biocytin, desbiotin) | Avidin (neutravidin, streptavidin) |
| Glutathione (GSH) | Glutathione-S-transferase |
| Glutathione-S-transferase | Glutathione (GSH) |

As used herein, the term "raw material of a polymer matrix" refers to a raw material capable of forming a polymer matrix by a reaction. In general, in order to form a polymer matrix, it is sufficient to contain at least one monomer and at least one suitable polymerization initiator, but in addition to these, for example, an additional monomer (which may be partially polymerized), an additional polymerization initiator, a crosslinking agent, a RAFT agent, a catalyst, a reducing agent, a solvent, and the like can be contained. Examples of the monomer include, but are not limited to, styrene, N-isopropylacrylamide, and 2-methacryloyloxyethyl phosphorylcholine. Examples of the polymerization initiator include, but are not limited to, 2,2'-azobis(isobutyronitrile) (AIBN) and ethyl α-bromoisobutyrate. Examples of the crosslinking agent include, but are not limited to, a melamine compound, a guanamine compound, a glycoluril compound, N,N'-methylenebisacrylamide, and (tri, tetra, penta, hexa, or poly)ethylene glycol dimethacrylate. Examples of the RAFT agent include, but are not limited thereto, benzyl benzodithioate and 2-cyano-2-[(dodecylsulfanylthiocarbonyl)sulfanyl]propane. Examples of the catalyst include CuBr₂, but are not limited thereto. Examples of the reducing agent include ascorbic acid, but are not limited thereto. The solvent is selected from those generally known as solvents without particular limitation, and examples thereof include pure water, a buffer solution, MeOH, EtOH, DMA, and DMF, but are not limited thereto.

As used herein, the phrase "(the raw material of) the polymer matrix is polymerized" refers to polymerizing (the raw material of) the polymer matrix when the raw material of the polymer matrix is present.

As used herein, the phrase "condition under which the particles are dissociated" refers to a condition under which the particles disposed on the substrate can be separated from the substrate by breaking the interaction between the substrate and the particles. Examples thereof include heating, cooling, pH preparation (an acid treatment or an alkali treatment), washing with a surfactant containing solution, ultrasonic irradiation, light irradiation, shaking, and a reduction treatment.

As used herein, the term "substance required for analysis" refers to a substance capable of capturing and detecting a target substance. Examples thereof include a capturing agent and a label. The capturing agent refers to any agent for capturing an object to be measured, and examples thereof include an antibody, an antibody fragment, an antibody mimetic, a nucleic acid aptamer (DNA, RNA, a peptide nucleic acid, or an artificial nucleic acid), a phospholipid recognition protein, and a lectin.

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. In the present disclosure, when a plurality of (two or more) markers or factors or means for capturing the markers are labeled by a fluorescence method, the markers are labeled with fluorescent substances having different maximum fluorescence emission wavelengths. A difference in maximum fluorescence emission wavelength is preferably 10 nm or more. In the case of labeling a ligand, any fluorescent substance can be used as long as it does not affect the function, and examples of the fluorescent substance include Alexa^{™} Fluor, BODIPY, ATTO, a quantum dot (QDot), and a fluorescent protein (GFP, YFP, mCherry, or the like). Alexa^{™} Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa^{™} 555 and Alexa^{™} 633 and a combination of Alexa^{™} 488 and Alexa^{™} 555. In the case of labeling a nucleic acid, any fluorescent dye can be used as long as it can bind to a base moiety thereof, but it is preferable to use a cyanine dye (for example, Cy3 or Cy5 of CyDyeTM series, or the like), a rhodamine 6G reagent, 2-acetylaminofluorene (AAF), an iodine derivative of AAF (AAIF), or the like. Examples of the fluorescent substance having a difference in maximum fluorescence emission wavelength of 10 nm or more include a combination of Cy5 and a rhodamine 6G reagent, a combination of Cy3 and fluorescein, and a combination of a rhodamine 6G reagent and fluorescein. In the present disclosure, such a label can be utilized to modify the object to be targeted so that the object can be detected by detection means used. Such modifications are known in the art and those skilled in the art can carry out such methods as appropriate for the label and depending on the object to be targeted.

As used herein, the phrase "the coefficient of variation is XX or less" means that a coefficient of variation in detection of a target substance by the sensor for analysis of the present disclosure is a constant value. The coefficient of variation can be measured as a standard deviation/mean value. In the present disclosure, the coefficient of variation may be 50% or less, 40% or less, 30% or less, 20% or less, or 10% or less, and a coefficient of variation of 20% or less may be one preferred embodiment. The coefficient of variation may be in the substrate and/or between the substrates and may satisfy both.

As used herein, the term "at a proportion of YY or more" means that the sensor for analysis of the present disclosure having a performance equal to or higher than a certain numerical value can be manufactured at a proportion of YY or more. As used herein, the performance can be measured using, for example, an automatic dispensing device with a fluorescence detector (manufactured by System Instruments Co., Ltd.), or can be measured using a coefficient of variation in target substance detection. In the present disclosure, it is preferable that the sensors for analysis having the performance can be produced at a proportion of 80% or more.

As used herein, the term "the density of XX to YY pieces/mm² on the substrate" means that a density of particles or concave portions disposed in the substrate is in a range of XX to YY pieces/mm². As used herein, the density is measured using a fluorescence microscope or a scanning electron microscope.

As used herein, the term "spin coating method" refers to a method in which a particle dispersion in which particles are dispersed in a solvent is added onto a substrate using a device that forms a thin film by centrifugal force by rotating a smooth substrate at a high speed, and then the particles are applied onto the substrate by rotating the substrate together.

As used herein, the term "method of dropping particles on a substrate" refers to a method of applying particles on a substrate by dropping a particle dispersion in which particles are dispersed in a solvent on a substrate.

As used herein, the term "method of immersing a substrate in a particle dispersion" refers to a method of applying particles on a substrate by immersing a substrate in a particle dispersion in which particles are dispersed in a solvent.

As used herein, the term "method of pulling up a substrate from a particle dispersion" refers to a method of applying particles on a substrate by pulling up a substrate vertically from a particle dispersion in which particles are dispersed in a solvent.

As used herein, the term "method of spraying a particle dispersion" refers to a method of applying particles on a substrate by spraying a particle dispersion in which particles are dispersed in a solvent to a substrate.

As used herein, the term "signal substance" refers to a substance that can detect a target substance, and is used synonymously with the term "label". Examples thereof include a fluorescent molecule, a radioactive element-containing substance, and a magnetic substance. From the viewpoint of ease of detection and the like, the signal substance is preferably a fluorescent substance.

As used herein, the term "specific" means that a substance that interacts with a target molecule with higher affinity than other substances when referring to affinity, and preferably means not to interact with other substances, and the latter is also referred to as specific in a narrow sense or selectively specific. The specificity can be determined using various intermolecular interaction analysis devices. For example, the measurement can be performed by various analysis devices using surface plasmon resonance (SPR), isothermal titration calorimetry (ITC), or quartz crystal macrobalance (QCM).

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present specification. It is also understood that the following embodiments of the present disclosure may be used alone or in combination.

### <Substrate for producing sensor for analysis>

In one aspect, the present disclosure provides a substrate for producing a sensor for analysis, the substrate including: A) a substrate; and B) particles disposed on the substrate in a monodisperse form. The substrate exhibits excellent properties in terms of stability, efficiency, performance, reproducibility, and the like as the substrate is produced in a form including particles disposed in a monodisperse form.

In one aspect, the present disclosure provides a substrate for producing a sensor for analysis, the substrate including: A) a substrate; and B) particles disposed on the substrate without agglomeration. The substrate exhibits excellent properties in terms of stability, efficiency, performance, reproducibility, and the like as the substrate is produced in a form including particles disposed without agglomeration.

In one aspect, the present disclosure provides a substrate for producing a sensor for analysis, the substrate including: A) a substrate; and B) particles disposed on the substrate in a state of a single layer. The substrate exhibits excellent properties in terms of stability, efficiency, performance, reproducibility, and the like as the substrate is produced in a form including particles disposed in a state of a single layer.

In another aspect, the present disclosure provides a substrate for producing a sensor for analysis, the substrate including: A) a substrate; and B) particles disposed on the substrate at a density of 1 × 10³ particles/mm² to 1 × 10⁶ particles/mm². The substrate exhibits excellent properties in terms of stability, efficiency, performance, reproducibility, and the like as the substrate is produced in a form including particles disposed at a specific density.

A material of the substrate used in the present disclosure may be, for example, a material selected from the group consisting of a metal, a metal oxide, glass, paper (cellulose), a cloth, silicon dioxide, silicon, a resin, and a combination thereof. Examples of the metal include gold, silver, copper, aluminum, titanium, tungsten, and molybdenum. Examples of the resin include poly(meth)acrylate, polystyrene, an acrylonitrile-butadiene-styrene copolymer (ABS), polycarbonate, polyester, polyethylene, polypropylene, nylon, polyurethane, a silicone resin, a fluororesin, a methylpentene resin, a phenol resin, a melamine resin, an epoxy resin, and a vinyl chloride resin.

The substrate used in the present disclosure may be formed by combining a plurality of materials selected from the above-described materials. For example, the substrate may be a substrate in which a metal film is provided on a surface of glass or a resin. In addition, the shape of the substrate may be a plate shape or a particle shape. Preferred examples of the substrate include a gold substrate, a glass substrate, a gold nanoparticle, and a silicon dioxide particle (a silica particle, a glass bead, or the like).

The particle used in the present disclosure may be a biomolecule or a particle core. The particle core is not particularly limited as long as it can be used as a template in molecular imprinting, and examples thereof include an artificially produced inorganic particle and organic particle. Examples of the inorganic particle include a metal (gold, silver, platinum, tin-doped indium oxide (ITO), antimony-doped tin oxide (ATO), or the like), an oxide of a metal (iron oxide, aluminum oxide, copper oxide, titanium oxide, zinc oxide, zirconium oxide, cerium oxide, cobalt oxide, or the like), graphene, graphene oxide, a carbon nanotube, nanodiamond, a nitride, a fluoride, a sulfide, a boride, a complex compound thereof, and hydroxyapatite, and preferably include silicon dioxide (silica). In addition, examples of the organic particle include a latex cured product, dextran, chitosan, polylactic acid, poly(meth)acrylic acid, polymethyl methacrylate, a poly(lactic-co-glycolic acid) (PLGA) copolymer, polystyrene, and polyethyleneimine. In addition, the particle may be a particle in which a binding functional group is bonded to a surface of a biomolecule or a particle core.

A density of the particles present on a surface of the substrate of the present disclosure can be controlled by appropriately preparing a method of disposing the particles, a solvent for dispersing the particles, a particle concentration, an amount of the particle dispersion, a temperature of the particle dispersion, and the like, when disposing the particles. By controlling the density of the particles present on the surface of the substrate, the density of the concave portions of the analysis sensor can also be controlled, and a sensor for analysis exhibiting excellent properties such as stability, efficiency, and performance can be produced.

In one embodiment, in the particles of the present disclosure, particles integrated with a modifier are disposed at a density of preferably about 10³ particles/mm² to about 10⁸ particles/mm², more preferably about 10⁴ particles/mm² to about 10⁷ particles/mm², and still more preferably about 10⁴ particles/mm² to about 10⁶ particles/mm². Examples of an upper limit of the density include about 1 × 10⁷ particles/mm², about 9 × 10⁶ particles/mm², about 8 × 10⁶ particles/mm², about 7 × 10⁶ particles/mm², about 6 × 10⁶ particles/mm², about 5 × 10⁶ particles/mm², about 4 × 10⁶ particles/mm², about 3 × 10⁶ particles/mm², about 2 × 10⁶ particles/mm², and about 1 × 10⁶ particles/mm², examples of a lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × 10⁴ particles/mm², and the particles are disposed at a density of preferably about 10³ particles/mm² to about 10¹⁰ particles/mm², more preferably about 10⁴ particles/mm² to about 10⁹ particles/mm², and still more preferably about 10⁴ particles/mm² to about 10⁸ particles/mm². Examples of the upper limit of the density include about 1 × 10¹⁰ particles/mm², about 9 × 10⁹ particles/mm², about 8 × 10⁹ particles/mm², about 7 × 10⁹ particles/mm², about 6 × 10⁹ particles/mm², about 5 × 10⁹ particles/mm², about 4 × 10⁹ particles/mm², about 3 × 10⁹ particles/mm², about 2 × 10⁹ particles/mm², and about 1 × 10⁹ particles/mm², and examples of the lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × 10⁴ particles/mm²,

In one embodiment, the particles used in the present disclosure include a substance used in the sensor for analysis. The substance used in the sensor for analysis can be a substance required for measurement, and refers to a substance that can be detected. Examples thereof include a capturing agent and a label. The capturing agent refers to any agent for capturing an object to be measured, and examples thereof include an antibody, an antibody fragment, an antibody mimetic, a nucleic acid aptamer (DNA, RNA, a peptide nucleic acid, or an artificial nucleic acid), a phospholipid recognition protein, and a lectin.

In a preferred embodiment, the particles include particles integrated with a modifier.

In another aspect, in the present disclosure, the substrate includes C) a polymer matrix disposed on the substrate body, the polymer matrix having a concave portion that at least partially fits into an object and having the particles disposed in the concave portion, if necessary, in addition to the particles and the substrate, and the particles are disposed in the concave portion. It is understood that the substrate for producing a sensor for analysis can adopt any embodiment described elsewhere herein, for example, in the sections such as <Sensor for analysis> and <Method for producing substrate for producing sensor for analysis>.

### <Sensor for analysis>

In another aspect, the present disclosure provides a sensor for analysis including: A) a substrate body; B) a polymer matrix disposed on the substrate body, the polymer matrix having a concave portion that at least partially fits into an object having the particles disposed in the concave portion, if necessary; C) a group for binding a signal substance disposed on the particle; and D) a group for binding a specific binding molecule that binds to a molecule to be detected, the group being disposed on the particle. It is understood that the sensor can adopt any embodiment described elsewhere herein, for example, in the sections such as the substrate for producing a sensor for analysis.

In one embodiment, the particles used in the present disclosure are preferably disposed on the substrate body at a density of 10³ particles/mm² to 10⁸ particles/mm². The density is preferably about 10³ particles/mm² to about 10⁷ particles/mm², more preferably about 10⁴ particles/mm² to about 10⁷ particles/mm², and still more preferably about 10⁵ particles/mm² to about 10⁶ particles/mm². Examples of the upper limit of the density include about 1 × 10⁷ particles/mm², about 9 × 10⁶ particles/mm², about 8 × 10⁶ particles/mm², about 7 × 10⁶ particles/mm², about 6 × 10⁶ particles/mm², about 5 × 10⁶ particles/mm², about 4 × 10⁶ particles/mm², about 3 × 10⁶ particles/mm², about 2 × 10⁶ particles/mm², and about 1 × 10⁶ particles/mm², and examples of the lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × 10⁴ particles/mm², or it is preferable that the particles are disposed on the substrate body at a density of 10¹⁰ × particles/mm². The density is preferably about 10³ particles/mm² to about 10⁹ particles/mm², more preferably about 10⁴ particles/mm² to about 10⁸ particles/mm², and still more preferably about 10⁵ particles/mm² to about 10⁷ particles/mm². Examples of the upper limit of the density include about 1 × 10¹⁰ particles/mm², about 9 × 10⁹ particles/mm², about 8 × 10⁹ particles/mm², about 7 × 10⁹ particles/mm², about 6 × 10⁹ particles/mm², about 5 × 10⁹ particles/mm², about 4 × 10⁹ particles/mm², about 3 × 10⁹ particles/mm², about 2 × 10⁹ particles/mm², and about 1 × 10⁹ particles/mm², and examples of the lower limit of the density include about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², and about 1 × 10⁴ particles/mm². When the density is smaller than the range, the measurement cannot be performed with sufficient sensitivity to detect the object to be detected, and when the density is larger than the range, the particles are agglomerated, and the sensor may not be produced with excellent reproducibility. A preferred density of the monodispersed or non-agglomerated concave portions may be 10⁴ particles/mm² to 10⁶ particles/mm²,

### <Substrate for producing sensor for analysis and method for producing sensor for analysis>

In another aspect, the present disclosure provides a method for producing a substrate for producing a sensor for analysis. The method includes A) a step of providing particles of the present disclosure; B) a step of adding the particles to a substrate so that the particles are disposed on a substrate body in a monodisperse form; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a concave portion by subjecting the substrate to a condition under which the particles are dissociated from the substrate.

In another aspect, the present disclosure provides a method for producing a substrate for producing a sensor for analysis. The method includes A) a step of providing particles of the present disclosure; B) a step of adding the particles to a substrate so that the particles are disposed on a substrate body without agglomeration; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a concave portion by subjecting the substrate to a condition under which the particles are dissociated from the substrate.

In another aspect, the present disclosure provides a method for producing a substrate for producing a sensor for analysis. The method includes A) a step of providing particles of the present disclosure; B) a step of adding the particles to a substrate so that the particles are disposed on a substrate body in a single layer; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; and E) a step of forming a concave portion by subjecting the substrate to a condition under which the particles are dissociated from the substrate.

In the present disclosure, in the step of adding the particles so that the particles are disposed on the substrate body in a monodisperse form and/or without agglomeration and/or in a single layer, it is preferable that the particles disposed in a monodisperse form and/or without agglomeration and/or in a single layer are added so that the particles maintain the state after polymerizing the polymer matrix. The dispersibility can be appropriately maintained or modified by providing an appropriate modifier and/or setting appropriate conditions, and therefore, the particles can maintain the state after polymerizing the polymer matrix, and those skilled in the art can appropriately carry out the polymerization based on the contents described in the Examples and the like in the present specification.

Specifically, in the present disclosure, the step of adding the particles so that the particles are disposed in a monodisperse form and/or without agglomeration and/or in a single layer includes adding particles to a substrate at a concentration of 10 × 10⁰ particles/µL to 2.5 × 10¹⁰ particles/µL or in an amount of 1.0 × 10⁰ particles/mm² to 2.5 × 10¹⁰ particles/mm² per surface area of the substrate. Examples of an upper limit of the concentration include about 1 × 10¹⁰ particles/µL, about 9 × 10⁹ particles/µL, about 8 × 10⁹ particles/µL, about 7 × 10⁹ particles/µL, about 6 × 10⁹ particles/µL, about 5 × 10⁹ particles/µL, about 4 × 10⁹ particles/µL, about 3 × 10⁹ particles/µL, about 2 × 10⁹ particles/µL, about 1 × 10⁹ particles/µL, about 9 × 10⁸ particles/µL, about 8 × 10⁸ particles/µL, about 7 × 10⁸ particles/µL, about 6 × 10⁸ particles/µL, about 5 × 10⁸ particles/µL, about 4 × 10⁸ particles/µL, about 3 × 10⁸ particles/µL, about 2 × 10⁸ particles/µL, about 1 × 10⁸ particles/µL, about 1 × 10⁸ particles/µL, about 9 × 10⁷ particles/µL, about 8 × 10⁷ particles/µL, about 7 × 10⁷ particles/µL, about 6 × 10⁷ particles/µL, about 5 × 10⁷ particles/µL, about 4 × 10⁷ particles/µL, about 3 × 10⁷ particles/µL, about 2 × 10⁷ particles/µL, about 1 × 10⁷ particles/µL, about 9 × 10⁶ particles/µL, about 8 × 10⁶ particles/µL, about 7 × 10⁶ particles/µL, about 6 × 10⁶ particles/µL, about 5 × 10⁶ particles/µL, about 4 × 10⁶ particles/µL, about 3 × 10⁶ particles/µL, about 2 × 10⁶ particles/µL, about 1 × 10⁶ particles/µL, about 9 × 10⁵ particles/µL, about 8 × 10⁵ particles/µL, about 7 × 10⁵ particles/µL, about 6 × 10⁵ particles/µL, about 5 × 10⁵ particles/µL, about 4 × 10⁵ particles/µL, about 3 × 10⁵ particles/µL, and about 2 × 10⁵ particles/µL, about 1 × 10⁵ particles/µL, and examples of a lower limit of the concentration include about 1 × 10⁰ particles/µL, about 2 × 10⁰ particles/µL, about 3 × 10⁰ particles/µL, about 4 × 10⁰ particles/µL, about 5 × 10⁰ particles/µL, about 6 × 10⁰ particles/µL, about 7 × 10⁰ particles/µL, about 8 × 10⁰ particles/µL, about 9 × 10⁰ particles/µL, about 1 × 10¹ particles/µL, about 2 × 10¹ particles/µL, about 3 × 10¹ particles/µL, about 4 × 10¹ particles/µL, about 5 × 10¹ particles/µL, about 6 × 10¹ particles/µL, about 7 × 10¹ particles/µL, about 8 × 10¹ particles/µL, about 9 × 10¹ particles/µL, about 1 × 10² particles/µL, about 2 × 10² particles/µL, about 3 × 10² particles/µL, about 4 × 10² particles/µL, about 5 × 10² particles/µL, about 6 × 10² particles/µL, about 7 × 10² particles/µL, about 8 × 10² particles/µL, about 9 × 10² particles/µL, about 1 × 10³ particles/µL, about 2 × 10³ particles/µL, about 3 × 10³ particles/µL, about 4 × 10³ particles/µL, about 5 × 10³ particles/µL, about 6 × 10³ particles/µL, about 7 × 10³ particles/µL, about 8 × 10³ particles/µL, about 9 × 10³ particles/µL, about 1 × 10⁴ particles/µL, about 2 × 10⁴ particles/µL, about 3 × 10⁴ particles/µL, about 4 × 10⁴ particles/µL, about 5 × 10⁴ particles/µL, about 6 × 10⁴ particles/µL, about 7 × 10⁴ particles/µL, about 8 × 10⁴ particles/µL, about 9 × 10⁴ particles/µL, and about 1 × 10⁵ particles/µL. Examples of an upper limit of the amount per surface area of the substrate in unit of mm² include about 1 × 10¹⁰ particles/mm², about 9 × 10⁹ particles/mm², about 8 × 10⁹ particles/mm², about 7 × 10⁹ particles/mm², about 6 × 10⁹ particles/mm², about 5 × 10⁹ particles/mm², about 4 × 10⁹ particles/mm², about 3 × 10⁹ particles/mm², about 2 × 10⁹ particles/mm², about 1 × 10⁹ particles/mm², about 9 × 10⁸ particles/mm², about 8 × 10⁸ particles/mm², about 7 × 10⁸ particles/mm², about 6 × 10⁸ particles/mm², about 5 × 10⁸ particles/mm², about 4 × 10⁸ particles/mm², about 3 × 10⁸ particles/mm², about 2 × 10⁸ particles/mm², about 1 × 10⁸ particles/mm², about 1 × 10⁸ particles/mm², about 9 × 10⁷ particles/mm², about 8 × 10⁷ particles/mm², about 7 × 10⁷ particles/mm², about 6 × 10⁷ particles/mm², about 5 × 10⁷ particles/mm², about 4 × 10⁷ particles/mm², about 3 × 10⁷ particles/mm², about 2 × 10⁷ particles/mm², about 1 × 10⁷ particles/mm², about 9 × 10⁶ particles/mm², about 8 × 10⁶ particles/mm², about 7 × 10⁶ particles/mm², about 6 × 10⁶ particles/mm², about 5 × 10⁶ particles/mm², about 4 × 10⁶ particles/mm², about 3 × 10⁶ particles/mm², about 2 × 10⁶ particles/mm², about 1 × 10⁶ particles/mm², about 9 × 10⁵ particles/mm², about 8 × 10⁵ particles/mm², about 7 × 10⁵ particles/mm², about 6 × 10⁵ particles/mm², about 5 × 10⁵ particles/mm², about 4 × 10⁵ particles/mm², about 3 × 10⁵ particles/mm², about 2 × 10⁵ particles/mm², and about 1 × 10⁵ particles/mm², and examples of a lower limit of the amount per surface area of the substrate in unit of mm² include about 1 × 10⁰ particles/mm², about 2 × 10⁰ particles/mm², about 3 × 10⁰ particles/mm², about 4 × 10⁰ particles/mm², about 5 × 10⁰ particles/mm², about 6 × 10⁰ particles/mm², about 7 × 10⁰ particles/mm², about 8 × 10⁰ particles/mm², about 9 × 10⁰ particles/mm², about 1 × 10¹ particles/mm², about 2 × 10¹ particles/mm², about 3 × 10¹ particles/mm², about 4 × 10¹ particles/mm², about 5 × 10¹ particles/mm², about 6 × 10¹ particles/mm², about 7 × 10¹ particles/mm², about 8 × 10¹ particles/mm², about 9 × 10¹ particles/mm², about 1 × 10² particles/mm², about 2 × 10² particles/mm², about 3 × 10² particles/mm², about 4 × 10² particles/mm², about 5 × 10² particles/mm², about 6 × 10² particles/mm², about 7 × 10² particles/mm², about 8 × 10² particles/mm², about 9 × 10² particles/mm², about 1 × 10³ particles/mm², about 2 × 10³ particles/mm², about 3 × 10³ particles/mm², about 4 × 10³ particles/mm², about 5 × 10³ particles/mm², about 6 × 10³ particles/mm², about 7 × 10³ particles/mm², about 8 × 10³ particles/mm², about 9 × 10³ particles/mm², about 1 × 10⁴ particles/mm², about 2 × 10⁴ particles/mm², about 3 × 10⁴ particles/mm², about 4 × 10⁴ particles/mm², about 5 × 10⁴ particles/mm², about 6 × 10⁴ particles/mm², about 7 × 10⁴ particles/mm², about 8 × 10⁴ particles/mm², about 9 × 10⁴ particles/mm², and about 1 × 10⁵ particles/mm². As one embodiment, the upper limit may be 1.0 × 10¹⁰ particles/(µL·mm²) to 1.0 × 10⁴ particles/(µL·mm²), and the lower limit may be 1.0 × 10⁰ particles/(µL·mm²) to 1.0 × 10⁴ particles/(µL·mm²).

In the present disclosure, the step of disposing the particles on the substrate can be performed by any method. For example, the particles of the present disclosure are dispersed in a solution, dropped on the substrate, and disposed by being allowed to stand or spin-coated.

In the present disclosure, the step of providing the raw material of the polymer matrix to the substrate on which the particles are immobilized can be performed by any method, for example, by adding a polymerizable monomer and providing a polymerizable functional group and a polymerizable monomer derived from the particle or the functional monomer as a substrate.

In the present disclosure, the raw material of the polymer matrix may be any material that is suitable for the intended analysis. In general, the material of the polymer matrix is sufficient to contain at least one monomer and at least one suitable polymerization initiator, but in addition to these, for example, an additional monomer (which may be partially polymerized), an additional polymerization initiator, a crosslinking agent, a RAFT agent, a catalyst, a reducing agent, a solvent, and the like can be contained. Examples of the monomer include, but are not limited to, styrene, N-isopropylacrylamide, and 2-methacryloyloxyethyl phosphorylcholine. Examples of the polymerization initiator include, but are not limited to, 2,2'-azobis(isobutyronitrile) (AIBN) and ethyl α-bromoisobutyrate. Examples of the crosslinking agent include, but are not limited to, a melamine compound, a guanamine compound, a glycoluril compound, N,N'-methylenebisacrylamide, and (tri, tetra, penta, hexa, or poly)ethylene glycol dimethacrylate. Examples of the RAFT agent include, but are not limited thereto, benzyl benzodithioate and 2-cyano-2-[(dodecylsulfanylthiocarbonyl)sulfanyl]propane. Examples of the catalyst include CuBr₂, but are not limited thereto. Examples of the reducing agent include ascorbic acid, but are not limited thereto. The solvent is selected from those generally known as solvents without particular limitation, and examples thereof include pure water, a buffer solution, MeOH, EtOH, DMA, and DMF, but are not limited thereto. It is understood that the particles and the step of disposing the particles on the substrate in a monodisperse form and/or without agglomeration and/or in a single layer can adopt any embodiment described elsewhere herein, for example, in the sections such as <Sensor for analysis> and <Method for producing substrate for producing sensor for analysis>.

In the present disclosure, the step of forming the substrate on which the polymer matrix is disposed by subjecting the substrate to a condition under which the polymer matrix is polymerized can be performed by any method. For example, a polymerizable monomer is added, a polymerizable functional group and a polymerizable monomer derived from the particle or the functional monomer are used as a substrate, and a molecularly imprinted polymer for a part of the surface of the particle of the present disclosure is synthesized using a polymerization initiating group as a polymerizable initiator. As a result, a polymer matrix having a concave portion can be formed on the surface of the substrate.

In the present disclosure, the step of forming the concave portion by subjecting the substrate to a condition under which the particles are dissociated from the substrate can be performed by any step, and for example, the particles of the present disclosure can be removed by cleaving a reversible linking group.

In the examples of the present disclosure, a large number of examples of the examples shown in the following table are demonstrated.

**[Table 11A-1]**

| 1 - 1 | 1-2 | 1 - 3 | 1-4 |
|---|---|---|---|
| Binding functional group | Corresponding binding functional group | Reversible linking group | Mode of bonding of reversible moiety |
| (Group for binding specific binding molecule to target molecule) | (Binding group) | (Reversible linking group) | |
| (Group for binding signal substance) | (Binding group) | | |
| Thiol group, disulfide group, and pyridyl disulfide group | Thiol group, disulfide group, and pyridyl disulfide group | Disulfide group | Covalent bonding |
| Boronyl group | Sugar group | Boronic acid cyclic ester | Covalent bonding |
| Boronyl group | cis-diol group | Boronic acid cyclic ester | Covalent bonding |
| Carbonyl group/aldehyde group | Amino group | Imino binding group | Covalent bonding |
| Amino group | Carbonyl group/aldehyde group | | Covalent bonding |
| Carboxyl group | Hydroxyl group | Carbonic acid ester group | Covalent bonding |
| Hydroxyl group | Carboxyl group/carbonic acid activated ester group | | |
| Carboxyl group | Thiol group | Carbonic acid thioester group | Covalent bonding |
| Thiol group | Carboxyl group/carbonic acid activated ester group | | |
| Aminooxy group | Carboxyl group/aldehyde group | Oxime group | Covalent bonding |
| Carbonyl group/aldehyde group | Aminooxy group | | |
| Carboxyl group/aldehyde group | Hydroxyl group | Acetal group | Covalent bonding |
| Hydroxyl group | Carboxyl group/aldehyde group | | |

**[Table 11A-2]**

| Metal complex (Ni-NTA (nickel-nitrilotriacetic acid-derived group) or the like) | Polyhistidine (His) tag (6 × His, 8 × His, 10 × His, or the like) | Coordination bond | Non-covalent bonding |
|---|---|---|---|
| Basic group (amino group, cyclic secondary amino group (for example, pyrrolidyl group), piperidi group, pyridyl group, imidazole group, guanidine group, or the like) | Acidic group (carboxyl group/carbonic acid activated ester group) | Hydrogen bond | Non-covalent bonding |
| Hydroxyl group | | | |
| Acidic group | Basic group | | Non-covalent bonding |
| (Carboxyl group or the like) | (Amino group, cyclic secondary amino group (for example, pyrrolidyl group or piperidi group), pyridyl group, imidazole group, guanidine group, or the like), hydroxyl group | | |
| Aromatic group (phenyl group of aminophenyl group orthe like, naphthyl group of aminonaphthyl group or the like, pyridyl group, or the like) | Aromatic group (phenyl group of aminophenyl group or the like, naphthyl group of aminonaphthyl group or the like, pyridyl group, or the like) | Hydrophobic bond | Non-covalent bonding |
| Hydrazide | Carbonyl group/aldehyde group | Hydrazone binding group | Covalent bonding |
| Carbonyl group/aldehyde group | Hydrazide | | |
| Avidin (neutravidin, streptavidin) | Biotin (biocytin, desbiotin) | Avidin-biotin bond | Non-covalent bonding |
| Biotin (biocytin, desbiotin) | Avidin (neutravidin, streptavidin) | | |
| Glutathione (GSH) | Glutathione-S-transferase (GST) | Glutathione (GSH)-glutathione-s-trans | Non-covalent bonding |

**[Table 11A-3]**

| Glutathione-S-transferase (GST) | Glutathione (GSH) | Ferase (GST) bond | Mode of bonding of reversible moiety |
|---|---|---|---|
| Phos-tag | Phosphate ion | Coordination bond | Non-covalent bonding |
| Phosphate ion | Phos-tag | | |

In another aspect, the present disclosure provides a method for producing a sensor for analysis. The method includes A) a step of providing particles; B) a step of adding the particles to a substrate so that the particles are disposed on a substrate body in a monodisperse form; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; E) a step of forming a concave portion by subjecting the substrate to a condition under which the particles are dissociated from the substrate; and F) a step of binding a substance required for analysis to the concave portion. The particles, the step of disposing the particles on the substrate in a monodisperse form, the conditions for polymerization of the polymer matrix, and the conditions for dissociation of the particles can adopt any embodiment described elsewhere herein, for example, in the sections such as <Sensor for analysis> and <Method for producing sensor for analysis>.

In another aspect, the present disclosure provides a method for producing a sensor for analysis. The method includes A) a step of providing particles; B) a step of adding the particles to a substrate so that the particles are disposed on a substrate body without agglomeration; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; E) a step of forming a concave portion by subjecting the substrate to a condition under which the particles are dissociated from the substrate; and F) a step of binding a substance required for analysis to the concave portion. The particles, the step of disposing the particles on the substrate without agglomeration, the conditions for polymerization of the polymer matrix, and the conditions for dissociation of the particles can adopt any embodiment described elsewhere herein, for example, in the sections such as <Sensor for analysis> and <Method for producing sensor for analysis>.

In another aspect, the present disclosure provides a method for producing a sensor for analysis. The method includes A) a step of providing particles; B) a step of adding the particles to a substrate so that the particles are disposed on a substrate body in a single layer; C) a step of providing a raw material of a polymer matrix to the substrate on which the particles are immobilized, if necessary; D) a step of forming a substrate on which the polymer matrix is disposed by subjecting the substrate to a condition under which the polymer matrix is polymerized, if necessary; E) a step of forming a concave portion by subjecting the substrate to a condition under which the particles are dissociated from the substrate; and F) a step of binding a substance required for analysis to the concave portion. The particles, the step of disposing the particles on the substrate in a single layer, the conditions for polymerization of the polymer matrix, and the conditions for dissociation of the particles can adopt any embodiment described elsewhere herein, for example, in the sections such as <Sensor for analysis> and <Method for producing sensor for analysis>.

In one embodiment, the step of binding the substance required for analysis to the concave portion can be performed by any method, for example, by modifying a cleaved reversible linking group produced in the concave portion by reduction with a group for binding a specific binding molecule and a group for binding a signal substance.

In one embodiment, the present disclosure provides a method for producing a sensor for analysis of the present disclosure in which the sensor for analysis having a coefficient of variation that is equal to or less than a certain reference value (for example, 20%) is produced by forming concave portions having a density of 2.0 × 10⁷ pieces/mm² or less. The coefficient of variation may occur either in the substrate or between the substrates, but at least one of them may be 20% or less, and it is preferable that both of them are 20% or less.

The density of the particles in the step of disposing the particles in a monodisperse form, without agglomeration, and/or in a single layer may be about 10³ particles/mm² to about 10⁸ particles/mm², preferably about 10³ particles/mm² to about 10⁷ particles/mm², more preferably about 10⁴ particles/mm² to about 10⁷ particles/mm², or about 10³ particles/mm² to about 10¹⁰ particles/mm², and is preferably about 10³ particles/mm² to about 10⁹ particles/mm², and more preferably about 10⁴ particles/mm² to about 10⁸ particles/mm². In one embodiment, the step of disposing the particles in a monodisperse form and/or without agglomeration and/or in a single layer includes a step of disposing the particles of the present disclosure on a substrate at a density of 10³ particles/mm² to 10¹⁰ particles/mm². A preferred density of the particles disposed on the substrate may be 10⁴ particles/mm² to 10⁷ particles/mm². The density here can be confirmed from surface observation with a fluorescence microscope or a scanning electron microscope.

In one embodiment, there is provided a method including a step of forming concave portions of the present disclosure, in which the monodispersed or non-agglomerated concave portions are present at a density of 10³ pieces/mm² to 10¹⁰ pieces/mm². A preferred density of the concave portions disposed on the substrate may be 10³ pieces/mm² to 10⁷ pieces/mm². In addition, the density here can be confirmed from surface observation with a fluorescence microscope or a scanning electron microscope.

In one embodiment, the step of disposing the particles in a monodisperse form and/or without agglomeration and/or in a single layer can be performed by any method, and includes, for example, a spin coating method, a method of dropping on a substrate, a method of immersing in a particle dispersion, a method of pulling up a substrate from a particle dispersion, a method of spraying (applying) a particle dispersion, or a method of printing by an inkjet printer.

### (Production example of sensor for analysis)

The step for producing the sensor for analysis can be performed as follows. The particles of the present disclosure are dispersed in a solution, dropped on a substrate, and disposed by being allowed to stand or spin-coated. A polymerizable monomer is added, and a polymerizable functional group and a polymerizable monomer derived from the particle or the functional monomer are used as a substrate. A molecularly imprinted polymer for a portion of the surface of the particle of the present disclosure is synthesized using a polymerizable initiating group as a polymerizable initiator. As a result, a polymer matrix having a concave portion is formed on the surface of the substrate. The particles of the present disclosure are removed by weakening (or dissociating) the interaction between the particle core and the modifier. The reversible linking group that is present (or exposed) in the concave portion is modified with the group for binding a specific binding molecule and the group for binding a signal substance.

### (Use)

The sensor for analysis of the present disclosure is used for sensing an object to be detected. The more specific use is determined depending on the type of the specific binding group, and for example, the sensor for analysis can be used for the purpose of diagnosis or therapeutic monitoring based on renal function, liver function, and the presence or absence or a degree of inflammation; and the presence or absence or a degree of tumor.

The sensor for analysis of the present disclosure can be used for the following use.

The sensor for analysis can be used for in vivo/in vitro imaging of an object to be measured, a therapeutic application, a virus sensor in a human body or environment, and analysis of food, an agricultural product, and livestock.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described herein, but is limited only by the claims.

### Examples

In the present examples, examples related to production and use of the compound of the present disclosure will be described. Note that CKX31 (OLYMPUS, Tokyo, Japan) was used for microscopic observation of cells, KUBOTA2800 (KUBOTA, Tokyo, Japan) was used for a centrifuge, CO2 WATER JACKETED INCUBATOR (Thermo Fisher Scientific Inc, Massachusetts, USA) was used for an incubator, KS-243 (TOMY SEIKO Co,Ltd., Tokyo, Japan) was used for an autoclave, and Bio Clean Bench (ORIENTAL GIKEN INC, Tokyo, Japan) was used for a clean bench. qNano Gold (Izon Science Ltd., Christchurch, New Zealand) was used for exosome concentration measurement. In addition, UV Ozone Cleaner (BioForce Nanosciences, Inc.) was used for a UV ozone treatment of a gold substrate, a fluorescence microscope (Olympus Corporation, Tokyo, Japan) with an SIC automatic dispensing device (SYSTEM INSTRUMENTS Co., Ltd., Tokyo, Japan) was used for fluorescence measurement, and Andor SOLIS (Andor Technology Ltd, Belfast, Northern Ireland) was used as spectroscopic software.

In addition, MALDI-TOF/MS (MALDI-7090, Shimadzu Corporation) was used for MALDI-TOF-MS, MALDI Solutions (Shimadzu Corporation) was used for analysis software, and protein calibration standard I (Bruker Corporation) was used for calibration. Sinapinic acid was used as a matrix. For a CD spectrum, a J-725 type circular dichroism spectropolarimeter (JASCO Corporation, Tokyo, Japan) was used. A pH was measured with a tabletop pH meter F-52 (HORIBA, Kyoto, Japan) for buffer preparation. Fluorescence spectrum measurement was performed using a fluorescence spectrophotometer F-2500 (Tokyo, Japan) manufactured by Hitachi High-Tech Corporation. As an ultrafiltration membrane used for ultrafiltration, Amicon Ultra-4 (10 kDa) was used. For absorption spectrum measurement, a Thermo ScientificTM Nano drop TM One ultra trace ultraviolet-visible spectrophotometer (Thermo Fisher) was used. An average particle size and a polydispersity index (PDI) of the produced particles were measured by a dynamic light scattering (DLS) method using ZETASIZER NANO-ZS MAL500735 (Malvern, UK), and Zetasizer software was used as analysis software. A transmission electron microscope (JEM-1230, manufactured by JEOL Ltd.) was used for TEM analysis.

### (Example 1: Synthesis of modifier)

### 1. Experimental operation

### 1-1. Synthesis of polymer

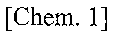

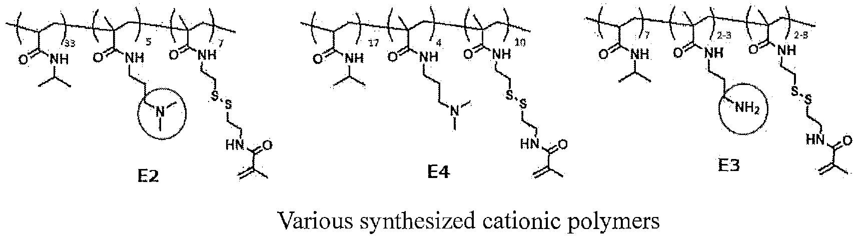

### 1-1-1. Synthesis of polymer E2

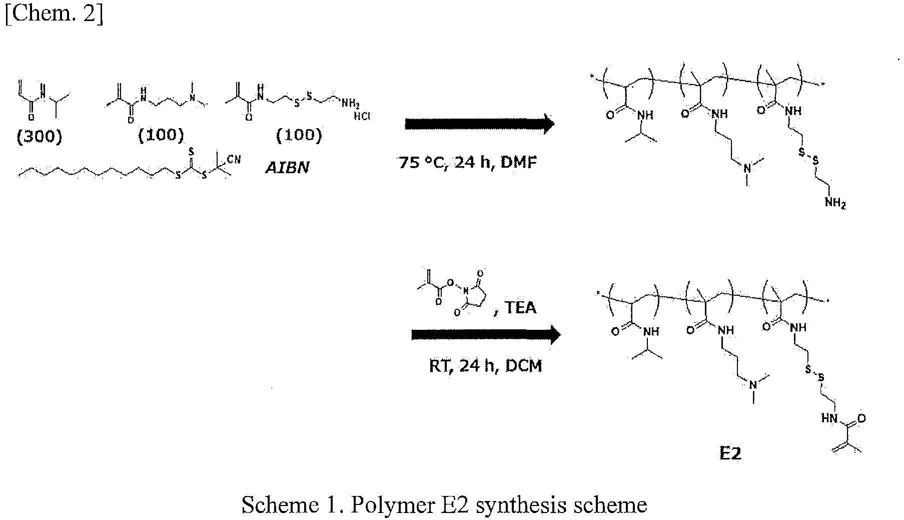

Reagents of the recipes shown in Table 1-1 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer

### (E2-0). (Yield: 100 mg)

100 mg of the obtained polymer was dispersed in a dichloromethane/dioxane = 1/1 (v/v) solution (10 mL), 92 mg (0.5 mmol) of N-succinimidyl methacrylate and 140 µL (1.0 mmol) of triethylamine (TEA) were added thereto, and stirring was performed at room temperature for 24 hours. The solvent after the reaction was distilled off under reduced pressure with an evaporator, a small amount of dichloromethane was added to the residue, and a precipitate generated by further adding an excessive amount of hexane was collected (this operation was performed twice). The obtained precipitate was vacuum-dried to obtain a polymer E2. (Yield: 101 mg)

A composition of the obtained polymer E2 was estimated from ¹H-NMR (in DMSO-d6) (AVANCE-500, Bruker). (FIG. 1-1)

**[Table 1-1]**

| Table 1-1 Polymer E2 synthesis recipe | | |
|---|---|---|
| | Sample | Concentration |
| Main raw material | N-isopropylacrylamide (NIPAm) | 300 mM |
| Cationic site | N-(3-Dimethylaminopropyl)methacrylamide hydrochloride | 100 mM |
| Modified site | Cys-methacrylamide hydrochloride | 100 mM |
| RAFT agent | 2-Cyano-2-[(dodecylsulfanylthiocarbonyl)sulfanyl]propane | 12.5mM |
| Initiator | 2,2'-Azobis(isobutyronitrile) (AIBN) | 6.25 mM |
| Solvent | DMF | 2 mL |

### 1-1-2. Synthesis of polymer E3

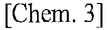

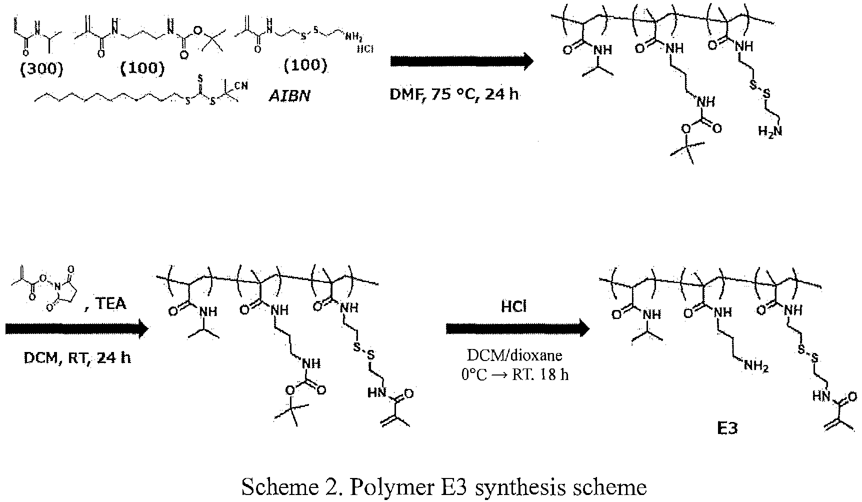

Reagents of the recipes shown in Table 1-2 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer.

### (Yield: 105 mg)

The polymer was dispersed in dichloromethane (10 mL), 110 mg (0.6 mmol) of N-succinimidyl methacrylate and 140 µL (1.0 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain a polymer E3. (Yield: 115 mg)

The obtained polymer was dispersed in dichloromethane (6 mL), 1.0 mL of 4 N HCl in dioxane was added thereto, and stirring was performed under ice cooling for 2 hours. Thereafter, stirring was further performed at room temperature overnight. A precipitate generated by adding hexane to the solution after the reaction was collected. A small amount of dichloromethane was added to the collected precipitate, an excessive amount of hexane was further added, and the precipitate was washed. The collected precipitate was vacuum-dried. (Yield: 105 mg)

A composition of the obtained polymer E3 was estimated from ¹H-NMR (in DMSO-d6) (AVANCE-500, Bruker). (FIG. 1-2)

**[Table 1-2]**

| Table 1-2 Polymer E3 synthesis recipe | | |
|---|---|---|
| | Sample | Concentration |
| Main raw material | N-isopropylacrylamide (NIPAm) | 300 mM |
| Cationic site | N-Boc-(3-aminopropyl)methacrylamide hydrochloride | 100 mM |
| Modified site | Cys-methacrylamide | 100 mM |
| RAFT agent | 2-Cyano-2-[(dodecylsulfanylthiocarbonyl)sulfanyl]propane | 12.5mM |
| Initiator | 2,2'-Azobis(isobutyronitrile) (AIBN) | 6.25 mM |
| Solvent | DMF | 2 mL |

### 1-1-3. Synthesis of polymer E4

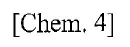

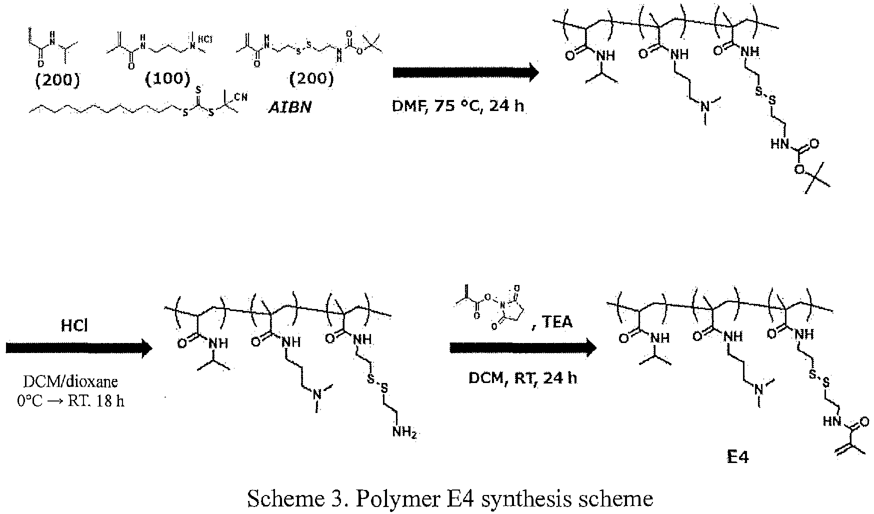

Reagents of the recipes shown in Table 1-3 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer.

### (Yield: 70 mg)

The obtained polymer was dispersed in dichloromethane (10 mL), 1.0 mL of 4 N HCl in dioxane was added thereto, and stirring was performed under ice cooling for 2 hours. Thereafter, stirring was further performed at room temperature overnight. A precipitate generated by adding hexane to the solution after the reaction was collected. A small amount of dichloromethane was added to the collected precipitate, an excessive amount of hexane was further added, and the precipitate was washed. The collected precipitate was vacuum-dried. Thereafter, the polymer was dispersed in dichloromethane (10 mL), 73 mg (0.4 mmol) of N-succinimidyl methacrylate and 84 µL (0.6 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain E4. (Yield: 62 mg)

A composition of the obtained E4 was estimated from ¹H-NMR (in DMSO-d6) (AVANCE-500, Bruker). (FIG. 1-3)

**[Table 1-3]**

| Table 1-3 Polymer E4 synthesis recipe | | |
|---|---|---|
| | Sample | Concentration |
| Main raw material | N-isopropylacrylamide (NIPAm) | 200 mM |
| Cationic site | N-(3-Dimethylaminopropyl)methacrylamide hydrochloride | 100 mM |
| Modified site | Cys-methacrylamide hydrochloride | 200 mM |
| RAFT agent | 2-Cyano-2-[(dodecylsulfanylthiocarbonyl)sulfanyl]propane | 12. 5mM |
| Initiator | 2,2'-Azobis(isobutyronitrile) (AIBN) | 6.25 mM |
| Solvent | DMF | 2 mL |

### 1-2-1 Synthesis of polymer F1

Reagents of the recipes shown in Table 1-4 were dissolved in DMF (2 mL), degassed and replaced with argon, and then subjected to a polymerization reaction in an oil bath (75°C) for 24 hours. After the solution after the reaction was cooled and brought into contact with air to stop the reaction, the reaction solution was dropped to diethyl ether to generate a precipitate, and the precipitate was collected. This operation was repeated twice. The collected precipitate was vacuum-dried to obtain a polymer.

### (Yield: 105.7 mg)

The obtained polymer was dispersed in dichloromethane (5 mL), 34.1 mg (0.19 mmol) of N-succinimidyl methacrylate and 38.9 µL (0.28 mmol) of triethylamine (TEA) were added thereto, and stirring was performed at room temperature for 24 hours. A precipitate generated by adding an excessive amount of hexane to the solution after the reaction was collected (this operation was performed twice). The obtained precipitate was vacuum-dried to obtain F1. (Yield: 19.1 mg)

A composition of the obtained F1 was estimated from ¹H-NMR (in DMSO-d6) (AVANCE-500, Bruker).

**[Table 1-4]**

| Table 1-4 Fluorescent polymer F1 synthesis recipe | | |
|---|---|---|
| | Sample | Concentration |
| Main raw material | N-isopropylacrylamide (NIPAm) | 275 mM |
| Cationic site | N-(3-Dimethylaminopropyl)methacrylamide hydrochloride | 100 mM |
| Modified site | Cys-methacrylamide hydrochloride | 100 mM |
| Fluorescent site | Methacryloxyethyl thiocarbamoyl rhodamine B | 25 mM |
| RAFT agent | 2-Cyano-2-[(dodecylsulfanylthiocarbonyl)sulfanyl]propane | 12. 5mM |
| Initiator | 2,2'-Azobis(isobutyronitrile): V-60 | 6.25 mM |
| Solvent | DMF | 2 mL |

### Synthesis of polymer (AN1)

64 mg of Boc-cystamine metacrylamide, 29 µL of t-butyl acrylate, and 68 mg of NIPAm were dissolved in 1.8 mL of DMF, and finally, 0.2 mL of DMF in which 8.6 mg of a RAFT agent (2-cyano-2-[(dodecylsulfanylthiocarbonyl)sulfanyl]propane) and 2.1 mg of an initiator (AIBN) were dissolved was added thereto, and degassing and replacement with argon were performed to remove dissolved oxygen. Thereafter, a polymerization reaction was performed in an oil bath set at 75°C (24 h). After the reaction, the reaction solution was dropped to an excessive amount of diethyl ether, and a precipitate was collected. (Yield: 51 mg)

The obtained polymer was dissolved in dichloromethane (5 mL) and stirring was performed under ice cooling. 1.5 mL of trifluoroacetic acid was added thereto, and stirring was performed overnight under light-shielding. After the reaction, a precipitate generated by adding diethyl ether was collected, and the obtained precipitate was washed with diethyl ether. (Yield: 22 mg)

The obtained polymer was dispersed in dichloromethane, 15 mg (0.08 mmol) of N-succinimidyl methacrylate and 17 µL (0.12 mmol) of triethylamine were added thereto, and stirring was performed at room temperature overnight. A precipitate generated by adding diethyl ether to the reaction solution was collected. The precipitate was dissolved in a small amount of dichloromethane and precipitated by adding an excessive amount of hexane thereto. This operation was repeated twice, and vacuum-drying was performed to obtain a target polymer (AN1). (Yield: 16 mg)

### 1-1. Synthesis of polymer (E101)

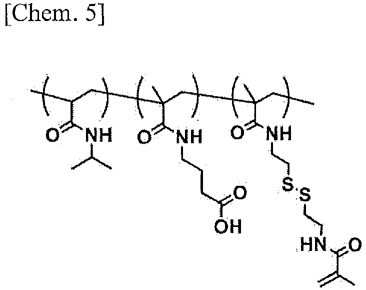

### Polymer recipe:

**[Table A-1]**

| | **Mw.** | | |
|---|---|---|---|
| Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | 256.82 | 100 | 1 |
| *4-[(2-Methyl-1-oxo-2-propen-1-yl)amino]butanoic acid* | 171.19 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101).

### 1-2. Polymer (E101)-particle core complex formation and immobilization on substrate

A polymer (E101) aqueous solution (2 mg/mL) and a particle core dispersion having a basic group on a surface were mixed at 1/1, v/v. The solution was diluted 80-fold with DMF. 4 µL of the solution was dropped on a gold substrate modified with 2-(2-bromoisobutyryloxy)undecyl thiol and amino-EG6 undecanethiol hydrochloride, and the substrate was allowed to stand for 1 hour. Thereafter, the substrate was washed with EtOH to obtain a particle-immobilized substrate.

### 2-1. Synthesis of polymer (E102)

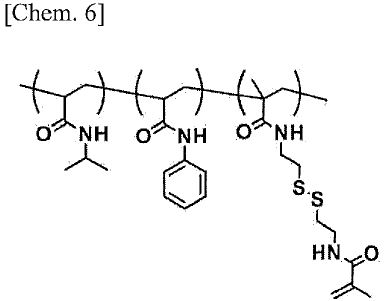

### Polymer recipe:

**[Table A-2]**

| | **Mw.** | | |
|---|---|---|---|
| Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | 256.82 | 100 | 1 |
| *N-Phenyl acrylamide* | 147.17 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E102).

### 2-2. Polymer (E102)-particle core complex formation and immobilization on substrate

A polymer (E102) aqueous solution (2 mg/mL) and a particle core dispersion having a hydrophobic group such as a benzene ring on a surface were mixed at 1/1, v/v. The solution was diluted 80-fold with DMF. 4 µL of the solution was dropped on a gold substrate modified with 2-(2-bromoisobutyryloxy)undecyl thiol alone or SAM mixed with undecanethiol, and the substrate was allowed to stand for 1 hour. Thereafter, the substrate was washed with EtOH to obtain a particle-immobilized substrate.

### 3-1. Synthesis of polymer (E103)

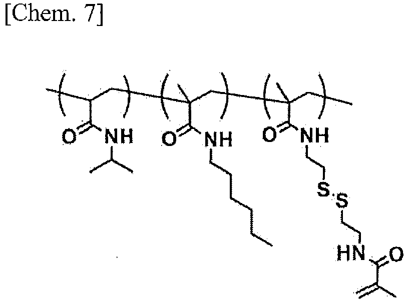

### Polymer recipe:

**[Table A-3]**

| | **Mw.** | | |
|---|---|---|---|
| Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | 256.82 | 100 | 1 |
| N-Hexylmethacrylamide | 169.26 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E103).

### 3-2. Polymer (E103)-particle core complex formation and immobilization on substrate

A polymer (E103) aqueous solution (2 mg/mL) and a particle core dispersion having a hydrophobic group such as a benzene ring or an alkyl chain on a surface were mixed at 1/1, v/v. The solution was diluted 80-fold with DMF. 4 µL of the solution was dropped on a gold substrate modified with 2-(2-bromoisobutyryloxy)undecyl thiol alone or SAM mixed with undecanethiol, and the substrate was allowed to stand for 1 hour. Thereafter, the substrate was washed with EtOH to obtain a particle-immobilized substrate.

### 4-1. Synthesis of polymer (E104)

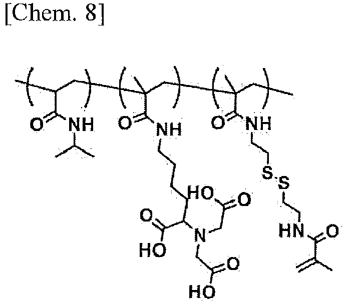

### Polymer recipe:

**[Table A-4]**

| | **Mw.** | | |
|---|---|---|---|
| Sample | | Concen tration (mM) | Final liquid volume (mL) |
| *Cystamine monomer* (*N*-(2-[(2-Aminoethyl)dithio]ethyl)-2-methyl-2-propenamide, hydrochloride) | 256.82 | 100 | 1 |
| *NTA monomer (L-Lysine, N2,N2-bis(carboxymethyl)-N6-(2-methyl-1-oxo-2-propen-1-yl)-)* | 330.33 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E104).

### 4-2. Polymer (E104)-particle core complex formation and immobilization on substrate

A polymer (E104) aqueous solution (2 mg/mL) and a particle core dispersion having a group capable of forming a coordinate bond such as a His-tag on a surface were mixed at 1/1, v/v. The solution was diluted 80-fold with DMF. A gold substrate modified with 2-(2-bromoisobutyryloxy)undecyl thiol and carboxy-EG6 undecanethiol was immersed in CH₂Cl₂ in which 0.1 M EDC and 0.05 M NHS were dissolved, and the substrate was allowed to stand for 1 hour. The substrate after the reaction was washed with CH₂Cl₂ and dried with nitrogen blow. 4 µL of a polymer (E104)-particle core mixed solution was dropped on the substrate, and the substrate was allowed to stand for 1 hour. Thereafter, the substrate was washed with EtOH to obtain a particle-immobilized substrate.

### 5-1. Synthesis of polymer (E105)

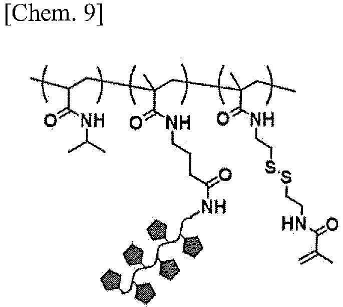

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101)-NHS(E112).

10 mg of the polymer prepared in the previous section and 28 µL of TEA were dissolved in DMF (1 mL), and stirring was performed. 3 mg of a synthetic peptide (having a structure in which six histidines were bonded from the N-terminal, three glycines were bonded, and a lysine was bonded to the C-terminal, and a carboxyl group at the C-terminal was amidated) was added thereto, and stirring was further performed overnight. After the reaction, a small amount of CH₂Cl₂ was added and then an excessive amount of n-hexane was added to generate a precipitate. The precipitate was collected, a small amount of CH₂Cl₂ was added thereto, an excessive amount of n-hexane was further added to wash the precipitate, and then the precipitate was vacuum-dried, thereby obtaining a polymer (E105).

### 5-2. Polymer (E105)-particle core complex formation and immobilization on substrate

A polymer (E105) aqueous solution (2 mg/mL) and a particle core dispersion having a group capable of forming a coordinate bond such as an NTA group on a surface were mixed at 1/1, v/v. The solution was diluted 80-fold with DMF. A gold substrate modified with 2-(2-bromoisobutyryloxy)undecyl thiol and amino-EG6 undecanethiol hydrochloride was immersed in DMSO in which 1 mM N-[5-(4-Isothiocyanatobenzyl)amido-1-carboxypentyl]iminodiacetic acid was dissolved, and the substrate was allowed to stand for 1 hour. The substrate after the reaction was washed with DMSO and EtOH and dried with nitrogen blow. 4 µL of a polymer (E105)-particle core mixed solution was dropped on the substrate, and the substrate was allowed to stand for 1 hour. Thereafter, the substrate was washed with EtOH to obtain a particle-immobilized substrate.

### 6-1. Synthesis of polymer (E106)

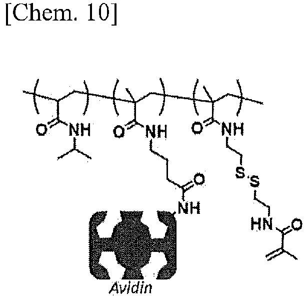

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E101)-NHS(E112).

10 mg of the polymer prepared in the previous section was dissolved in a 50 mM carbonate buffer (pH 8.5) (1 mL), and stirring was performed. 3 mg of Avidin was added thereto, and stirring was further performed overnight. The reaction solution was placed in a dialysis membrane for 100 kDa, and dialysis was carried out in a phosphate buffer (pH 7.4). After the dialysis, concentration was performed by ultrafiltration to obtain a target solution of the polymer (E106).

### 6-2. Polymer (E106)-particle core complex formation and immobilization on substrate

A polymer (E106) aqueous solution (2 mg/mL) and a particle core dispersion having biotin or a derivative thereof on a surface were mixed at 1/1, v/v. The solution was appropriately diluted with a phosphate buffer. A gold substrate modified with 2-(2-bromoisobutyryloxy)undecyl thiol and amino-EG6 undecanethiol hydrochloride was immersed in DMSO in which 0.1 M EDC and 0.05 M biotin were dissolved, and the substrate was allowed to stand for 1 hour. The substrate after the reaction was washed with DMSO and EtOH and dried with nitrogen blow. 40 µL of a polymer (E105)-particle core mixed solution was dropped on the substrate, and the substrate was allowed to stand for 1 hour. Thereafter, the substrate was washed with a phosphate buffer to obtain a particle-immobilized substrate.

### 7-1. Synthesis of polymer (E107)

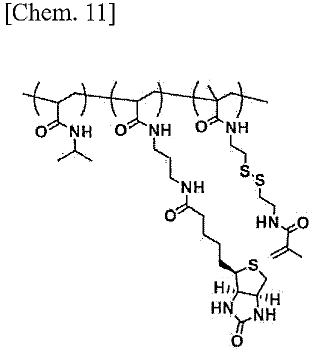

### Polymer recipe:

**[Table A-5]**

| | **Mw.** | | |
|---|---|---|---|
| Sample | | Concen tration (mM) | Final liquid volume (mL) |
| *Cystamine monomer* (N-[2-[(2-Aminoethyl)dithio]ethyl]-2-methyl-2-propenamide, hydrochloride) | 256.82 | 100 | 1 |
| *biotin monomer ((3aS,4S,6aR)-Hexahydro-2-oxo-N-[3-[(1-oxo-2-propen-1-yl)amino]propyl]-1H-thieno[3,4-d]imidazole-4-pentanamide)* | 354.47 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E107).

### 7-2. Polymer (E107)-particle core complex formation and immobilization on substrate

A polymer (E107) aqueous solution (2 mg/mL) and a particle core dispersion whose surface was modified with Avidin at a ratio of 1/1, v/v. The solution was appropriately diluted with PBS. A gold substrate modified with 2-(2-bromoisobutyryloxy)undecyl thiol and carboxy-EG6 undecanethiol was immersed in CH₂Cl₂ in which 0.1 M EDC and 0.05 M NHS were dissolved, and the substrate was allowed to stand for 1 hour. The substrate after the reaction was washed with CH₂Cl₂ and dried with nitrogen blow. 1 mg/mL of an Avidin solution was dropped on the substrate, and the substrate was allowed to stand for 1 hour. After the reaction, the substrate was washed with a phosphate buffer. A polymer (E107)-particle core mixed solution was dropped on the substrate, and the substrate was allowed to stand for 1 hour. Thereafter, the substrate was washed with a phosphate buffer to obtain a particle-immobilized substrate.

### 8-1. Synthesis of polymer (E108)

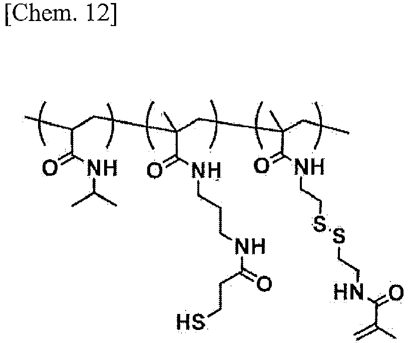

100 mg of the polymer (E3) and 140 µL (1.0 mmol) of TEA were dissolved in CH₂Cl₂ and stirring was performed under ice cooling. 40 mg (0.2 mmol) of EDC and 22 mg (0.2 mmol) of 3-mercaptopropionic acid were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E108).

### 8-2. Polymer (E106)-particle core complex formation and immobilization on substrate

A polymer (E108) aqueous solution (2 mg/mL) and a particle core dispersion having a thiol-reactive group such as a maleimide group or a pyridyl disulfide group on a surface were mixed at 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 3-maleimidopropionic acid or 3-(2-pyridyldithio)propionic acid was dissolved, and the substrate was allowed to stand for 1 hour. The substrate after the reaction was washed with EtOH and dried with nitrogen blow. 4 µL of a polymer (E108)-particle core mixed solution was dropped on the substrate, and the substrate was allowed to stand for 1 hour. Thereafter, the substrate was washed with EtOH to obtain a particle-immobilized substrate.

### 9-1. Synthesis of polymer (E109)

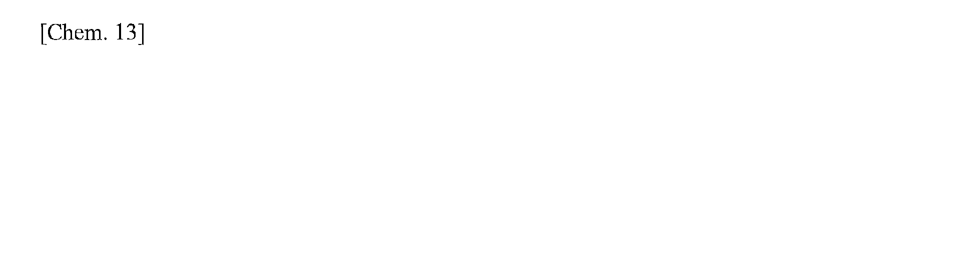

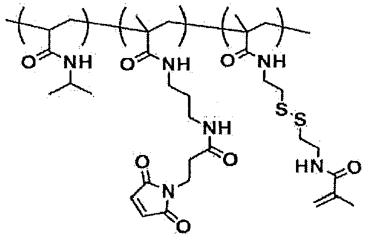

100 mg of the polymer (E3) and 140 µL (1.0 mmol) of TEA were dissolved in CH₂Cl₂ and stirring was performed under ice cooling. 40 mg (0.2 mmol) of EDC and 34 mg (0.2 mmol) of 3-maleimidopropionic acid were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E108).

### 9-2. Polymer (E109)-particle core complex formation and immobilization on substrate

A polymer (E109) aqueous solution (2 mg/mL) and a particle core dispersion having a thiol group on a surface were mixed at 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 3-mercaptopropionic acid were dissolved, and the substrate was allowed to stand for 1 hour. The substrate after the reaction was washed with EtOH and dried with nitrogen blow. 4 µL of a polymer (E109)-particle core mixed solution was dropped on the substrate, and the substrate was allowed to stand for 1 hour. Thereafter, the substrate was washed with EtOH to obtain a particle-immobilized substrate.

### 10-1. Synthesis of polymer (E110)

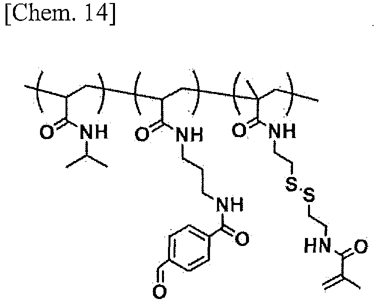

### Polymer recipe:

**[Table A-6]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *aldehyde monomer (4-Formyl-N-[2-[(1-oxo-2-propen-1-yl)amino]propyl]benzamide)* | | 258.26 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E110).

### 10-2. Polymer (E110)-particle core complex formation and immobilization on substrate

A polymer (E110) aqueous solution (2 mg/mL) and a particle core dispersion whose surface was modified with an amino group at a ratio of 1/1, v/v. The solution was appropriately diluted with DMF. A polymer (E110)-particle core mixed solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 11-1. Synthesis of polymer (E111)

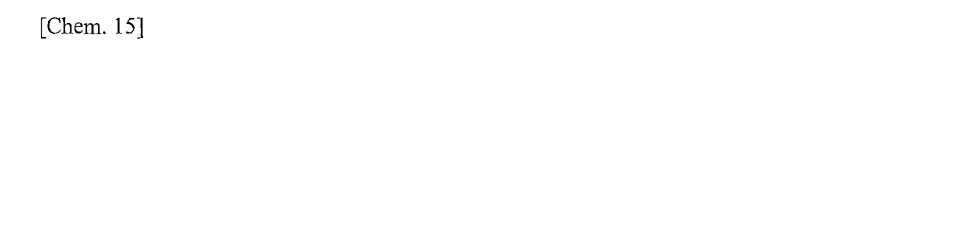

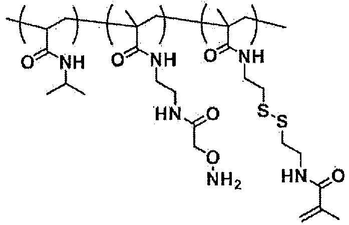

### Polymer recipe:

**[Table A-7]**

| | | **Mw**. | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *Boc-oxime monomer (1,1-Dimethylethyl 11-methyl-5,10-dioxo-3-oxa-2,6,9-triazadodec-11-enoate)* | | 301.34 | 100 | 1 |
| NIPAm | | 113.16 | 300 | 1 |
| 2-Cyano-2-[(dodecylsulfanylthiocarbohyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 54 mg (0.3 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

The obtained polymer was dissolved in CH₂Cl₂ and stirring was performed under ice cooling. 1 mL of 4 N HCl in dioxane was added thereto, and stirring was further performed overnight. After the reaction, a precipitate generated by adding n-hexane was collected. A small amount of CH₂Cl₂ was added to the precipitate, an excessive amount of n-hexane was further added to wash the precipitate, and then the precipitate was vacuum-dried, thereby obtaining a polymer (E111).

### 11-2. Polymer (E111)-particle core complex formation and immobilization on substrate

A polymer (E111) aqueous solution (2 mg/mL) and a particle core dispersion whose surface was modified with an aldehyde group at a ratio of 1/1, v/v. The solution was appropriately diluted with DMF. A gold substrate modified with 2-(2-bromoisobutyryloxy)undecyl thiol and carboxy-EG6 undecanethiol was immersed in DMF in which 0.1 M EDC and 0.05 M 4-formylbenzoic acid were dissolved, and the substrate was allowed to stand for 1 hour. The substrate after the reaction was washed with EtOH and dried with nitrogen blow. 4 µL of a polymer (E111)-particle core mixed solution was dropped on the substrate, and the substrate was allowed to stand for 1 hour. Thereafter, the substrate was washed with EtOH to obtain a particle-immobilized substrate.

### 12-1. Synthesis of polymer (E112)

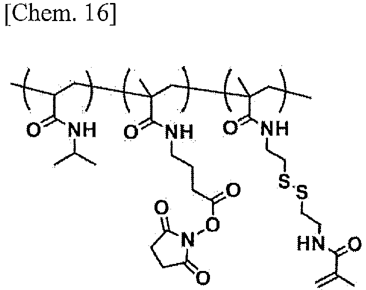

100 mg of the polymer (E101) and 140 µL (1.0 mmol) of TEA were dissolved in CH₂Cl₂ and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E112).

### 12-2. Polymer (E112)-particle core complex formation and immobilization on substrate

A polymer (E112) aqueous solution (2 mg/mL) and a particle core dispersion having an amino group at a ratio of 1/1, v/v. The solution was allowed to stand overnight and diluted 80-fold with DMF. 4 µL of a polymer (E112)-particle core mixed solution was dropped on a gold substrate modified with 2-(2-bromoisobutyryloxy)undecyl thiol and 1-decanaminium, 10-mercapto-N,N,N-trimethyl-, and chloride, and the substrate was allowed to stand for 1 hour. Thereafter, the substrate was washed with EtOH to obtain a particle-immobilized substrate.

### 13-1. Synthesis of polymer (E113)

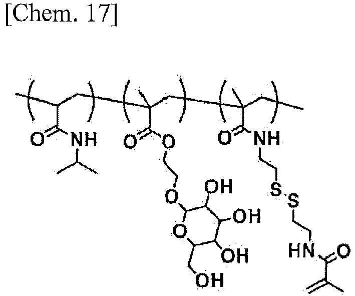

### Polymer recipe:

**[Table A-8]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *GFAM* | | 292.28 | 100 | 1 |
| NIPAm | | 113.16 | 300 | 1 |
| 2-Cyano-2-[(dodecylsulfanylthiocarbonyl)suifanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E113).

### 13-2. Polymer (E111)-particle core complex formation and immobilization on substrate

A polymer (E113) aqueous solution (2 mg/mL) and a particle core dispersion whose surface was modified with a phenylboronic acid group at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which 0.1 M EDC and 0.05 M 4-carboxyphenylboronic acid (or 3-fluoro-4-carboxyphneyl boronic acid) was dissolved, and the substrate was allowed to stand for 1 hour. The substrate after the reaction was washed with EtOH and dried with nitrogen blow. 4 µL of a polymer (E113)-particle core mixed solution was dropped on the substrate, and the substrate was allowed to stand for 1 hour. Thereafter, the substrate was washed with EtOH to obtain a particle-immobilized substrate.

### 14-1. Synthesis of polymer (E114)

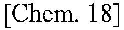

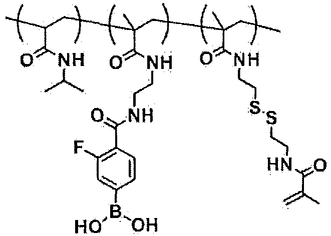

### Polymer recipe:

**[Table A-9]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *4-(2-Methacrylamidoethylcarbamoyl)-3-fluorophenylboronic acid* | | 292.06 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E114).

### 14-2. Polymer (E114)-particle core complex formation and immobilization on substrate

A polymer (E114) aqueous solution (2 mg/mL) and a particle core dispersion whose surface was modified with group having a cis-diol structure at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which TEA and α-D-mannopyranosylphenyl isothiocyanate were dissolved, and the substrate was allowed to stand for 1 hour. The substrate after the reaction was washed with EtOH and dried with nitrogen blow. 4 µL of a polymer (E114)-particle core mixed solution was dropped on the substrate, and the substrate was allowed to stand for 1 hour. Thereafter, the substrate was washed with EtOH to obtain a particle-immobilized substrate.

### 15-1. Synthesis of polymer (E115)

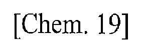

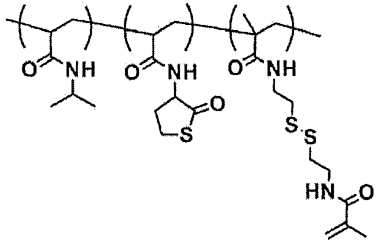

### Polymer recipe:

**[Table A-10]**

| | | **Mw.** | | |
|---|---|---|---|---|
| | Sample | | Concen tration (mM) | Final liquid volume (mL) |
| *Cystamine monomer* (*N*-[2-[(2-Aminoethyl)dithio]ethyl]-2-methyl-2-propenamide, hydrochloride) | | 256.82 | 100 | 1 |
| *N-Acryloylhomocysteine thiolactone* | | 171.22 | 100 | 1 |
| NIPAm | | 113.16 | 300 | 1 |
| 2-Cyano-2-((dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E115).

### 15-2. Polymer (E115)-particle core complex formation and immobilization on substrate

A polymer (E114) aqueous solution (2 mg/mL) and a particle core dispersion whose surface was modified with an amino group at a ratio of 1/1, v/v. The solution was appropriately diluted with DMF. A gold substrate modified with 2-(2-bromoisobutyryloxy)undecyl thiol and amino-EG6 undecanethiol hydrochloride was immersed in DMF in which EDC and 3-(2-pyridyldithio)propionic acid were dissolved, and the substrate was allowed to stand for 1 hour. The substrate after the reaction was washed with EtOH and dried with nitrogen blow. 4 µL of a polymer (E115)-particle core mixed solution was dropped on the substrate, and the substrate was allowed to stand for 1 hour. Thereafter, the substrate was washed with EtOH to obtain a particle-immobilized substrate.

### 16-1. Synthesis of polymer (E116)

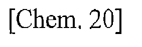

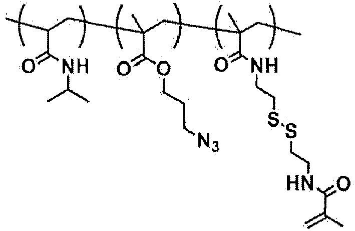

### Polymer recipe:

**[Table A-11]**

| | | **Mw.** | | |
|---|---|---|---|---|
| | Sample | | Concen tration (mM) | Final liquid volume (mL) |
| *Cystamine monomer* (*N*-(2-[(2-Aminoethyl)dithio]ethyl]-2-methyl-2-propenamide, hydrochloride) | | 256.82 | 100 | 1 |
| *3-Azidopropyl methacrylate* | | 169.18 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 27 mg (0.15 mmol) of N-succinimidyl methacrylate was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E116).

### 16-2. Polymer (E116)-particle core complex formation and immobilization on substrate

A polymer (E116) aqueous solution (2 mg/mL) and a particle core dispersion whose surface was modified with an alkyne group at a ratio of 1/1, v/v. Copper sulfate and sodium ascorbate were added thereto, and stirring was performed for 1 hour. After the reaction, the solution was replaced with pure water by centrifugation. The solution was appropriately diluted with DMF. A gold substrate modified with 2-(2-bromoisobutyryloxy)undecyl thiol and amino-EG6 undecanethiol hydrochloride was immersed in DMF in which EDC and 4-(3-butyn-1-yldithio)butanoic acid were dissolved, and the substrate was allowed to stand for 1 hour. The substrate after the reaction was washed with EtOH and dried with nitrogen blow. 4 µL of a polymer (E116)-particle core mixed solution (containing copper sulfate and ascorbic acid) was dropped on the substrate, and the substrate was allowed to stand for 1 hour. Thereafter, the substrate was washed with EtOH to obtain a particle-immobilized substrate.

### 17-1. Synthesis of polymer (E116)

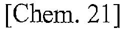

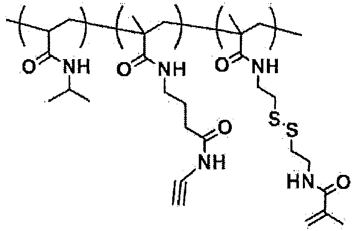

The polymer (E112) and 140 µL (1.0 mmol) of TEA were dissolved in CH₂Cl₂ and stirring was performed under ice cooling. 11 mg (0.2 mmol) of propargylamine were added thereto and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E117).

### 17-2. Polymer (E117)-particle core complex formation and immobilization on substrate

A polymer (E117) aqueous solution (2 mg/mL) and a particle core dispersion whose surface was modified with an azide group at a ratio of 1/1, v/v. Copper sulfate and sodium ascorbate were added thereto, and stirring was performed for 1 hour. After the reaction, the solution was replaced with pure water by centrifugation. The solution was appropriately diluted with DMF. A gold substrate modified with 2-(2-bromoisobutyryloxy)undecyl thiol and amino-EG6 undecanethiol hydrochloride was immersed in DMF in which EDC and 3-[(2-azidoethyl)dithio]propanoic acid were dissolved, and the substrate was allowed to stand for 1 hour. The substrate after the reaction was washed with EtOH and dried with nitrogen blow. 4 µL of a polymer (E117)-particle core mixed solution (containing copper sulfate and ascorbic acid) was dropped on the substrate, and the substrate was allowed to stand for 1 hour. Thereafter, the substrate was washed with EtOH to obtain a particle-immobilized substrate.

### 18-1. Synthesis of polymer (E201)

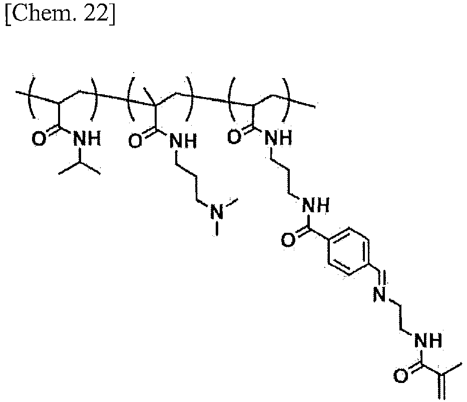

### Polymer recipe:

**[Table A-12]**

| | | **Mw.** | | |
|---|---|---|---|---|
| | Sample | | Concen tration (mM) | Final liquid volume (mL) |
| *aldehyde monomer (4-Formyl-N-[2-[(1-oxo-2-propen-1-yl)amino]propyl]benzamide)* | | 258.26 | 100 | 1 |
| *N*-(3-(Dimethylamino)propyl)methacrylamide | | 170.25 | 100 | 1 |
| NIPAm | | 113.16 | 300 | 1 |
| 2-Cyano-2-[(dodecylsulfahylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 25 mg (0.2 mmol) of N-(2-aminoethyl)methacrylamide was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E201).

### 18-2. Polymer (E201)-particle core complex formation and immobilization on substrate

A polymer (E201) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 19-1. Synthesis of polymer (E202)

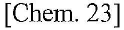

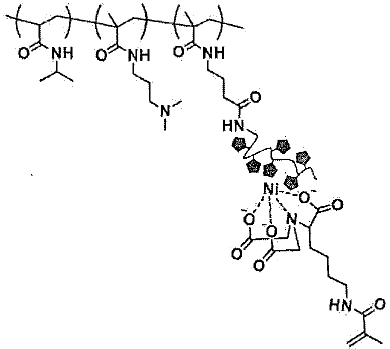

### Polymer recipe:

**[Table A-13]**

| | | **Mw.** | | |
|---|---|---|---|---|
| | Sample | | Concent ration (mM) | Final liquid volume (mL) |
| *4-[(2-Methyl-1-oxo-2-propen-1-yl)amino]butanoic acid* | | 171.19 | 100 | 1 |
| *N*-(3-(Dimethylamino)propyl)methacrylamide | | 170.25 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C) (20 h). After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

10 mg of the polymer prepared in the previous section and 28 µL of TEA were dissolved in DMF (1 mL), and stirring was performed. 3 mg of a synthetic peptide (having a structure in which six histidines were bonded from the N-terminal, three glycines were bonded, and a lysine was bonded to the C-terminal, and a carboxyl group at the C-terminal was amidated) was added thereto, and stirring was further performed overnight. After the reaction, a small amount of CH₂Cl₂ was added and then an excessive amount of n-hexane was added to generate a precipitate. The precipitate was collected, a small amount of CH₂Cl₂ was added thereto, an excessive amount of n-hexane was further added to wash the precipitate, and then the precipitate was vacuum-dried.

The polymer prepared in the previous section was dissolved in MeOH, Ni-complexed L-Lysine and N2,N2-bis(carboxymethyl)-N6-(2-methyl-1-oxo-2-propen-1-yl)- was added thereto, and stirring was performed. CH₂Cl₂ was added, and the precipitated precipitate was collected and vacuum-dried to obtain a polymer (E202).

### 19-2. Polymer (E202)-particle core complex formation and immobilization on substrate

A polymer (E202) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 20-1. Synthesis of polymer (E203)

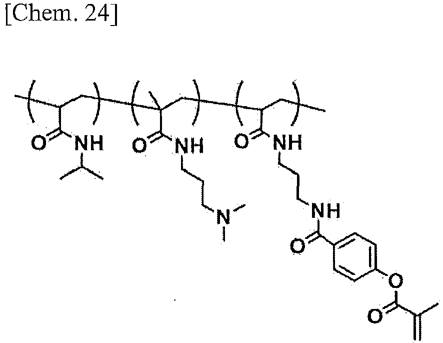

### Polymer recipe:

**[Table A-14]**

| | | **Mw.** | | |
|---|---|---|---|---|
| | Sample | | Concen tration (mM) | Final liquid volume (mL) |
| *N-(3-Aminopropyl)methacrylamide hydrochloride* | | 178.66 | 100 | 1 |
| *N*-(3-(Dimethylamino)propyl)methacrylamide | | 170.25 | 100 | 1 |
| NIPAm | | 113.16 | 300 | 1 |
| 2-Cyano-2-[(dodecylsulfanylthiocarbonyl)sulfanyl]propane | | 345.63 | 12.5 | 1 |
| AIBN | | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of 4-methacryloyloxybenzoic acid was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E203).

### 20-2. Polymer (E203)-particle core complex formation and immobilization on substrate

A polymer (E203) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 21-1. Synthesis of polymer (E204)

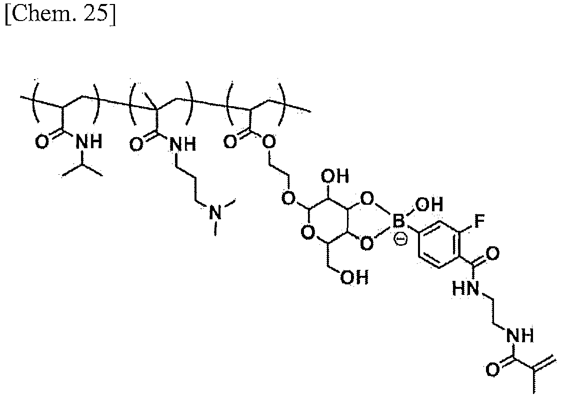

### Polymer recipe:

**[Table A-15]**

| | **Mw.** | | |
|---|---|---|---|
| Sample | | Concen tration (mM) | Final liquid volume (mL) |
| *GEMA* | 292:28 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 59 mg (0.2 mmol) of 4-(2-methacrylamidoethylcarbamoyl)-3-fluorophenylboronic acid was added thereto, and stirring was performed overnight (14 h). After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E204).

### 21-2. Polymer (E204)-particle core complex formation and immobilization on substrate

A polymer (E204) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 22-1. Synthesis of polymer (E205)

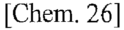

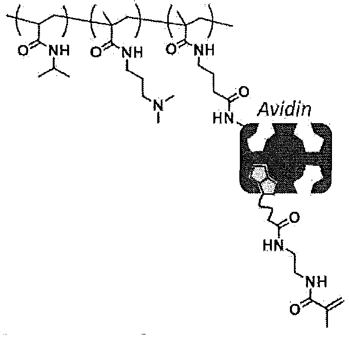

### Polymer recipe:

**[Table A-16]**

| | **Mw.** | | |
|---|---|---|---|
| Sample | | Concen tration (mM) | Final liquid volume (mL) |
| *4-[(2-Methyl-1-oxo-2-propen-1-yl)amino]butanoic acid* | 171.19 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 40 mg (0.2 mmol) of EDC and 22 mg (0.2 mmol) of NHS were added thereto, and stirring was further performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

10 mg of the polymer prepared in the previous section was dissolved in a 50 mM carbonate buffer (pH 8.5) (1 mL), and stirring was performed. 3 mg of Avidin was added thereto, and stirring was further performed overnight. The reaction solution was placed in a dialysis membrane for 100 kDa, and dialysis was carried out in a phosphate buffer (pH 7.4). After dialysis, concentration was performed by ultrafiltration. Thereafter, a biotin monomer ((3aS,4S,6aR)-hexahydro-2-oxo-N-[3-[(1-oxo-2-propen-1-yl)amino]propyl]-1H-thieno[3,4-d]imidazole-4-pentanamide) was bonded to obtain a target polymer (E205).

### 22-2. Polymer (E205)-particle core complex formation and immobilization on substrate

A polymer (E205) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### 23-1. Synthesis of polymer (E206)

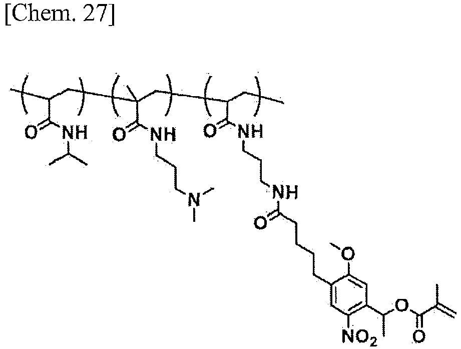

### Polymer recipe:

**[Table A-17]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N*-(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AlBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 38 mg (0.2 mmol) of EDC and 73 mg (0.2 mmol) of 4-[4-[1-(methacryloyloxy)ethyl]-2-methoxy-5-nitrophenoxy]butanoic acid were added thereto, and stirring was performed overnight under light-shielding. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E206).

### 23-2. Polymer (E206)-particle core complex formation and immobilization on substrate

A polymer (E206) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour under light-shielding. Thereafter, the substrate was washed with EtOH to obtain a particle-immobilized substrate.

### 24-1. Synthesis of polymer (E207)

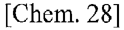

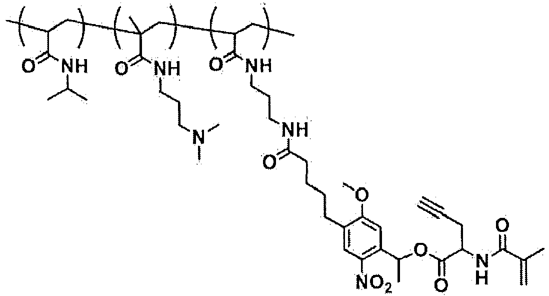

### Polymer recipe:

**[Table A-18]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N-*(3-(Dimethylamino)propyl)methacrylamide | 170.25 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h)

After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The polymer prepared in the previous section and 140 µL (1.0 mmol) of TEA were dissolved in CH₂Cl₂, and stirring was performed under ice cooling. 38 mg (0.2 mmol) of EDC and 92 mg (0.2 mmol) of a compound A were added thereto, and stirring was performed overnight under light-shielding. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a polymer (E207).

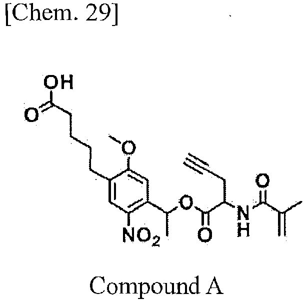

### 24-2. Polymer (E207)-particle core complex formation and immobilization on substrate

A polymer (E207) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour under light-shielding. Thereafter, the substrate was washed with EtOH to obtain a particle-immobilized substrate.

### 25-1. Synthesis of polymers (E301 to E312)

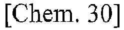

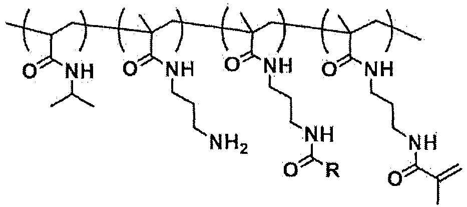

### Polymer recipe:

**[Table A-19]**

| | **Mw.** | | |
|---|---|---|---|
| Sample | | Concen tration (mM) | Final liquid volume (mL) |
| *N-(3-Aminopropyl)methacrylamide hydrochloride* | 178.66 | 100 | 1 |
| *N-(t-BOC-aminopropyl)methacrylamide* | 242.31 | 100 | 1 |
| NIPAm | 113.16 | 300 | 1 |
| 2-Cyano-2-[(dodecylsulfanylthiocarbonyl)sulfanyl]propane | 345.63 | 12.5 | 1 |
| AIBN | 164.21 | 6.25 | 1 |

The compound of the recipe was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 75°C). (20 h) After the polymerization, the reaction was stopped by ice-cooling the Schlenk flask and adding air, and a precipitate generated by adding a large amount of diethyl ether to the reaction solution was collected. A small amount of CH₂Cl₂ was added to the residue, an excessive amount of n-hexane was further added to wash the precipitate, and then the precipitate was vacuum-dried.

The prepared polymer and 140 µL (1.0 mmol) of TEA were dissolved in CH₂Cl₂, and stirring was performed under ice cooling. 36 mg (0.2 mmol) of N-succinimidyl methacrylate was added thereto, and stirring was performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried.

The polymer was dissolved in CH₂Cl₂ and stirring was performed under ice cooling. 1 mL of 4 N HCl in dioxane was added thereto, and stirring was further performed overnight. After the reaction, a precipitate generated by adding n-hexane was collected. A small amount of CH2Cl2 was added to the precipitate, an excessive amount of n-hexane was further added to wash the precipitate, and then the precipitate was vacuum-dried.

The polymer and 140 µL (1.0 mmol) of TEA were dissolved in CH₂Cl₂ and stirring was performed under ice cooling. 0.25 mmol of any of the compounds in the table on the right and 20 mg (0.1 mmol) of *EDC were added thereto, and stirring was performed overnight. After the reaction, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain target polymers (E301 to E312).

Use at time of synthesizing *E304 to E309 and E311 and E312

**[Table A-20]**

| | Substituent to be introduced | Compound example |
|---|---|---|
| **E301** | Carboxy | 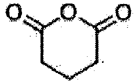 |
| **E302** | Carboxylic acid active ester group | 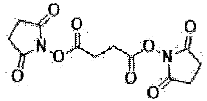 |
| **E303** | Metal complex (NTA group) | 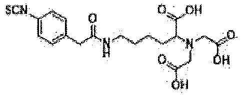 |
| **E304** | Thiol group | 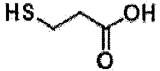 |
| **E305** | Pyridyl disulfide group | 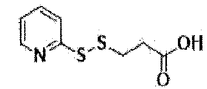 |
| **E306** | Maleimide group | 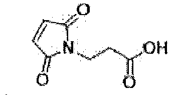 |
| **E307** | Halogenated acetamide group | 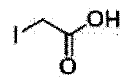 |
| **E308** | Azide group | 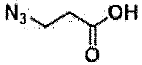 |
| **E309** | Alkyne group | 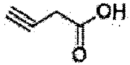 |
| **E310** | cis-diol group (sugar group) | 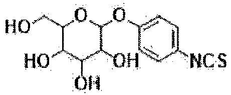 |
| **E311** | Boronyl group | 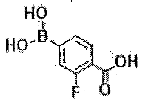 |
| **E312** | Thiolactone group | 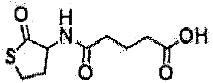 |

### 25-2. Polymer (E301)-particle core complex formation and immobilization on substrate

A polymer (E301) aqueous solution (2 mg/mL) and a particle core dispersion having an acidic group on a surface were mixed at 1/1, v/v. The solution was diluted 80-fold with DMF. 4 µL of the solution was dropped on a gold substrate modified with 2-(2-bromoisobutyryloxy)undecyl thiol and carboxy-EG6 undecanethiol, and the substrate was allowed to stand for 1 hour. Thereafter, the substrate was washed with EtOH to obtain a particle-immobilized substrate.

### (Example 1-2: Binding of polymer to particles (non-covalent bond))

### 1-2-1. Synthesis of E3 conjugated silica NPs

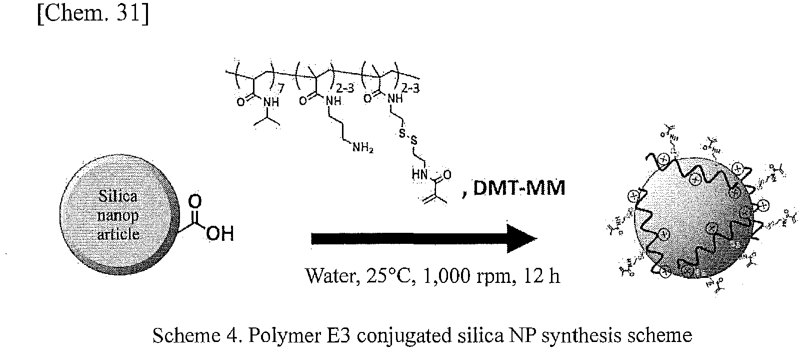

100 µL of a silica nanoparticle dispersion (2.0 × 10¹² particles/mL), 500 µL of a polymer E3 aqueous solution (2.0 mg/mL), and 400 µL of a 4-(4,6-dimethoxy-1,3,5-triazine-2-yl)-4 methylmorpholinium chloride (DMT-MM) aqueous solution (12.5 mM) were mixed, and stirring was performed with a thermoshaker at 25°C and 1,000 rpm for 12 hours. After the reaction, the reaction product in the solution was removed by centrifugation (25°C, 10,000 g, 15 min) × 5. Each supernatant was confirmed by UV-vis measurement (NanoDrop).

According to a similar procedure, the polymer E2 and the polymer E4 were integrated with silica particles.

### (Results)

### 1-2-2. DLS and Z-potential measurement

20 µL of a silica nanoparticle dispersion (2.0 × 10¹² particles/mL), 100 µL of various cationic polymer aqueous solutions (2.0 mg/mL), and 80 µL of pure water were mixed and thoroughly stirred with a vortex. The solution was diluted 100-fold with pure water to obtain a DLS and Z-potential measurement sample. As a measuring instrument, Zetasizer pro (Marvern Panalytical, UK) was used.

From the results of particle size measurement by DLS (FIG. 1-4 and Table 1-5) and the results of Z-potential measurement (FIG. 1-5 and Table 1-5), it was shown that the produced cationic polymer was adsorbed in the form of silica nanoparticles, and surface characteristics thereof were changed from a negatively charged state to a positively charged state. In addition, since a large change in particle size was not observed before and after the polymer was adsorbed to the silica nanoparticles, it was shown that the silica nanoparticle breakup dispersibility was maintained even after the polymer adsorption.

**[Table 1-5]**

| Table 1-5 DLS measurement and Z-potential measurement for each particle | | |
|---|---|---|
| | Average particle size (nm) | Surface charge (mV) |
| Silica nanoparticle | 208 | -53.2 |
| Silica nanoparticle + E2 | 216 | 56.2 |
| Silica nanoparticle + E4 | 237 | 60.9 |
| Silica nanoparticle + E3 | 220 | 40.5 |

### (Example 1-3: Binding of polymer to particles)

### Synthesis of polymer (E2-0)-silica nanoparticle complex (RM)

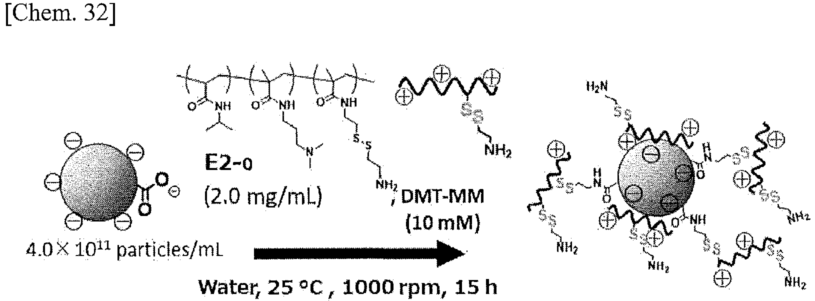

Silica nanoparticles (200 nm, terminal COOH), 2.0 mg/mL of E2-0, and 10 mM DMT-MM were mixed in pure water (500 µL) so as to be 4.0 × 10¹¹ particles/mL, and the mixture was shaken by a thermoshaker at 25°C and 1,000 rpm for 15 hours. After the reaction, 500 µL of pure water was added, the particles were precipitated by centrifugation (25°C, 1,000 rpm, 15 min), and 900 µL of the supernatant was removed. This operation was performed five times in total to purify the particles. The results of performing DLS measurement on the particle size and surface charge of the obtained particles are illustrated in FIG. 1-6.

Since the average particle size (Z-Average) was 232 nm (pdi: 0.022) and the surface charge (Z-potential) was +55.3 mV, it was confirmed that the polymer was modified because the surface charge was greatly positively changed while maintaining the monodispersibility of the particles.

### (Example 2: Preparation of measurement substrate)

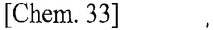

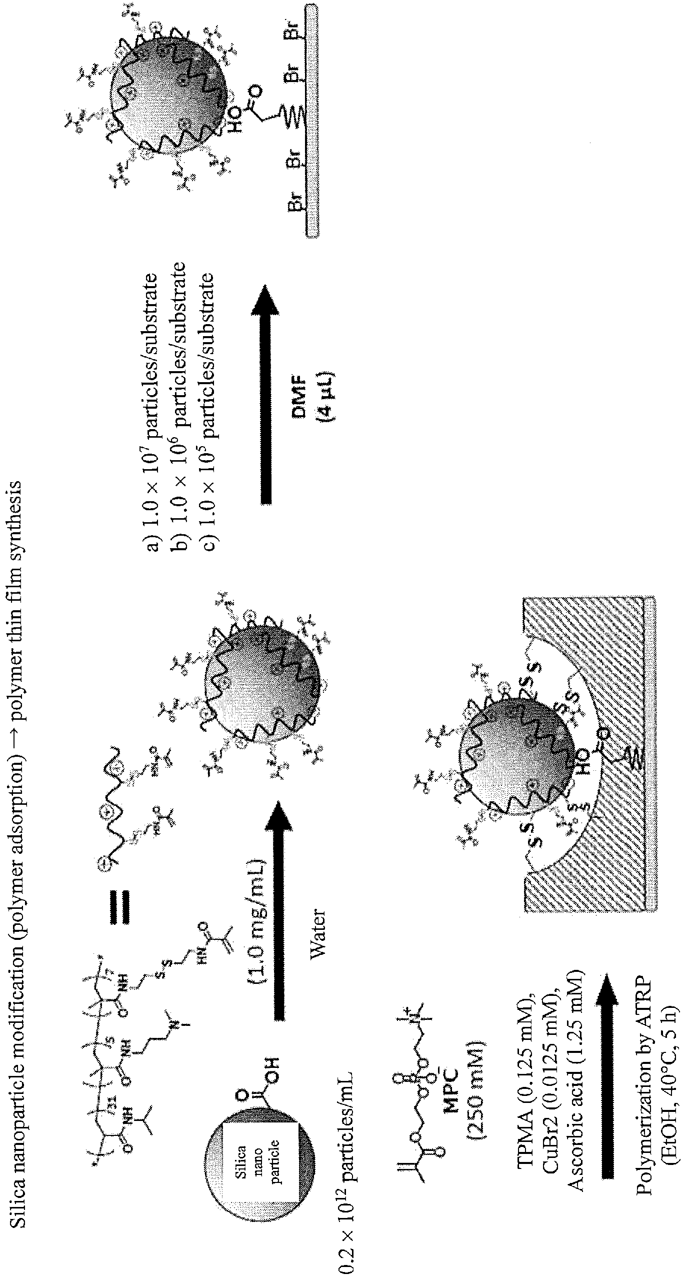

### 2-1. Experiment

### 2-1-1. Surface modification of gold substrate

A gold substrate (9.8 mm × 4.3 mm) was rinsed with EtOH and then subjected to UV-O3 treatment for 15 min to clean up a substrate surface. A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 2-1-2. Preparation of particle-immobilized substrate

A silica nanoparticle dispersion (particle size: 200 nm, particle concentration: 2.0 × 10¹¹ particles/mL) and the polymer E2 (1.0 mg/mL) were mixed in water at the above-described final concentration to form a complex.

The complex solution was diluted with DMF, dropped (4 µL) on the area of the substrate on which the complex was immobilized so as to have the number shown in Table 2-2, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 2-1.

**[Table 2-1]**

| Table 2-1 Experiment condition table | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.5 sec | 0.25 sec | 0.02 sec |
| 2 | 3×10⁶ | ↑ | ↑ | ↑ |
| 3 | 1×10⁶ | ↑ | ↑ | ↑ |
| 4 | 3×10⁵ | 2 sec | ↑ | ↑ |
| 5 | 1×10⁵ | ↑ | 0.5 sec | ↑ |

### 2-1-3. Synthesis of silica nanoparticle imprinted polymer on particle-immobilized substrate

Prepolymer solutions having compositions shown in Table 2-2 were prepared, dissolved oxygen was removed by freeze-degassing, and then polymerization was performed at 25°C for 18 hours in a glove box. The substrate after polymerization was washed with pure water.

The substrate was immersed in a 100 mM tris(2-carboxyethyl)phosphine hydrochloride solution (methanol : pure water = 1 : 1), and an ultrasonic treatment was performed for 5 min to remove silica nanoparticles, thereby forming pores in a surface layer of a polymer thin film.

**[Table 2-2]**

| Table 2-2 Prepolymer solution composition | | |
|---|---|---|
| Sample | Sample | Concentration |
| Main raw material | MPC (2-methacryloyloxyethyl phosphorylcholine) | 500 mM |
| initiator | EBIB (ethyl *α*-bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ*L |

### 2-2. Results

### 2-2-1. Preparation of particle-immobilized substrate

The observation results with a fluorescence microscope are illustrated in FIG. 2-1. The fluorescence derived from the silica nanoparticles was confirmed on the substrate under any conditions, and thus it was confirmed that the silica nanoparticles (polymer complex) were immobilized on the substrate.

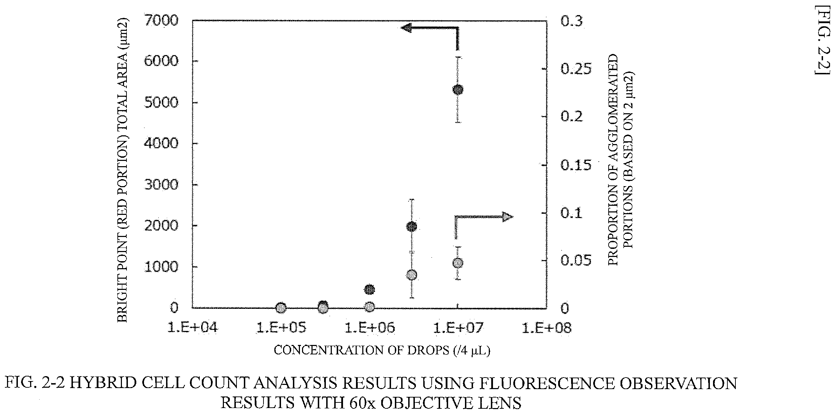

In addition, FIG. 2-2 illustrates a result of performing analysis using fluorescence observation with a 60x objective lens and hybrid cell count. From FIG. 2-2, it was found that the number of silica nanoparticles on the substrate tended to increase as the number of drops increased. In addition, in No. 4 and No. 5 in the drawing in which it was clearly determined from the image that the particles were monodispersed, not agglomerated, and in a single layer, since there were almost no bright spots with an area of more than 2 µm², the particles having a bright spot area exceeding this were determined to be an aggregate. By setting the numerical value as a boundary value, it was possible to objectively express by a numerical value that agglomeration occurred at No. 4 (3 × 10⁶ particles/4 µL) or more where bright spots exceeding the boundary value 2 µm² occurred and no agglomeration occurred at No. 5 (1 × 10⁶ particles/4 µL) or less, and it was possible to control the particle form on the substrate depending on the number of drops on the substrate.

### (Example 3: Preparation of measurement substrate (cationic polymer))

### 3. Examination using silica particles (influence of cationic polymer composition)

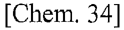

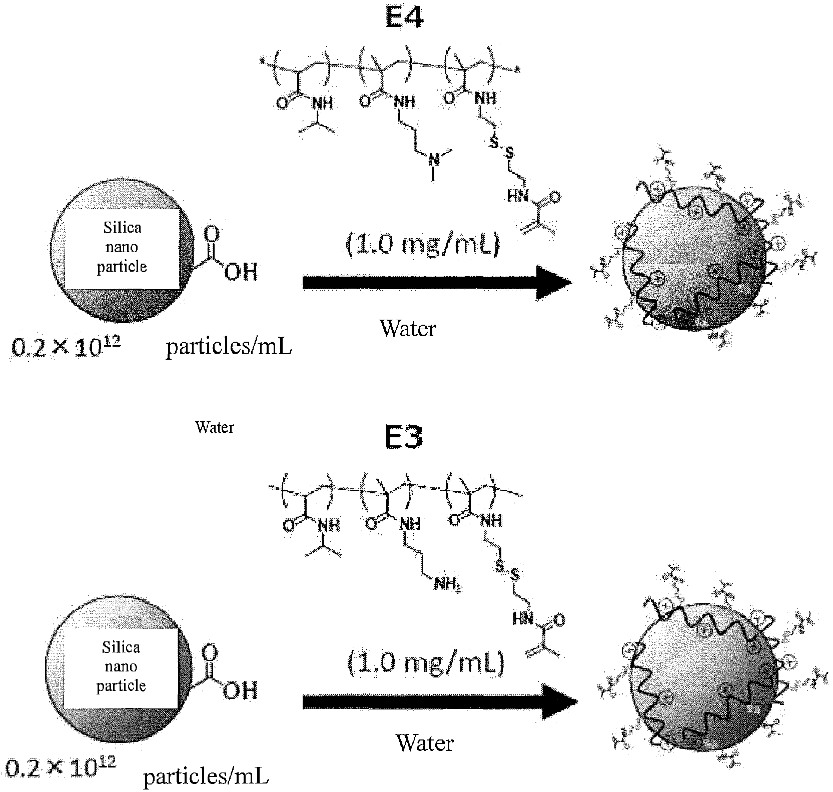

### 3-1. Experiment

### 3-1-1. Surface modification of gold substrate

A gold substrate (9.8 mm × 4.3 mm) was rinsed with EtOH and then subjected to UV-O3 treatment for 15 min to clean up a substrate surface.

A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 3-1-2. Preparation of particle-immobilized substrate

Each of a silica nanoparticle dispersion (particle size: 200 nm, particle concentration: 2.0 × 10¹¹ particles/mL) and the polymers E4 and E3 (1.0 mg/mL) was mixed in water at the above-described final concentration to form a complex.

The complex solution was diluted with DMF, dropped (4 µL) on the area of the substrate on which the complex was immobilized as shown in Table 3-1 or 3-2, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Tables 3-1 and 3-2, respectively.

**[Table 3-1]**

| Table 3-1 Experiment condition table (polymer E4) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.5 sec | 0.25 sec | 0.02 sec |
| 2 | 3×10⁶ | ↑ | ↑ | ↑ |
| 3 | 1×10⁶ | ↑ | ↑ | ↑ |
| 4 | 3×10⁵ | 2 sec | ↑ | ↑ |
| 5 | 1×10⁵ | ↑ | 0.5 see | ↑ |

**[Table 3-2]**

| Table 3-2 Experiment condition table (polymer E3) | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁷ | 1.5 sec | 0.25 sec | 0.02 sec |
| 2 | 3×10⁶ | ↑ | ↑ | ↑ |
| 3 | 1×10⁶ | ↑ | ↑ | ↑ |
| 4 | 3×10⁵ | ↑ | 0.5 sec | ↑ |
| 5 | 1×10⁵ | ↑ | ↑ | ↑ |

### 3-2. Results

### 3-2-1. Preparation of particle-immobilized substrate

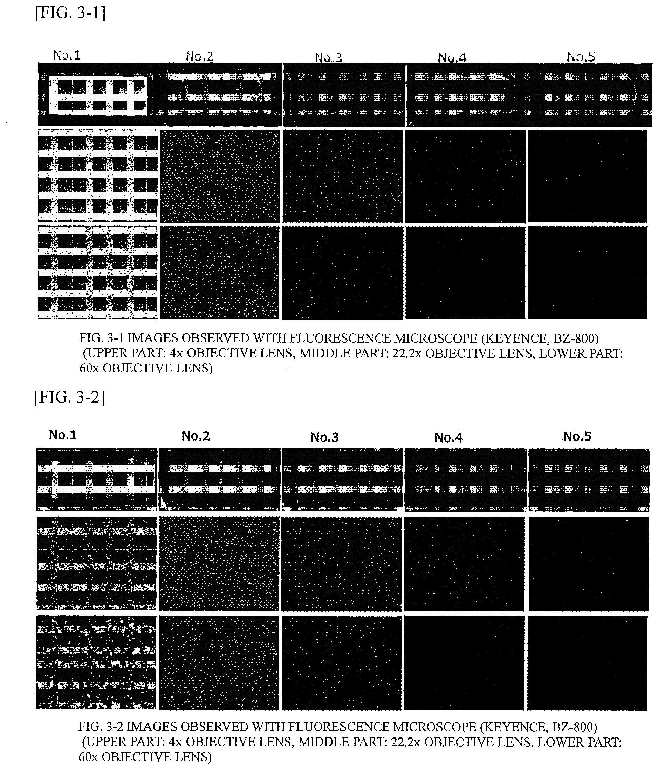

The observation results with a fluorescence microscope are illustrated in FIGS. 3-1 and 3-2. From this result, it was found that the particle form on the substrate was controlled depending on the number of drops to the substrate even with different polymer compositions.

### (Example 4: Preparation of measurement substrate (glass substrate))

### 4. Examination using silica particles (influence of substrate)

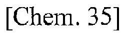

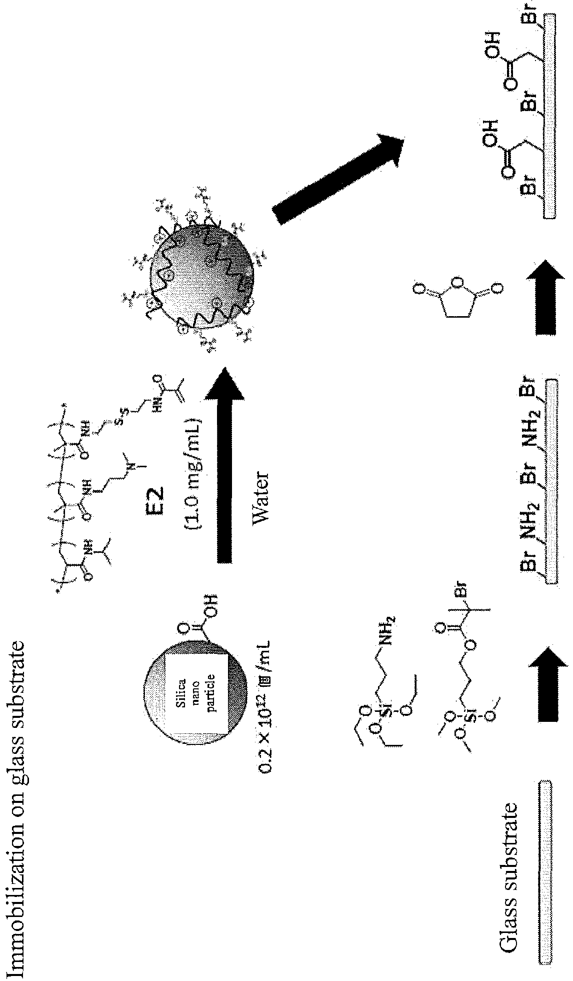

### 4-1-1. Surface modification of glass substrate

A glass substrate (9.8 mm × 4.3 mm) was rinsed with EtOH and then subjected to UV-O3 treatment for 15 min to clean up a substrate surface.

In an EtOH solvent (1% pure water), each of 3-aminopropyltriethoxysilane (ATPES) and 3-(trimethoxysilyl)propyl 2-bromo-2-methylpropanoate was mixed at 1 vol%, and the glass substrate was immersed in the solution. The glass substrate was allowed to stand at 25°C for 1 hour, then washed with EtOH, and dried in an oven at 90°C for 2 hours to introduce an amino group and an ATRP starting group on the surface of the glass substrate.

5 mg/mL of a succinic anhydride solution (solvent = THF : TEA = 95 : 5) was prepared and the substrate described above was immersed therein. After allowing the glass substrate to stand at 25°C for 4 hours, the glass substrate was washed with pure water, and the position of the amino group on the surface of the glass substrate was defined as a carboxyl group.

The substrate was immersed in a 1 mM 2-bromoisobutanoic acid N-hydroxysuccinimide ester solution (solvent = DMF), reacted at 25°C for 1 hour, and then washed with EtOH. Thus, a glass substrate having an ATRP starting group and a carboxyl group on a surface thereof was obtained.

### 4-1-2. Preparation of particle-immobilized substrate

Each of a silica nanoparticle dispersion (particle size: 200 nm, particle concentration: 2.0 × 10¹¹ particles/mL) and the polymer E2 (1.0 mg/mL) was mixed in water at the above-described final concentration to form a complex.

The complex solution was diluted with DMF, dropped (4 µL) on the area of the substrate on which the complex was immobilized as shown in Table 4-1, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 4-1.

**[Table 4-1]**

| Table 4-1 Experiment condition table | | |
|---|---|---|
| No. | Number of particles [particles/4 µL] | Fluorescence microscope measurement conditions (exposure time) |
| | | 60x objective lens |
| 1 | 3×10⁷ | ? |
| 2 | 1×10⁷ | ↑ |
| 3 | 3×10⁶ | ↑ |
| 4 | 1×10⁶ | ↑ |
| 5 | 3×10⁵ | ↑ |
| 6 | 1×10⁵ | ↑ |
| 7 | 3×10⁴ | ↑ |
| 8 | 1×10⁴ | ↑ |

### 4-2. Results

### 4-2-1. Preparation of particle-immobilized substrate

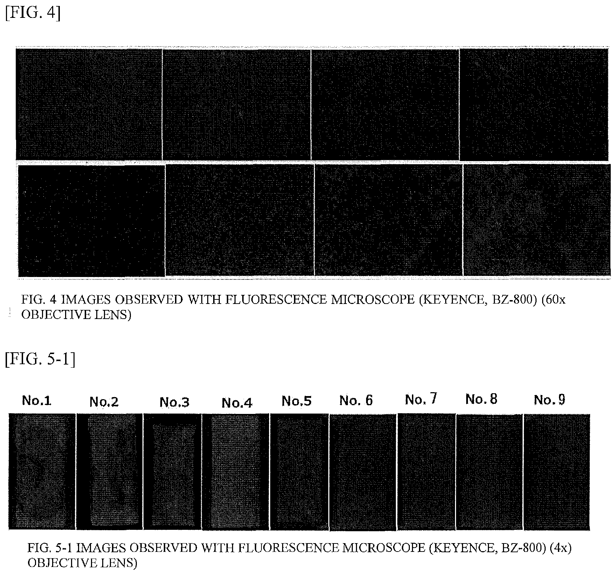

The observation results with a fluorescence microscope are illustrated in FIG. 4. From this result, it was found that the particle form on the substrate was controlled depending on the number of drops to the substrate even with different substrates.

### (Example 5: Preparation of measurement substrate (polystyrene particles))

### 5. Examination using polystyrene particles

### 5-1. Experiment

### 5-1-1. Surface modification of gold substrate

A gold substrate (9.8 mm × 4.3 mm) was rinsed with EtOH and then subjected to UV-O3 treatment for 15 min to clean up a substrate surface.

A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 5-1-2. Preparation of particle-immobilized substrate

A polystyrene particle dispersion (particle size: 250 nm, particle concentration: 2.0 × 10¹¹ particles/mL) and the polymer E2 (1.0 mg/mL) were mixed in water at the above-described final concentration to form a complex.

The complex solution was diluted with DMF, dropped (4 µL) on the area of the substrate on which the complex was immobilized as shown in Table 5-1, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 5-1.

**[Table 5-1]**

| Table 5-1 Experiment condition table | | | | |
|---|---|---|---|---|
| No. | Number of particles [particles/4 µL] | Fluorescence microscope measurement conditions (exposure time) | | |
| | | 4x objective lens | 22.2x objective lens | 60x objective lens |
| 1 | 1×10⁸ | 0.2 sec | 0.05 sec | - |
| 2 | 3×10⁷ | ↑ | ↑ | - |
| 3 | 1×10⁷ | ↑ | ↑ | 0.005 sec |
| 4 | 3×10⁶ | 1 sec | 0.2 sec | ↑ |
| 5 | 1×10⁶ | ↑ | ↑ | ↑ |
| 6 | 3×10⁵ | ↑ | ↑ | ↑ |
| 7 | 1×10⁵ | ↑ | ↑ | ↑ |
| 8 | 3×10⁴ | ↑ | ↑ | - |
| 9 | 1×10⁴ | ↑ | ↑ | - |

### 5-2. Results

### 5-2-1. Preparation of particle-immobilized substrate

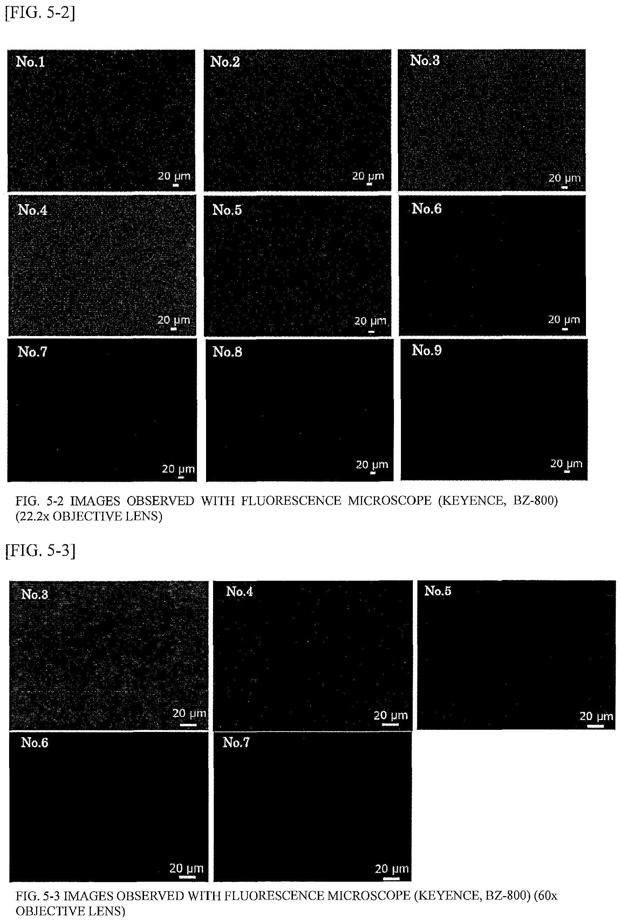

The observation results with a fluorescence microscope are illustrated in FIGS. 5-1 and 5-2. The fluorescence derived from the polystyrene particles was confirmed on the substrate under any conditions, and thus it was confirmed that the polystyrene particles (polymer complex) were immobilized on the substrate.

In addition, the obtained fluorescence microscopic image was analyzed using software (hybrid cell count), and the results are shown in Table 5-2. As a result of the analysis, the number of polystyrene particles on the substrate tended to increase with an increase in the number of particles to be dropped, and particularly under the condition where the number of drops was large (No. 1 and No. 2), the number of bright spots decreased as compared with No. 3, and thus it was found that the number of aggregates increased.

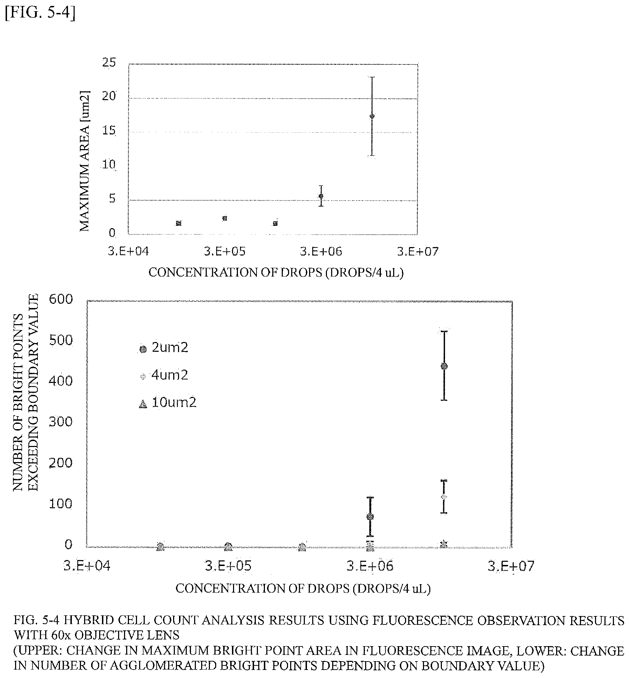

Further, in No. 1 (1 × 10⁸ particles/4 µL) and No. 2 (3 × 10⁷ particles/4 µL), the degree of agglomeration of the particles on the substrate was large, and the uniformity was poor. On the other hand, in No. 8 (3 × 10⁴ particles/4 µL) and No. 9 (1 × 10⁴ particles/4 µL), the immobilization amount of the polystyrene particles was extremely small. Therefore, in order to perform detailed examination in No. 3 (1 × 10⁷ particles/4 µL) to No. 7 (1 × 10⁵ particles/4 µL), analysis was performed using fluorescence observation with a 60x objective lens and hybrid cell count, and the results thereof are shown in FIGS. 5-3 and 5-4, respectively.

From FIG. 5-3, it was found that the maximum bright spot area increased as the number of drops increased, and the scale of agglomeration increased. In addition, in No. 6 and No. 7 in the drawing in which it was clearly determined from the image that the particles were monodispersed, not agglomerated, and in a single layer, since the area of the bright spots was about 2 µm², the particles having a bright spot area exceeding this were determined to be an aggregate. By setting the numerical value as a boundary value, the number of bright spots equal to or larger than the boundary value determined as 2 µm²·4 µm²·10 µm² is illustrated in FIG. 5-4. From this result, agglomeration occurred at No. 4 (3 × 10⁶ particles/4 µL) or more where bright spots exceeding the boundary value 2 µm² occurred and no agglomeration occurred at No. 5 (1 × 10⁶ particles/4 µL) or less, and it was possible to control the particle form on the substrate depending on the number of drops on the substrate.

**[Table 5-2]**

| Table 5-2 Hybrid cell count analysis using images captured with 22.2x objective lens | | | | | |
|---|---|---|---|---|---|
| Concentration of drops | Total number of bright spots | Number of monodispersed bright spots ( ≤ 10 µm²) | Number of agglomerated bright spots ( ≤ 10 µm²) | Maximum bright spot area [µm²] | Total bright spot area [µm²] |
| 1×10⁸ | 4059 | 3716 | 343 | 454.911 | 12072.21 |
| 3×10⁷ | 9152 | 7648 | 1504 | 223 | 29613 |
| 1×10⁷ | 16441 | 14889 | 1552 | 423 | 33967 |
| 3×10⁶ | 8472 | 7364 | 1108 | 150 | 20317 |
| 1×10⁶ | 4289 | 3377 | 912 | 39.029 | 20715.15 |
| 3×10⁵ | 258 | 181 | 77 | 57 | 1145 |
| 1×10⁵ | 61 | 37 | 24 | 20.412 | 191.914 |
| 3×10⁴ | 30 | 26 | 4 | 15 | 89 |
| 1×10⁴ | 12 | 9 | 3 | 22 | 66 |

### (Example 6: Performance test (evaluation of particle density on substrate))

6A Density of concave portions on substrate

### Examination using fluorescent cationic polymer

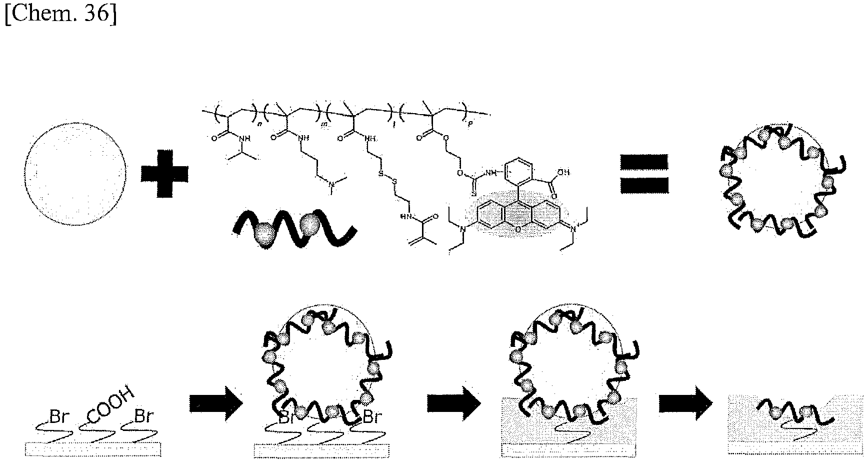

### 6-1. Experiment

### 6-1-1. Surface modification of gold substrate

A gold substrate (9.8 mm × 4.3 mm) was rinsed with EtOH and then subjected to UV-O3 treatment for 15 min to clean up a substrate surface.

A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 6-1-2. Preparation of particle-immobilized substrate

A silica nanoparticle dispersion (particle size: 200 nm, particle concentration: 2.0 × 10¹¹ particles/mL) and the polymer F1 (1.0 mg/mL) were mixed in water at the above-described final concentration to form a complex.

The complex solution was diluted with DMF, dropped (4 µL) on the area of the substrate on which the complex was immobilized as shown in Table 6-1, and allowed to stand at 25°C for 1 hour.

The substrate was washed with DMF, EtOH, and pure water in this order to obtain a particle-immobilized substrate.

The substrate was evaluated with a fluorescence microscope (KEYENCE, BZ-800), and analyzed using software (hybrid cell count). The measurement conditions are shown in Table 6-1.

**[Table 6-1]**

| Table 6-1 Experiment condition table (polymer Fl) | | | | | | | |
|---|---|---|---|---|---|---|---|
| No. | Number of particles [particles/4 µL] | Fluorescence microscope measurement conditions (exposure time) | | | | | |
| | | Green | | | Red | | |
| | | After particle immobilization | After polymerization | After template removal | After p article immobilization | After polymerization | After template removal |
| 1 | 1×10⁷ | 0.1 sec | 0.1 sec | 0.2 sec | 0.01 sec | 0.01 sec | 0.02 sec |
| 2 | 3×10⁶ | ↑ | ↑ | ↑ | ↑ | ↑ | ↑ |
| 3 | 1×10⁶ | ↑ | ↑ | 0.5 see | ↑ | ↑ | 0.05 sec |

### 6-1-3. Synthesis of silica nanoparticle imprinted polymer on particle-immobilized substrate

Prepolymer solutions having compositions shown in Table 6-2 were prepared, dissolved oxygen was removed by freeze-degassing, and then polymerization was performed at 25°C for 18 hours in a glove box. The substrate after polymerization was washed with pure water.

The substrate was immersed in a solution of methanol: pure water = 1 : 1, and an ultrasonic treatment was performed for 0.5 min to remove silica nanoparticles, thereby forming pores in a surface layer of a polymer thin film.

**[Table 6-2]**

| Table 6-2 Prepolymer solution composition | | |
|---|---|---|
| | Resource | Concentration |
| Main raw material | MPC (2-methacryloyloxyethyl phosphorylcholine) | 500 mM |
| Initiator | EBIB (ethyl *α*-bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ*L |

### 6-2. Results

### 6-2-1. Preparation of particle-immobilized substrate

Particle size with Zeta Sizer for complex of silica nanoparticles and fluorescent cationic polymer F1
- The results of performing the surface Z potential measurement are shown in Table 6-3. Since the surface Z potential changed from negative to positive before and after formation of the complex, it was confirmed that the silica nanoparticles and the fluorescent cationic polymer F1 formed a complex, and it was also confirmed from the particle size and the PDI that the particles were not agglomerated and the dispersibility was not impaired.

**[Table 6-3]**

| Table 6-3 Particle size and surface Z-potential measurement results by Zeta Sizer | | | |
|---|---|---|---|
| Object to be measured | Particle size | PDI | Surface Z-potential |
| Silica nanoparticle | 201.2 nm | 0.01824 | -29.97 mV |
| Silica nanoparticle + F1 complex | 225.8 nm | 0.1005 | 57.94 mV |

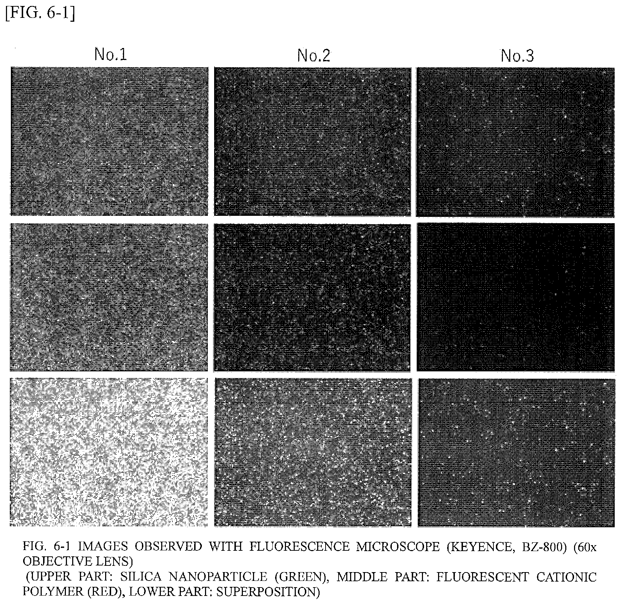

In addition, the observation results with a fluorescence microscope are illustrated in FIG. 6-1. From the fact that green fluorescence derived from the silica nanoparticles and red fluorescence derived from the fluorescent cationic polymer were confirmed in all the samples, it was confirmed that the silica nanoparticle-fluorescent cationic polymer complex was immobilized on the substrate.

### 6-2-2. Confirmation of fluorescent cationic polymer F1 after removal of silica nanoparticles

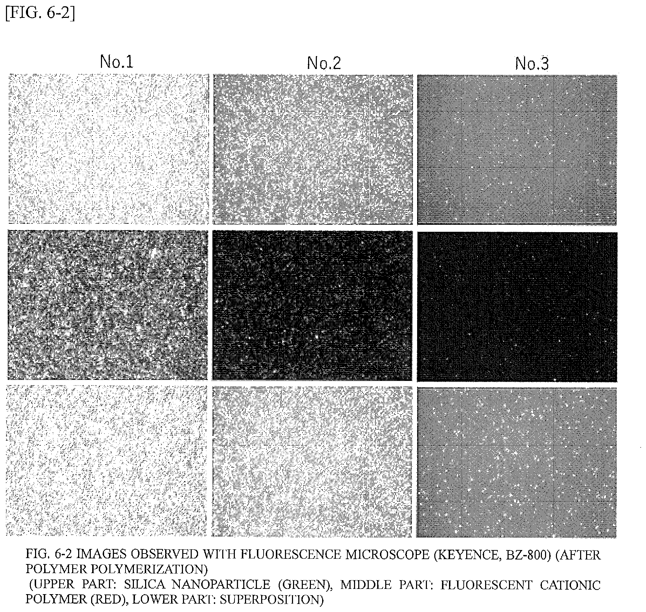

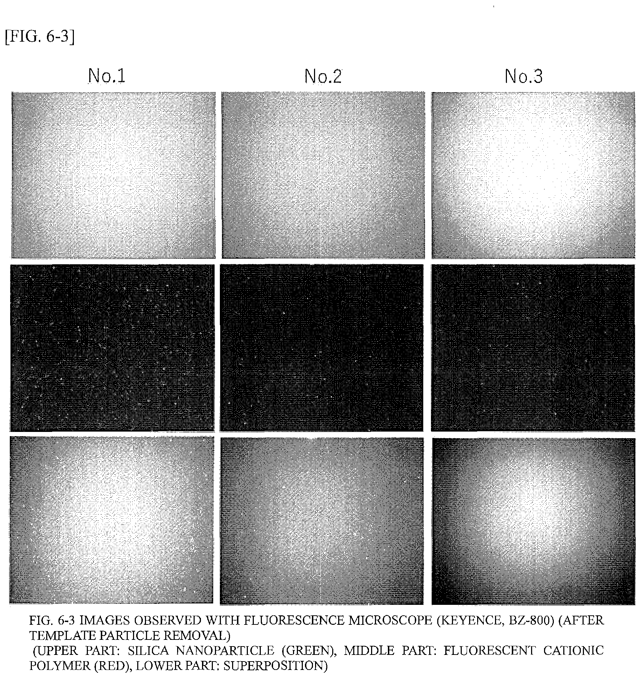

The results of observation with a fluorescence microscope after polymerization of the polymer layer on the substrate after immobilization of the silica nanoparticle-fluorescent cationic polymer complex and the results of observation with a fluorescence microscope after removal of the silica nanoparticles after polymerization are illustrated in FIGS. 6-2 and 6-3, respectively.

From FIG. 6-2, the fluorescence derived from the silica nanoparticles and the fluorescent cationic polymer was confirmed on the substrate even after polymerization. However, from FIG. 6-3 after removal of the silica nanoparticles, the green fluorescence derived from the silica nanoparticles was confirmed, but the red fluorescence derived from the fluorescent cationic polymer was confirmed. From this, it was confirmed that the fluorescent cationic polymer remained in the polymer layer (in the pores after removal of silica nanoparticles) prepared on the substrate even after the silica nanoparticles were removed by an experimental operation.

In addition, since the red fluorescence remaining on the substrate after the removal of the silica particles was derived from the cationic polymer present in the pores after the removal of the silica particles, the pore density on the substrate was calculated as the number of bright spots of the red fluorescence = the number of pores. As a result, the pore density formed on the substrate under the present experimental conditions correlated with the number of silica particles dropped on the substrate, and was in a range of 10³ to 10⁶ particles/mm². From this, it was found that the pore density after removal of silica particles was also controlled in addition to the particle form on the substrate depending on the number of particles to be dropped on the substrate.

### 6. B Particle density on substrate

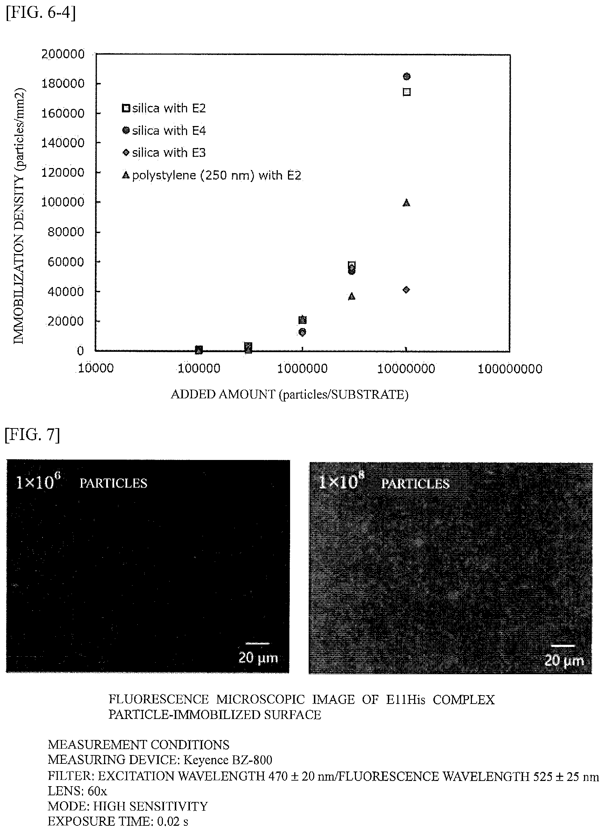

From FIGS. 3-1, 3-2, and 5-1 illustrating the results of analyzing the particles on the measurement substrate obtained in Examples 2, 3, and 5 using fluorescence observation with a 60x objective lens and hybrid cell count, it was found that the number of silica nanoparticles on the substrate tended to increase as the number of drops increased. In addition, in No. 4 and No. 5 in which it was clearly determined from the image that the particles were monodispersed, not agglomerated, and in a single layer, since there were almost no bright spots with an area of more than 2 µm², the particles having a bright spot area exceeding this were determined to be an aggregate. By setting the numerical value as a boundary value, it was possible to objectively express by a numerical value that agglomeration occurred at No. 4 (3 × 10⁶ particles/4 µL) or more where bright spots exceeding the boundary value 2 µm² occurred and no agglomeration occurred at No. 5 (1 × 10⁶ particles/4 µL) or less, and it was possible to control the particle form on the substrate depending on the number of drops on the substrate. The results of analyzing the particles present on the measurement substrate were summarized in FIG. 6-4. When the particles were scattered at a concentration of No. 5 (1 × 10⁶ particles/4 µL) or less at which agglomeration did not occur, and when the particles were present at a particle density of 1 × 10⁵ particles/mm² or less on the substrate, the particles were monodispersed, not agglomerated, and disposed in a single layer on the substrate.

### (Example 7: Complexation of His-Tag polymer and silica nanoparticles)

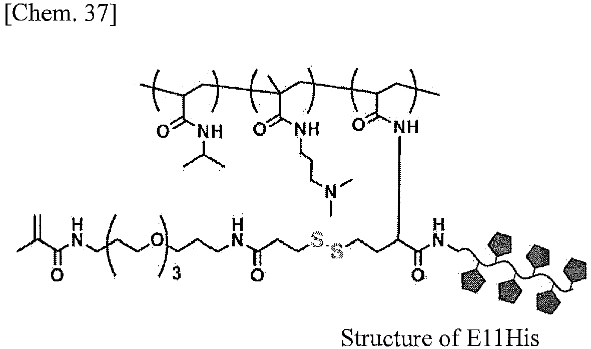

**[Table B]**

| | Mw. | | | |
|---|---|---|---|---|
| Sample | | Concent ration (mM) | Final liquid volume (mL) | Weight (mg) |
| N-Acryloylhomocysteine thiolactone | 171.22 | 100 | 1 | 17 |
| N-(3-(Dimethylamino)propyl)methacrylamide | 170.25 | 200 | 1 | 35 |
| NIPAm | 113. 16 | 700 | 1 | 79 |
| 2-Cyano-2-[(dodecylsulfanylthiocarbonyl)sulfanyl]propane | 345.63 | 10 | 1 | 3.5 |
| AIBN | 164.21 | 5 | 1 | 0.8 |

The compound of the recipe was placed in a Schlenk flask (25 mL) and dissolved in DMF (final liquid volume: 1 mL). A RAFT agent and an initiator were added last. A stirrer was placed, degassing and replacement with argon were performed, and polymerization was performed in an oil bath (set temperature: 70°C) (17 h).

### (Stirring speed: 300 rpm)

After the polymerization, the reaction was stopped by ice-cooling the Schlenk flask and introducing air. The reaction solution was added to a large amount of diethyl ether, and the generated precipitate was collected. The precipitate was washed with diethyl ether and vacuum-dried.

5 mg of the obtained polymer, 4 mg of a His-tag (having a structure in which six histidines were bonded from the N-terminal, three glycines were bonded, and a lysine was bonded to the C-terminal, and a carboxyl group at the C-terminal was amidated),

3.5 mg of pyridyldithioethyl-PEG3-methacrylamide, and 2.8 µL of TEA were dissolved in DMF (0.3 mL), and stirring was performed for 9 hours. CH₂Cl₂ and n-hexane were added to the reaction solution, and the generated precipitate was collected and vacuum-dried to obtain a polymer (E11His).

### iii) E11His

On a gold substrate modified with 2-(2-bromoisobutyryloxy)undecyl thiol and an NTA-SAM formation regent, a 4 mM NiClz aqueous solution was dropped and allowed to stand (room temperature, 15 min), and then the substrate was washed with pure water. A mixed solution (silica concentration 2.0 × 10¹¹ particles/mL, polymer concentration 1 mg/mL) of silica nanoparticles (200 nm, COOH-terminated) and an E11 His aqueous solution was diluted 4-fold or 400-fold with DMF. 4 µL of the solution was dropped on the gold substrate after the reaction with NiCl₂ and allowed to stand for 1 hour. Thereafter, the surface was washed with EtOH and observed with a fluorescence microscope. The results are illustrated in FIG. 7.

### (Example 8: Cationic silica nanoparticle + anionic polymer Ex8)

### 1. Method for synthesizing anionic polymer Ex8

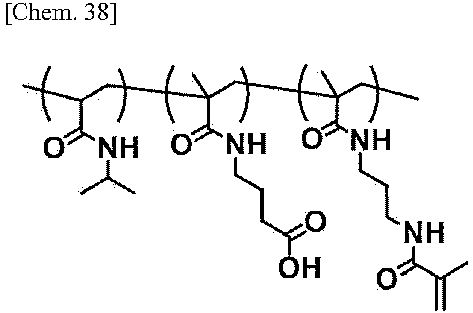

The compound of the recipe in the table was placed in a Schlenk flask (25 mL) and dissolved in DMF (final liquid volume: 1 mL). A RAFT agent and an initiator were added at the end, a stirrer was placed, degassing and replacement with argon were performed, and polymerization was performed in an oil bath (set temperature: 70°C). (16 h)

After the polymerization, the reaction was stopped by ice-cooling the Schlenk flask and adding air, and a precipitate generated by adding a large amount of diethyl ether to the reaction solution was collected. The collected precipitate was washed with diethyl ether and then vacuum-dried to obtain a polymer.

The obtained polymer and 70 µL (0.5 mmol) of TEA were dissolved in CH₂Cl₂, stirring was performed under ice cooling, 54 mg (0.3 mmol) of N-succinimidyl methacrylate was added thereto, and stirring was performed overnight. Thereafter, a precipitate generated by adding n-hexane to the reaction solution was collected. The precipitate was redissolved in a small amount of CH₂Cl₂ and an excessive amount of n-hexane was added to generate a precipitate, and the precipitate was collected and vacuum-dried to obtain a target polymer (Ex8).

### Yield: 120 mg

**[Table C]**

| | **Mw.** | | |
|---|---|---|---|
| Sample | | Concen tration (mM) | Final liquid volume (mL) |
| N-(3-Aminopropyl)methacrylamide hydrochloride | 178.66 | 100 | 1 |
| 4-[(2-Methyl-1-oxo-2-propen-1-yl)amino]butanoic acid | 171.19 | 200 | 1 |
| Isopropylacrylamide (NIPAm) | 113.16 | 700 | 1 |
| 2-Cyano-2-[(dodecylsulfanylthiocarbonyl) sulfanyl]propane | 345.63 | 10 | 1 |
| AIBN | 164.21 | 5 | 1 |

### 2. Complex of silica nanoparticles and polymer

After 1.98 mg (39.6 µL) of silica (Red-plane, 200 nm, 50 mg/mL) and 60.4 µL (total 100 uL) of pure water were mixed and added to 800 uL of EtOH contained in 1.5 mL Eppendorf, 100 uL of 2 M HCl and 15 uL of APTES were further added thereto, and the mixture was reacted with a thermoshaker at 25°C and 1,000 rpm for 20 h. After the reaction, the mixture was centrifugally washed with ethanol (5,000 rpm, 10 min × 3).

20 µL of the prepared silica nanoparticle dispersion (NH₂-terminated, 200 nm, 4.0 × 10¹¹ particles/mL, 50 mM carbonate buffer (pH 8.0)) and 20 µL of an Ex8 carbonate buffer solution (2 mg/mL) were mixed, and the mixed solution was allowed to stand at 25°C for 15 minutes.

### 3. DLS measurement

The complex of 2 was diluted 100-fold with pure water, and the particle size and Z-potential were measured using DLS.

### 4. Immobilization on substrate

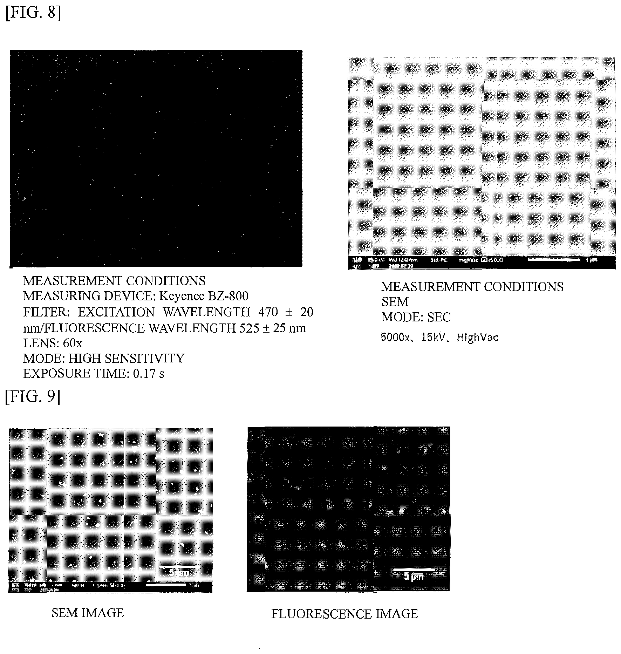

The complex of 2 was diluted 80-fold with DMF. 4 µL of the solution was dropped on a gold substrate modified with 2-(2-bromoisobutyryloxy)undecyl thiol:amino-EG6 undecanethiol (1:1 vol), and the substrate was allowed to stand at 25°C for 1 h. Thereafter, the surface was washed with EtOH, and the surface was observed with a fluorescence microscope and an SEM (FIG. 8).

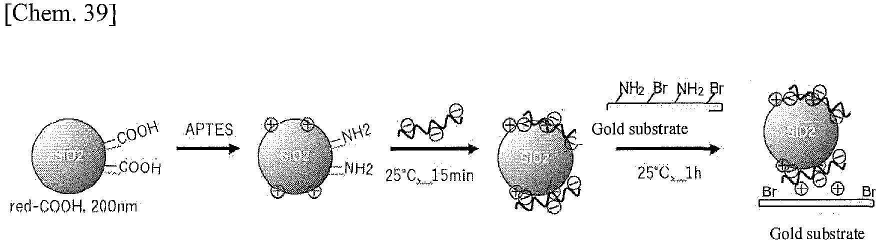

From the results of particle size measurement by DLS and the results of Z-potential measurement, it was suggested that the anionic polymer was adsorbed to the silica nanoparticles, and the surface was changed from a positively charged state to a negatively charged state. In addition, since there was no large change in particle size, it was shown that dispersibility was maintained even after polymer adsorption.

**[Table D]**

| | Silica nanoparticle | Ex8 complex |
|---|---|---|
| size (nm) | 221.5 | 251.7 |
| zeta (mV) | 34.4 | -14.4 |

### (Results)

From the observation results of FIG. 8, it was observed that the fluorescent bright spots derived from the silica nanoparticles were monodispersed, not agglomerated, and distributed in a single layer. From this, it was shown that even when the anionic polymer Ex8 and the cationic silica nanoparticles were complexed, the particles were immobilized on the substrate in a monodisperse form, without agglomeration, and in a single layer as in the case of the cationic polymer Ex8 and the anionic silica nanoparticles.

### (Example 9: Cationic nanoparticle + anionic polymer)

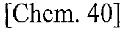

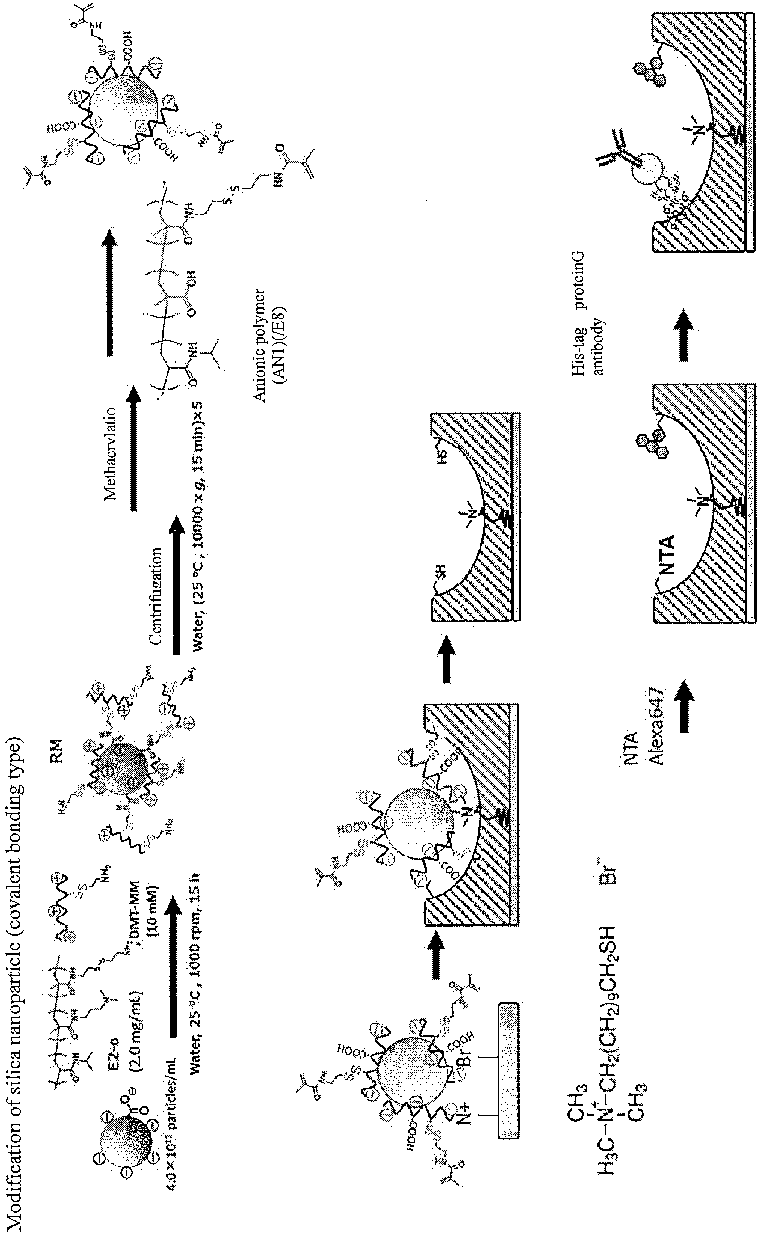

### (Silica nanoparticles used)

### • E2-0 conju red-COOH silica (RM) 200 nm

### 4.0 × 10¹¹ particles/ml

### (Polymer solution)

### • Polymer final concentration 0.87 mg/mL (10 mM PB, pH 7.47)

### (Anionic silica)

### • The silica nanoparticle dispersion and the polymer AN-1 were mixed to form a complex. (Silica final concentration 1.0 × 10¹¹ pieces/ml, polymer final concentration 0.435 mg/mL (5 mM PB))

The complex solution was diluted 4-fold, 40-fold, 400-fold, and 4,000-fold with DMF, dropped (4 µL) on the substrate on which the complex was immobilized, and allowed to stand at 25°C for 1 hour. The substrate was washed with ethanol to obtain a particle-immobilized substrate.

### (Polymer matrix synthesis)

### Form thin film by ATRP (25 °C, 20 h)

### (Removal source of particle core)

300 µL of 50% acetic acid/MeOH was placed in a 1.5 mL tube, and the substrates were immersed one by one, subjected to an ultrasonic treatment for 8 minutes, and then washed with pure water.

### (S-S reductive cleavage)

The substrate was placed in a 50 mM tris(2-carboxyethyl)phosphine hydrochloride 50% MeOH aqueous solution and reacted at 40°C for 1 h. The substrate after reaction was washed with pure water.

### (Introduction of fluorescent dye)

30 µL of a 50 µM Alexa 647-C2-maleimide and 50 µM maleimido-C3-NTA (5% DMSO 10 mM PB (7.4)) solution was dropped on a substrate, the substrate was allowed to stand at 25°C for 1 hour to allow a reaction to proceed, and then washing with pure water was performed.

A flat pipette tip manufactured by FUKAE KASEI Co., Ltd. was mounted, and a change in fluorescence intensity on the surface of the substrate was measured with an automatic dispensing device with a fluorescence microscope.

### Sequence

1. Chip attachment/PBS suction (150 µL) and image acquisition before antibody immobilization
2. Ni complex formation
   Suction of 100 µL of 4 mM NiCl₂ (aqueous solution), reaction 5 min (25°C)
   Wash pure water × 4 times and suction and discharge 150 µL
3. proteinG immobilization
   Suction of 100 µL of 1 µM His-proteinG (PBS, abeam), reaction 10 min (25°C)
   Wash
   0.01% tween (registered trademark) 20 PBS × 2 times and PBS × 2 times
   Suction and discharge 150 µL
4. Antibody immobilization
   Suction of 100 µL of 100 nM Anti-CD9 (commercially available product) (PBS), reaction 10 min (25°C)
   Wash
   0.01% tween (registered trademark) 20 PBS × 2 times and PBS × 2 times
   Suction and discharge 150 µL
5. Background (F0: 0.1% BSA, 100 mM PB (pH 7.0), 0.15 M NaCl) 5 times of measurements
6. Exosome adsorption
   SKBR3 culture supernatant exosome adjusted to 0, 3, 30, 300, and 1,000 fM (0.1% BSA, 100 mM PB (pH 7.0), 0.15 M NaCl)
   Suction of 100 µL of sample (SKBR3-derived exosome), reaction 5 min (25°C)
   Image acquisition
   Fluorescence microscope
   Camera: Zyla 4.2
   Filter: Cy5
   (Excitation wavelength 604 to 644 nm, fluorescence wavelength 672 to 712 nm)
   Objective lens: ×5
   Exposure time: 0.5 sec
   Light amount: 12%
   Light source: mercury lamp
   Suction and discharge 150 µL of 0.01% tween (registered trademark) 20 PBS
   Repeat 1 to 6

### (Results)

For a sensing chip produced using the anionic polymer-modified silica particles for the substrate on the cationic surface, a relative fluorescence intensity change of about 5% was confirmed by addition of 10 fM of an exosome derived from SK-BR3 cell line.

### (Example 10: Biodegradable nanoparticles:polylactic acid/glycolic acid copolymer (PGLA))

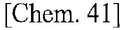

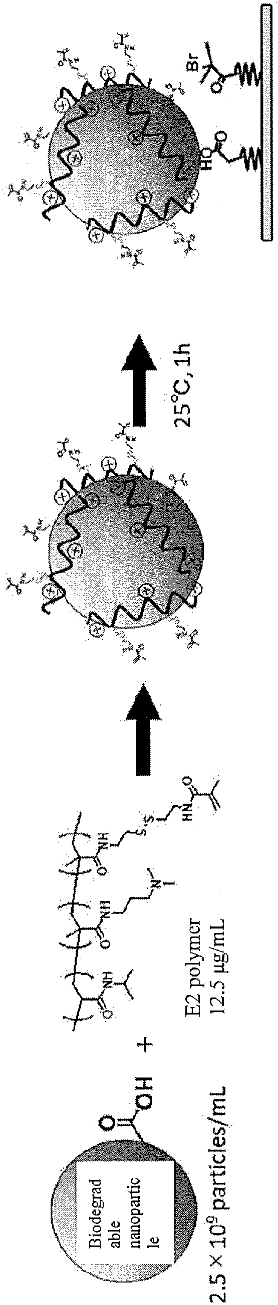

### (SAM formation on substrate)

A gold substrate was immersed in an EtOH solution with 0.5 mM 2-(2-bromoisobutyryloxy) undecyl thiol and 0.5 mM carboxy-EG6 undecane thiol to form SAM (30°C, 20 h)

### (Preparation of particles)

After the fluorescent carboxylated PGLA nanoparticle (200 nm) aqueous suspension was ultrasonicated (Lv2) for 1 min, the nanoparticles and the E2 polymer aqueous solution were mixed so that the final concentration was 2 × 10¹¹ particles/mL and 1 mg/mL, and 4 µL of a solution diluted 80-fold with EtOH was dropped on the substrate (1.0 × 10⁷ particles/4 µL) (25°C, 1 h), allowed to stand, and then washed three times with EtOH.

### (SEM observation and fluorescence observation)

After the E2 polymer aqueous solution was mixed so as to be 1 mg/mL, the solution was diluted with EtOH, 4 µL of the solution was dropped on the substrate (1.0 × 10⁷ particles/4 µL) (25°C, 1 h), and the substrate was allowed to stand and then washed with EtOH three times.

Fluorescence observation on the substrate in EtOH was performed (KEYENCE BZ-X800, excitation wavelength 470 ± 20 nm/fluorescence wavelength 525 ± 25 nm). Thereafter, vacuum drying was performed, gold sputtering was performed, and then, a scanning electron microscope (SEM) image was observed. (FIG. 9)

### (Results)

The results showed that the state was comparable to that of the silica nanoparticles or polystyrene nanoparticles, and it was found that this method was applicable to all types of core particles.

### (Example 11: Silica nanoparticle + cationic polymer E2P20 containing aromatic component)

### 1. E2P20 synthesis method

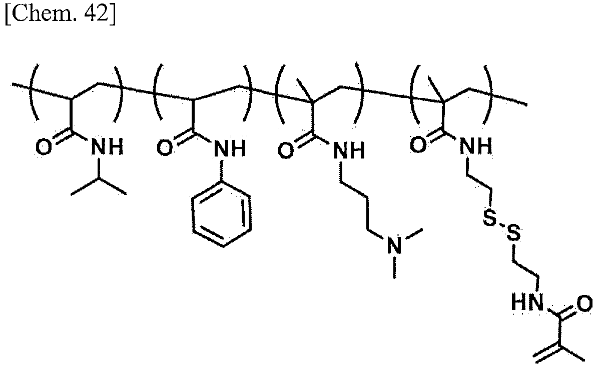

A cationic polymer E2P20 containing an aromatic component (phenyl group) was synthesized as follows.

The compound of the recipe was placed in a Schlenk flask (25 mL) and dissolved in DMF (final liquid volume: 2 mL). A RAFT agent and an initiator were added last. A stirrer was placed, degassing and replacement with argon were performed, and polymerization was performed in an oil bath (set temperature: 75°C) (24 h).

### (Stirring speed: 300 rpm)

After the polymerization, the reaction was stopped by ice-cooling the Schlenk flask and introducing air. The reaction solution was added to a large amount of diethyl ether, and the generated precipitate was collected. The precipitate was washed with diethyl ether and vacuum-dried.

The polymer prepared in the previous section and 42 µL of TEA were dissolved (or dispersed) in CH₂Cl₂, and stirring was performed. 37 mg of N-succinimidyl methacrylate was added thereto, and stirring was performed overnight. After the reaction, a precipitate generated by adding n-hexane to the reaction solution was collected. A precipitate generated by adding MeOH, CH₂Cl₂, and n-hexane to the precipitate was collected and vacuum-dried to obtain a target polymer (E2P20).

### Yield: 110 mg

**[Table E]**

| Sample | Mw. | Concen tration (mM) | Final liquid volume (mL) | Weight (mg) | liquid volume (µL) |
|---|---|---|---|---|---|
| Cys metacrylamide | 256.82 | 100 | 2 | 51.364 | - |
| N-(3-(Dimethylamino)propyl)methacrylamide | 170.25 | 100 | 2 | 34.050 | 36.223 |
| N-Phenylacrylamide | 147.18 | 100 | 2 | 29.436 | |
| N-isopropylacrylamide(NIPAm) | 113.16 | 200 | 2 | 45.264 | - |
| 2-Cyano-2-[(dodecylsulfanylthiocarbonyl) sulfanyl]propane | 345.63 | 12.5 | 2 | 8.641 | - |
| AIBN | 164.21 | 6.25 | 2 | 2.053 | - |

### 2. Complex of silica nanoparticles and polymer

40 µL of a silica nanoparticle aqueous dispersion (COOH-terminated, 200 nm, Lot.0921940-03, 4.0 × 10¹¹ particles/mL) and 40 µL of an E2P20 aqueous solution (2 mg/mL) were mixed, and the mixed solution was allowed to stand at 25°C for 15 minutes, thereby forming a complex of silica nanoparticles and E2P20.

### 3. DLS measurement

The complex of silica nanoparticles and E2P20 was diluted 100-fold with pure water, and the particle size and Z-potential were measured using DLS.

### 4. Immobilization on substrate

The complex of silica nanoparticles and E2P20 was diluted 80-fold with DMF, 4 µL of the diluted complex was dropped on a gold substrate modified with 2-(2-bromoisobutyryloxy)undecyl thiol : carboxy-EG6 undecanethiol (1 : 1), and the substrate was allowed to stand at 25°C for 1 h. Thereafter, the surface was washed with EtOH, and the surface was observed with a fluorescence microscope and an SEM.

From the results of particle size measurement by DLS and the results of Z-potential measurement, it was suggested that the cationic polymer was adsorbed to the silica nanoparticles, and the surface was changed from a negatively charged state to a positively charged state. In addition, since there was no large change in particle size, it was shown that dispersibility was maintained even after polymer adsorption.

**[Table F]**

| | Silica nanoparticle | After E2P20 complexation |
|---|---|---|
| size (nm) | 210.1 | 223.2 |
| zeta (mV) | -41.2 | 45.8 |

### (Results)

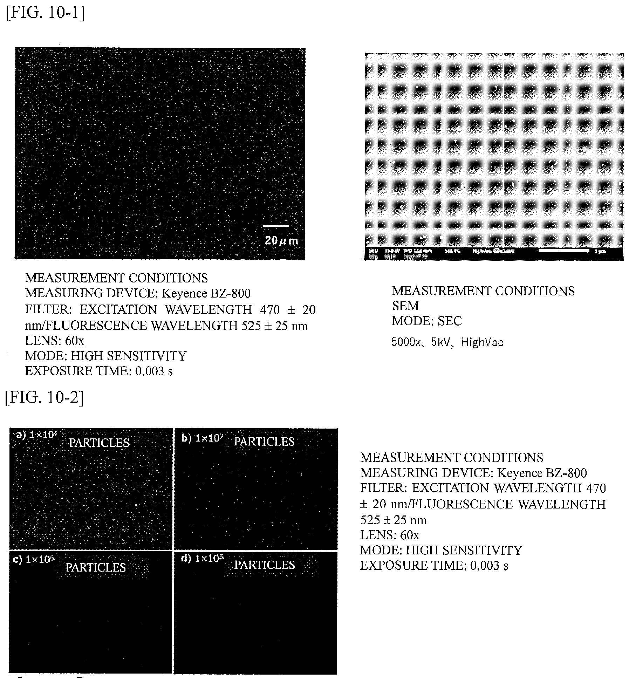

From the observation results illustrated in FIG. 10-1, it was observed that the fluorescent bright spots derived from the silica nanoparticles were monodispersed, not agglomerated, and distributed in a single layer. The results showed that when E2P20 in which an aromatic component (phenyl group) was introduced into E2 was complexed with silica nanoparticles and immobilized on a substrate as in the case of E2, E2P20 was monodispersed, not agglomerated, and was immobilized on the substrate in a single layer.

### (Example 12: Polystyrene nanoparticle + cationic polymer E2P20 containing aromatic component)

A cationic polymer E2P20 containing an aromatic component was complexed using polystyrene nanoparticles instead of silica nanoparticles in Example 11. 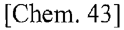 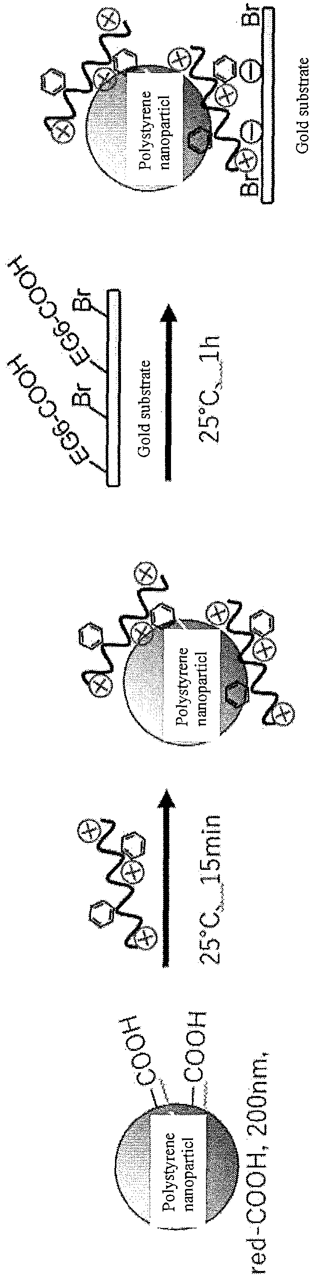 20 µL of polystyrene (PS) nanoparticles (250 nm, COOH-terminated, 2.0 × 10¹² particles/mL) and 20 µL of an E2P20 aqueous solution (2 mg/mL) were mixed, and the solution was diluted 40-fold, 400-fold, 4,000-fold, and 40,000-fold with DMF. 4 µL of the solution was dropped on a gold substrate modified with 2-(2-bromoisobutyryloxy)undecyl thiol and carboxy-EG₆ undecanethiol, and the substrate was allowed to stand for 1 hour. Thereafter, the surface was washed with EtOH and observed with a fluorescence microscope. (FIG. 10-2)

### (Results)

As in the case of the silica nanoparticles, it was observed that even when polystyrene nanoparticles were used, the fluorescent bright spots were distributed in a monodisperse form, without agglomeration, and in a single layer regardless of the number of dropped particles. From this, it was shown that even when an aromatic component was introduced into a cationic polymer, the aromatic component was immobilized in a monodisperse form, without agglomeration, and in a single layer.

### (Example 13: Performance test (sensor sensitivity), confirmation of reproducibility) 13-1. Experiment

### 13-1-1. Surface modification of gold substrate

A gold substrate (9.8 mm × 4.3 mm) was washed with EtOH and then subjected to UV-O3 treatment for 15 min to wash a substrate surface.

A 1 mM EtOH solution was prepared by mixing 2-(2-bromoisobutyryloxy)undecyl thiol and carboxy-EG₆ undecanethiol at a ratio of 1 : 1 in an EtOH solvent, and the gold substrate was immersed in the solution. After the substrate was allowed to stand at 30°C for 20 hours, the substrate was washed with EtOH and pure water to form a self-assembled monolayer (SAM) on the surface of the gold substrate.

### 13-1-2. Preparation of particle-immobilized substrate

A silica nanoparticle aqueous dispersion (terminal functional group: COOH, particle size: 200 nm, particle concentration: 4.0 × 10¹¹ particles/mL) and a polymer E2 aqueous solution (2.0 mg/mL) were mixed at 1/1 (v/v) to form a complex.

The complex solution was diluted with DMF, dropped (4 µL) on the substrate on which the complex was immobilized so as to be 1 × 10⁸, 1 × 10⁷, 1 × 10⁶, and 1 × 10⁵ pieces, and allowed to stand at 25°C for 1 hour. The substrate was washed with DMF and EtOH in this order to obtain a particle-immobilized substrate. For each addition condition, 10 substrates are prepared.

### 13-1-3. Synthesis of polymer matrix and analysis sensor on particle-immobilized substrate

Prepolymer solutions having compositions shown in Table 7 were prepared, dissolved oxygen was removed by freeze-degassing, and then polymerization was performed at 25°C for 20 hours in a glove box replaced with Ar gas. The substrate after polymerization was washed with a large amount of pure water.

The substrate after polymerization was immersed in an AcOH/MeOH (1/1, v/v) solution and irradiated with ultrasonic waves (5 min). Thereafter, the substrate was washed with MeOH and pure water to remove the silica nanoparticles, and a concave portion was formed in the polymer thin film.

Thereafter, the substrate was immersed in a 50 mM Tris(2-carboxyethyl)phosphine hydrochloride solution (methanol/pure water = 1/1, v/v), and subjected to an ultrasonic treatment for 5 minutes to remove the E2 polymer and expose the thiol group.

**[Table 7]**

| Table 7 Prepolymer solution composition | | |
|---|---|---|
| | Sample | Concentration |
| Main raw material | MPC (2-methacryloyloxyethyl phosphorylcholine) | 500 mM |
| Initiator | EBIB (ethyl *α* -bromoisobutyrate) | 1.25 mM |
| Ligand | TPMA (tris(2-pyridylmethyl)amine) | 0.125 mM |
| Catalyst | CuBr₂ | 0.0125 mM |
| Reducing agent | Ascorbic acid | 1.25 mM |
| Solvent | EtOH | 270 *µ*L |

50 µM Alexa Fluor(R) 647 C2 maleimide and a 50 µM maleimide C3 NTA PBS solution (30 µL) were dropped on the substrate, the substrate was allowed to stand at 25°C for 1 hour, and fluorescent molecules and NTA groups were introduced into the concave portions. The substrate after the reaction was washed with MeOH and pure water.

A 4 mM NiCh aqueous solution (30 µL) was dropped on the substrate, and the substrate was allowed to stand at 25°C for 30 minutes to form a Ni-NTA complex. The substrate after reaction was washed with pure water. Thereafter, a 1 µM His-tag-modified proteinG PBS solution (30 µL) was dropped on the substrate, and the substrate was allowed to stand at 25°C for 1 hour, and proteinG was introduced using complex formation of Ni-NTA and a His-tag. Finally, a 0.3 µM anti-CD9 antibody PBS solution (30 µL) was added dropped on the substrate, and the substrate was allowed to stand at 25°C for 1 hour to construct an analysis sensor.

### 13-1-4. Adsorption experiment

Using a fluorescence microscope equipped with an automatic dispensing device (manufactured by System Instruments Co., Ltd.), an experiment of capturing an exosome of a frontline cancer cell (PC-3) was performed. The concentration of the PC-3 exosome PBS (10 mM phosphate, 140 mM NaCl, pH 7.4) solution was set to 0 and 1.0 fM. Measurement conditions of the fluorescence microscope were as follows: a filter was Cy5 (excitation wavelength of 604 to 644 nm, fluorescence wavelength of 672 to 712 nm), an objective lens was 5 times, an exposure time was 0.2 sec, a light amount was 12%, and a light source was a mercury lamp. Measurement was performed in the range of the central portion of the substrate. The sequence of the automatic dispensing device was: 1. chip attachment, 2. sample suction (100 µL), 3. reaction (1 min, 25°C), 4. full discharge, 5. suction of 150 µL of 10 mM PBS (140 mM NaCl, pH 7.4), and 6. measurement position keep (then, repeat 2 → 6).

The initial fluorescence intensity (F₀) and the relative fluorescence intensity change at the time of addition of the exosome were measured for each of 10 analysis sensors produced using a substrate in which the number of immobilized particles was 1 × 10⁸, 1 × 10⁷, 1 × 10⁶, or 1 × 10⁵. The coefficient of variation (= standard deviation/average value [%]) was calculated from the average value and the standard deviation of the relative fluorescence intensity change of each analysis sensor, and the influence of the number of immobilized particles on the reproducibility was examined by observing the correlation between the number of immobilized particles and the coefficient of variation of the produced analysis sensor.

### (Comparative Example 1: Silica nanoparticle agglomeration immobilization under conditions of WO 2018/221271 A and Toshifumi Takeuchi et al., J. Am. Chem. Soc., March 10, 2020, Vol. 142, Page. 6617-6624)

### 1. Introduction of His-tag and thiol group to silica nanoparticles

Fluorescent silica nanoparticles (red-COOH, 200 nm, Lot. 0442240-01) were adjusted to 25 mg/mL (3.0 × 10¹² part./mL) with water. To 1 mL of a silica nanoparticle aqueous suspension, 10 µL of a 50 mM His-tag (having a structure in which six histidines were bonded from the N-terminal, three glycines were bonded, and lysine was bonded to the C-terminal, and a carboxyl group at the C-terminal was amidated) aqueous solution, 10 µL of a 50 mM 2-aminoethanthiol-HCl aqueous solution, and 10 µL of a 50 mM DMT-MM aqueous solution were added, and the mixed solution was reacted at 25°C and 1,000 rpm for 19 hours using a thermoshaker to introduce a His-tag and a thiol group into silica nanoparticles.

### 2. Production of substrate

A gold substrate was immersed in an EtOH solution of 0.5 mM 2-(2-bromoisobutyryloxy) undecyl thiol and 0.5 mM amino-EG6 undecane thiol to introduce an amino group and a bromo group to the substrate surface (30°C, 20 h).

### 3. Introduction of NTA onto substrate to form nickel complex

80 µL of a DMSO solution of 5 mM isothiocyanobenzyl-NTA was dropped on a substrate into which an amino group and a bromo group were introduced, and the substrate was allowed to stand at 25°C for 2 hours. The substrate was washed with DMSO twice and pure water twice, and drying with N₂ was performed. 30 µL of a 4 mM NiCl₂ aqueous solution was dropped on the substrate, and the substrate was allowed to stand at 25°C for 15 minutes and washed with pure water 3 times to form a Ni complex of NTA.

### 4. Immobilization of silica nanoparticles in which His-tag and thiol group are introduced

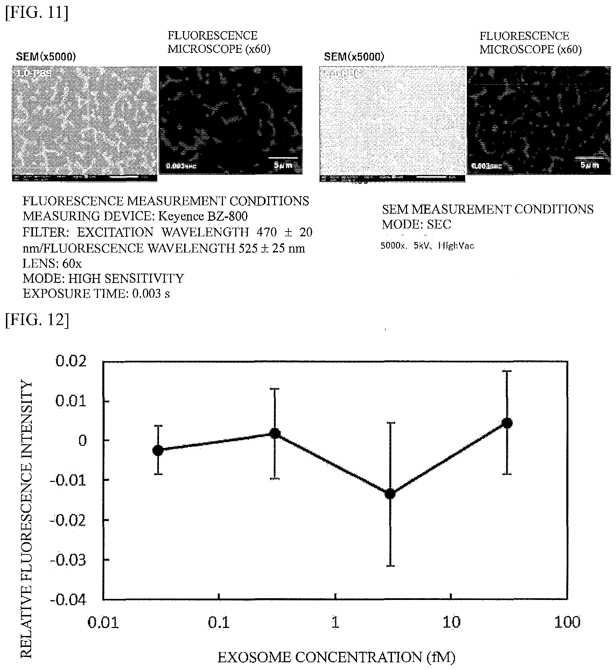

50 µL of silica nanoparticles into which a His-tag and a thiol group were introduced was dropped on the substrate using phosphate buffered saline (PBS) as a solvent, and the substrate was allowed to stand at 25°C for 1 hour. The substrate was washed with PBS 3 times, and the substrate surface was observed with a fluorescence microscope (Keyence BZ-800) and an SEM. (FIG. 11)

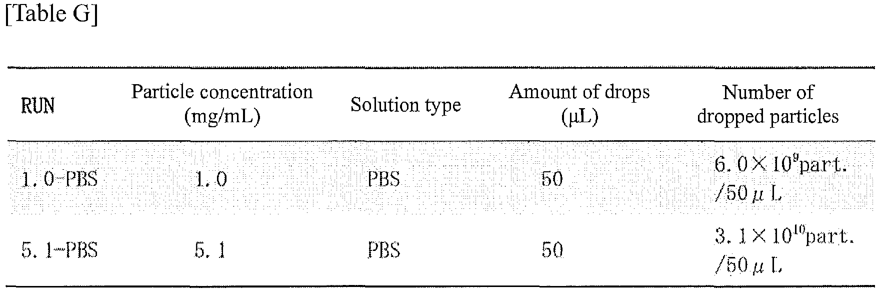

From the observation results illustrated in FIG. 11, it was observed that silica particles were partially single, but most of the silica particles were agglomerated. From this, it was shown that when the silica nanoparticles were modified with a His-tag and immobilized on the substrate, the silica nanoparticles were monodispersed, not agglomerated, and immobilized in a single layer.

### (Comparative Example 2: Sensitivity of sensor produced without using particles)

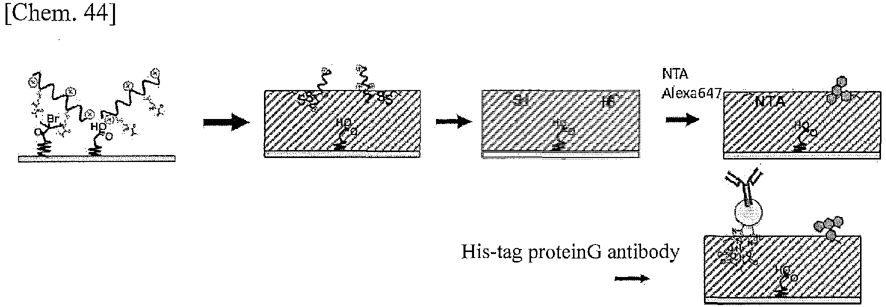

### (SAM formation on substrate)

A gold substrate (9.8 mm × 4.3 mm) was subjected to UV-O3 treatment for 20 min to clean up a substrate surface. A 1 mM EtOH solution was prepared by mixing 2-(2-bromoisobutyryloxy)undecyl thiol and carboxy-EG6 undecanethiol at a ratio of 1 : 1 in an EtOH solvent, and the gold substrate was immersed in the solution. After the substrate was allowed to stand at 30°C for 20 hours, the substrate was washed with EtOH to form a self-assembled monolayer (SAM) on the surface of the gold substrate. A polymer E2 aqueous solution (1 mg/mL) was diluted 80-fold with DMF, 4 µL of the diluted solution was dropped on the substrate on which the SAM was formed, and the substrate was allowed to stand at 25°C for 1 hour. The substrate was washed with ethanol.

### (Polymer matrix synthesis)

After a prepolymer solution and an ascorbic acid solution were prepared and dissolved oxygen was removed by freeze freeze-degassing, the solution was mixed in a glove box and polymerization was performed at 25°C for 20 hours. The substrate after polymerization was washed with pure water.

**[Table H]**

| | ATRP recipe | |
|---|---|---|
| MPC | | 500 mM |
| Ethyl *α-*bromoisobutyrate | | 1.25 mM |
| TPMA | | 0.125 mM |
| CuBr₂ | | 0.0125 mM |
| L-Ascorbic Acid | | 1.25 mM |
| EtOH | | 270 *µ*L |

### (Acetic acid treatment)

The substrate was placed in a 50% MeOH and 50% acetic acid solution, subjected to an ultrasonic treatment for 5 minutes, and then washed with pure water.

### (S-S reductive cleavage)

A 0.5 mM tris(2-carboxyethyl)phosphine hydrochloride aqueous solution was placed in a thermoshaker and preheated at 40°C for 5 minutes. Then, the acetic acid-treated substrate was placed and reacted at 40°C for 1 min. The substrate after reaction was washed with pure water.

### (Fluorescence, NTA introduction)

30 µL of a 20% DMSO-containing 10 m MPB (pH 7.4) solution of 50 µM maleimido-C3-NTA and 50 µM Alexa 647-C2-maleimide was dropped on the substrate, and the substrate was allowed to stand at 25°C for 1 h and reacted and then washed with DMSO and pure water.

### (Introduction of His-tag proteinG and antibody)

30 µL of a 4 mM NiCl₂ aqueous solution was dropped on the substrate, and the substrate was allowed to stand (25°C, 15 min). The substrate after reaction was washed with pure water. 30 µL of a PBS solution of 1 µM His-tagged proteinG (His-ProteinG) was dropped on the substrate, and the substrate was allowed to stand at 25°C for 1 hour to immobilize His-ProteinG. The substrate after His-ProteinG immobilization was washed with PBS.

30 µL of a PBS solution of 0.1 µM Anti-CD9 was dropped, and the substrate was allowed to stand at 25°C for 1 hour to introduce an antibody into the substrate. The substrate after introduction of the antibody was washed with PBS.

A flat pipette tip manufactured by FUKAE KASEI Co., Ltd. was mounted, and a change in fluorescence intensity on the surface of the substrate was measured with an automatic dispensing device with a fluorescence microscope.

The concentrations of the PC3 exosome particle PBS solutions were 0.03, 0.3, 3, and 30 fM.
1. Chip attachment
2. Suction of 150 µL of PBS and F0 measurement × 5
3. Suction of 100 µL of sample (exosome derived from PC3)
4. Reaction 5 min (25°C)
5. Total discharge
6. Suction of 150 µL of 10 mM PBS (140 mM NaCl, pH 7.4)
7. Automatic measurement
   Fluorescence microscope
   Camera: Zyla 4.2
   Filter: Cy5
   (Excitation wavelength 604 to 644 nm, fluorescence wavelength 672 to 712 nm)
   Objective lens: ×5
   Exposure time: 0.5 sec
   Light amount: 12%
   Light source: mercury lamp
   8. Repeat 3 to 7

### (Results)

The results are illustrated in FIG. 12. There was no response to the exosome in the non-pore-formed substrate using no silica particle core.

### (Example 14: Animal experiment)

In the present example, a demonstration example by animal experiments is shown.

Exosomes in dog and cat blood were measured. The exosome was measured by collecting blood of dogs and cats with cancer or visceral diseases determined by a veterinarian. After the completion, the exosome was measured again and the difference was observed to examine whether cancer and visceral disease of dogs and cats were detected.

In addition, the following uses can be assumed.

### Non-human use

1. Examination of diseases including cancer of pet (cats, dogs, birds, and the like) animals
2. Detection of infectious disease causing viruses and infectious proteins (such as prions) and other pathogenic/infectious factors of livestock (cows, horses, pigs, chicken, and the like)
3. Detection of infectious disease causing viruses and infectious proteins (such as prions) and other pathogenic/infectious factors of harmful animals (birds, bats, foxes, mongooses, raccoons, and the like)
4. Quality and freshness check of marine products
5. Quality and freshness check of agricultural products
6. Check for pathogens and viruses of plants and trees

### (Example 15: Clinical application)

The present example demonstrates the application in cancer diagnosis as follows.

Whether or not cancer can be detected by the sensor is examined by observing a difference in response between the sensors before and after a total resection surgery for the presence or absence of cancer detection under the patient's consent in a clinical study approved by a clinical research review board of a clinical experiment implementation hospital. Whether it is possible to check cancer metastasis or recurrence is examined by continuous observation. Whether the therapeutic effect of the drug therapy can be evaluated by the difference in sensor response before and after the medication is examined. Whether the lifestyle disease can be diagnosed from the relationship between the ingested food and the sensor response is examined. In addition, the presence of a cancer cell surface antigen is estimated from the sensor response, and whether companion diagnosis is possible is examined. Furthermore, it is examined whether the quality control of the cell in the treatment using the cell is possible from the sensor response.

In addition to the above, the following applications are assumed for the technique of the present disclosure.
Cancer test
Check of remainder after cancer surgery
Check of metastasis and recurrence of cancer
Therapeutic effect of pharmacotherapy
Examination of lifestyle diseases
Companion diagnosis for dosing
Quality control of cells in cell engineering

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents is specifically described in the present specification.

### Industrial Applicability

The technique provided in the present disclosure can be utilized in any field (including diagnosis) utilizing a test analysis technique.

## Claims

1. A substrate for producing a sensor for analysis comprising:
A) a substrate body; and
B) particles disposed on the substrate body in a state of a single layer.

2. The substrate for producing a sensor for analysis according to claim 1, wherein the particles are disposed without agglomeration.

3. The substrate for producing a sensor for analysis according to claim 1 or 2, wherein the particles include particles integrated with a modifier.

4. The substrate for producing a sensor for analysis according to any one of claims 1 to 3, the substrate further comprising C) a polymer matrix disposed on the substrate body, the polymer matrix having a concave portion that at least partially fits into an object, wherein the particles are disposed in the concave portion.

5. A convex sensor for analysis comprising:
A) a substrate body;
B) a polymer matrix disposed on the substrate body, the polymer matrix having a concave portion that at least partially fits into an object to be detected and having the particles disposed in the concave portion without agglomeration;
C) a group for binding a signal substance disposed on the particle; and
D) a group for binding a specific binding molecule that binds to a molecule to be detected, the group being disposed on the particle,
wherein the particles are disposed on the substrate body at a density of 1 × 10³ particles/mm² to 1 × 10¹⁰ particles/mm².

6. A sensor for analysis comprising:
A) a substrate body;
B) a polymer matrix disposed on the substrate body, the polymer matrix having a concave portion that at least partially fits into an object to be detected;
C) a group for binding a signal substance disposed in the concave portion; and
D) a group for binding a specific binding molecule that binds to a molecule to be detected, the group being disposed in the concave portion,
wherein the concave portions are disposed in the substrate body at a density of 1 × 10³ pieces/mm² to 1 × 10¹⁰ pieces/mm² without agglomeration.

7. The sensor for analysis according to claim 6, wherein the concave portions are present at a density of 1 × 10⁴ pieces/mm² to 1 × 10⁶ pieces/mm² without agglomeration.

8. A method for producing a substrate for producing a convex sensor for analysis, the method comprising:
A) a step of providing particles;
B) a step of adding the particles to a substrate so that the particles are disposed on a substrate body without agglomeration;
C) a step of providing a raw material of a polymer matrix to the substrate on which the particles are immobilized; and
D) a step of forming a substrate on which the polymer matrix is disposed by subjecting the substrate to a condition under which the polymer matrix is polymerized.

9. A method for producing a substrate for producing a sensor for analysis, the method comprising:
A) a step of providing particles;
B) a step of adding the particles to a substrate so that the particles are disposed on a substrate body without agglomeration;
C) a step of providing a raw material of a polymer matrix to the substrate on which the particles are immobilized;
D) a step of forming a substrate on which the polymer matrix is disposed by subjecting the substrate to a condition under which the polymer matrix is polymerized; and
E) a step of forming a concave portion by subjecting the substrate to a condition under which the particles are dissociated from the substrate.

10. The method according to claim 8 or 9, wherein the step of adding the particles so that the particles are disposed without agglomeration includes adding particles to a substrate at a concentration of 1.0 × 10⁰ particles/µL to 1.0 × 10¹⁰ particles/µL or in an amount of 1.0 × 10⁰ particles/mm² to 1.0 × 10¹⁰ particles/mm² per surface area of the substrate.

11. A method for producing a convex sensor for analysis, the method comprising:
A) a step of providing particles;
B) a step of adding the particles to a substrate so that the particles are disposed on a substrate body without agglomeration;
C) a step of providing a raw material of a polymer matrix to the substrate on which the particles are immobilized;
D) a step of forming a substrate on which the polymer matrix is disposed by subjecting the substrate to a condition under which the polymer matrix is polymerized; and
E) a step of binding a substance required for analysis to the particles.

12. A method for producing a sensor for analysis, the method comprising:
A) a step of providing particles;
B) a step of adding the particles to a substrate so that the particles are disposed on a substrate body without agglomeration;
C) a step of providing a raw material of a polymer matrix to the substrate on which the particles are immobilized;
D) a step of forming a substrate on which the polymer matrix is disposed by subjecting the substrate to a condition under which the polymer matrix is polymerized;
E) a step of forming a concave portion by subjecting the substrate to a condition under which the particles are dissociated from the substrate; and
F) a step of binding a substance required for analysis to the concave portion.

13. The method for producing a sensor for analysis according to claim 12, wherein the sensors for analysis having a coefficient of variation of a relative fluorescence intensity change in target substance detection of 20% or less are produced at a proportion of 80% or more by forming concave portions having a density of 1 × 10¹⁰ pieces/mm² or less.

14. The method for producing a sensor for analysis according to claim 12, wherein the sensors for analysis having a coefficient of variation of a relative fluorescence intensity change in target substance detection of 20% or less are produced at a proportion of 80% or more by forming concave portions whose area occupancy rate on the substrate is equal to or less than the closest packing.

15. The method according to any one of claims 8 to 14, wherein the step of disposing the particles without agglomeration includes a step of disposing the particles on the substrate at a density of 1 × 10³ particles/mm² to 1 × 10¹⁰ particles/mm².

16. The method according to any one of claims 8 to 15, wherein the particles are in a state of a single layer.

17. The method according to any one of claims 9 and 12 to 14, wherein in the step of forming the concave portion, the concave portions are present at a density of 1 × 10³ particles/mm² to 1 × 10¹⁰ particles/mm².

18. The method according to any one of claims 8 to 17, wherein the step of disposing the particles includes a spin coating method, a method of dropping the particles on a substrate, a method of immersing a substrate in a particle dispersion, a method of pulling up a substrate from a particle dispersion, or a method of spraying a particle dispersion.
